# EUROPEAN PATENT APPLICATION

(11) **EP 3 834 849 A1**
(43) Date of publication of application: **16.06.2021**
(21) Application number: 19841265.2
(22) Date of filing: 24.07.2019
(51) Int. Cl.: A61K 48/00, A61K 39/395, C07K 16/00, C12N 5/10, C12N 15/13, A61P 35/00

(54) **METHOD FOR TREATING TUMOR USING IMMUNE EFFECTOR CELL**

(30) Priority: 24.07.2018 CN 201810821044; 03.04.2019 CN 201910267885
(71) Applicant: CAFA THERAPEUTICS LIMITED, Dublin D01 AE27 (IE)
(72) Inventor: LI, Zonghai, Shanghai 200231 (CN); WANG, Huamao, Shanghai 200231 (CN)
(74) Representative: Lavoix
(86) International application number: PCT/CN2019/097453
(87) International publication number: WO 2020/020210

(57) **Abstract**

Provided is an immune effector cell expressing a chimeric antigen receptor that confers an individual specific recognization of BCMA, and a therapeutic method using the immune effector cell. The method improves the efficacy of immune effector cells in tumor treatments.

## Description

### FIELD OF THE INVENTION

The present invention belongs to the field of immunotherapy; specifically, it relates to immune cell therapy that targets and recognizes tumor antigens, triggers activation of immune effector cells, and exerts anti-tumor effects.

### BACKGROUND OF THE INVENTION

Multiple myeloma (MM) is a malignant plasma cell tumor that accounts for 2% of all cancer deaths. The main pathology is the unlimited expansion and enrichment of plasma cells in the bone marrow, leading to osteonecrosis. At present, the main treatment options are chemotherapy and stem cell transplantation. The chemotherapy drugs are mainly steroids, thalidomide, lenalidomide, bortezomib and so on.

BCMA is a B-cell maturation antigen. It is a type III transmembrane protein composed of 185 amino acid residues. It belongs to the TNF receptor superfamily. Its ligand belongs to the TNF superfamily, such as a proliferation-inducing ligand (APRIL), B lymphocyte stimulating factor (BAFF). After the binding of BCMA to its ligand, the proliferation and survival of B cells can be activated. BCMA is specifically expressed in plasma cells and multiple myeloma cells, but not expressed in hematopoietic stem cells and other normal tissue cells, so BCMA can be used as an ideal target for targeted therapy of MM.

### SUMMARY OF THE INVENTION

The purpose of the present invention is to provide a technical means having excellent killing effects on BCMA-positive tumors.

In the first aspect of the present invention, provided is a method for treating BCMA-positive tumors. The method comprises administering to a subject at least one course of treatment of immune effector cells expressing chimeric antigen receptor (CARs), wherein the immune effector cells specifically recognizes BCMA.

In a specific embodiment, the dose of immune effector cells in each course of treatment does not exceed about 1×10⁹ cells/kg subject's body weight or does not exceed about 1×10¹⁰ cells in total. Preferably, the dose of immune effector cells in each course of treatment does not exceed about 1×10⁸ cells/kg the subject's body weight or does not exceed about 1×10⁹ cells in total. Preferably, the dose of immune effector cells in each course of treatment does not exceed about 1×10⁷ cells/kg the subject's body weight or does not exceed about 5×10⁸ cells in total.

In a specific embodiment, the total dose of the immune effector cells in each course of treatment is no less than 1×10⁵. Preferably, the total dose of the immune effector cells in each course of treatment is no less than 1×10⁶. Preferably, the total dose of the immune effector cells in each course of treatment is no less than 1×10⁷.

In a specific embodiment, the immune effector cells are administered to the subject for 2-5 courses of treatment.

In a specific embodiment, the immune effector cells of a subsequent course of treatment are administered after the immune effector cells administered previously are not detected in the body.

In a specific embodiment, the immune effector cells of the subsequent course of treatment are administered at a time point of about 4 to 24 weeks after the administration of the previous course of treatment.

In a specific embodiment, the dose of the immune effector cells administered in the subsequent course of treatment is lower than, equal to, or higher than that of the immune effector cells administered in the previous course of treatment.

In a specific embodiment, the dose of immune effector cells administered in the subsequent course of treatment is higher than that of immune effector cells administered in the previous course of treatment. Preferably, the dose of immune effector cells administered in the subsequent course of treatment is 2 times, 5 times, 7 times or 10 times the dose of the immune effector cells administered in the previous course of treatment.

In a specific embodiment, the immune effector cells of each course of treatment are divided into N administrations within 15 days, and N is a natural number no less than 1, in a preferred embodiment, N is 1, 2, 3, or 4.

In a specific embodiment, the subject has any one of the following characteristics when administering the immune effector cells in a subsequent course of treatment:
(i) the serum level of the factor indicating cytokine release syndrome (CRS) in the subject is about 10 times lower, about 25 times lower, and/or about 50 times lower than that of the factor in the subject immediately before the administration of the immune effector cells in the previous course of treatment;
(ii) a grade 3 or higher neurotoxicity is not shown;
(iii) the neurotoxicity or CRS level is reduced compared to the peak level of neurotoxicity or CRS level after the administration of the immune effector cells of the previous course of treatment; or
(iv) the subject shows no detectable humoral or cell-mediated immune response against CAR expressed by cells of the previous course of treatment.

In a specific embodiment, when the immune effector cells of the subsequent course of treatment are administered as described above, the subject has the following characteristics (iii), the CRS level is reduced by at least 50% compared with the peak level of CRS after administering the immune effector cells in the previous course of treatment, preferably, reduced by at least 20%, more preferably, by at least 5%, or the CRS level is comparable to the CRS level before administering the immune effector cells of the previous course of treatment.

In a specific embodiment, the method further includes pretreatment before administering the immune effector cells, and the pretreatment comprises administering a chemotherapeutic agent or radiation therapy to the subject, or a combination thereof.

In a specific embodiment, the pretreatment is carried out 2-12 days before administering the immune effector cells. In a preferred embodiment, it is carried out 2-7 days before administering the immune effector cells.

In a specific embodiment, the chemotherapeutic agent is selected from any one or a combination of the following: cyclophosphamide, fludarabine.

In a specific embodiment, the administration amount of fludarabine is about 10-50 mg/m²/day, or about 15-40 mg/m²/days, or about 15-35mg/m²/day, or 15-30mg/m²/day, or 20-36mg/m²/day, or about 20-30mg/m²/day.

In a preferred embodiment, the administration amount of fludarabine is about 20-30 mg/m²/day.

In a preferred embodiment, the administration amount of fludarabine is about 20-26 mg/m²/day.

In a specific embodiment, the administration amount of cyclophosphamide is about about 100-700 mg/m²/day, or about 150-600 mg/m²/day, or about 190-600 mg/m²/day, or about 190-560 mg/m²/day.

In a specific embodiment, the administration amount of cyclophosphamide is about 150-400 mg/m²/day, preferably, about 190-350 mg/m²/day.

In a preferred embodiment, the administration amount of cyclophosphamide is about 400-600mg/m²/day, preferably, about 450-600 mg/m²/day, more preferably, about 450-560mg/m²/day.

When fludarabine and cyclophosphamide are administered at the same time, the respective administration amounts of fludarabine and cyclophosphamide are also as described above.

In a specific embodiment, the chemotherapeutic agent is continuously used for no more than 6 days.

In a specific embodiment, the cyclophosphamide is continuously used for 1-5 days.

In a specific embodiment, the fludarabine is used continuously for 2-4 days.

When fludarabine and cyclophosphamide are administered at the same time, the continuous use time of each of fludarabine and cyclophosphamide is as described above.

In a specific embodiment, the tumor is multiple myeloma.

In a specific embodiment, the chimeric antigen receptor comprises an antibody specifically binding to BCMA, a transmembrane domain, and an intracellular domain, and the heavy chain variable region and the light chain variable region of the antibody comprises:
the HCDR1 shown in SEQ ID NO: 1, the HCDR2 shown in SEQ ID NO: 2, the HCDR3 shown in SEQ ID NO: 3; and the LCDR1 shown in SEQ ID NO: 6, the LCDR2 shown in SEQ ID NO: 7, the LCDR3 shown in SEQ ID NO: 8; or
the HCDR1 shown in SEQ ID NO: 1, the HCDR2 shown in SEQ ID NO: 2, the HCDR3 shown in SEQ ID NO: 4; and the LCDR1 shown in SEQ ID NO: 6, the LCDR2 shown in SEQ ID NO: 7, the LCDR3 shown in SEQ ID NO: 9; or
the HCDR1 shown in SEQ ID NO: 1, the HCDR2 shown in SEQ ID NO: 2, the HCDR3 shown in SEQ ID NO: 5; and the LCDR1 shown in SEQ ID NO: 6, the LCDR2 shown in SEQ ID NO: 7, the LCDR3 shown in SEQ ID NO: 10; or
The HCDR1 shown in SEQ ID NO: 11, the HCDR2 shown in SEQ ID NO: 12, the HCDR3 shown in SEQ ID NO: 5; and the LCDR1 shown in SEQ ID NO: 6, the LCDR2 shown in SEQ ID NO: 7, the LCDR3 shown in SEQ ID NO: 10; or
The HCDR1 shown in SEQ ID NO: 13, the HCDR2 shown in SEQ ID NO: 14, the HCDR3 shown in SEQ ID NO: 5; and the LCDR1 shown in SEQ ID NO: 6, the LCDR2 shown in SEQ ID NO: 7, the LCDR3 shown in SEQ ID NO: 10.

In a specific embodiment, the heavy chain variable region and the light chain variable region of the antibody comprise the HCDR1 shown in SEQ ID NO: 11, the HCDR2 shown in SEQ ID NO: 12, the HCDR3 shown in SEQ ID NO: 5; and the LCDR1 shown in SEQ ID NO: 6, the LCDR2 shown in SEQ ID NO: 7, the LCDR3 shown in SEQ ID NO: 10.

In a specific embodiment, the chimeric antigen receptor includes an antibody that specifically binds to BCMA, a transmembrane domain, and an intracellular domain, and the light chain variable region of the antibody comprises the LCDR1 shown in SEQ ID NO: 6, the LCDR2 shown in SEQ ID NO: 7, and the LCDR3 shown in SEQ ID NO: 10.

In a specific embodiment, the light chain variable region of the antibody comprises the amino acid sequence shown in SEQ ID NO:20.

In a specific embodiment, the heavy chain variable region of the antibody comprises the HCDR3 shown in SEQ ID NO:5.

In a specific embodiment, the heavy chain variable region of the antibody comprises the amino acid sequence shown in SEQ ID NO: 15 and the light chain variable region of the antibody comprises the amino acid sequence shown in SEQ ID NO: 16; or
the heavy chain variable region of the antibody comprises the amino acid sequence shown in SEQ ID NO: 17 and the light chain variable region of the antibody comprises the amino acid sequence shown in SEQ ID NO: 18; or
the heavy chain variable region of the antibody comprises the amino acid sequence shown in SEQ ID NO: 19 and the light chain variable region of the antibody comprises the amino acid sequence shown in SEQ ID NO: 20; or
the heavy chain variable region of the antibody comprises the amino acid sequence shown in SEQ ID NO: 21 and the light chain variable region of the antibody comprises the amino acid sequence shown in SEQ ID NO: 20; or
the heavy chain variable region of the antibody comprises the amino acid sequence shown in SEQ ID NO: 21 and the light chain variable region of the antibody comprises the amino acid sequence shown in SEQ ID NO: 20.

In a specific embodiment, the heavy chain variable region of the antibody comprises the amino acid sequence shown in SEQ ID NO: 21 and the light chain variable region of the antibody comprises the amino acid sequence shown in SEQ ID NO: 20.

In a specific embodiment, the antibody comprises a sequence of scFv shown in SEQ ID NO: 25, 27, or 29.

In a specific embodiment, the chimeric antigen receptor comprises an amino acid sequence shown in SEQ ID NO: 30, 31, 32, 33, 34, 35, 36, 37, 38, 39, 40, or 41.

In a specific embodiment, the chimeric antigen receptor comprises an amino acid sequence shown in any one of SEQ ID NO: 36, 37, and 38.

In a preferred example, the chimeric antigen receptor comprises an amino acid sequence shown in SEQ ID NO:36.

In a specific embodiment, the immune effector cells are T cells, NK cells or NKT cells; in a preferred embodiment, the immune effector cells are T cells.

In a specific embodiment, the immune effector cells are derived from the subject's own body.

In a specific embodiment, the immune effector cells are derived from the allogeneic.

In a specific embodiment, the administration interval of the immune effector cells in each course of treatment is 4 weeks to 24 weeks.

In a preferred example, the number of immune cells in each course of treatment is basically the same.

In a preferred example, the number of immune effector cells administered in the subsequent course of treatment is larger than the number of immune cells administered in the previous course of treatment.

In a preferred example, the number of immune effector cells administered in the subsequent course of treatment is smaller than the number of immune cells administered in the previous course of treatment.

In a specific embodiment, the subject has not received treatment with immune cells expressing chimeric antigen receptors targeting BCMA before administering the immune effector cells.

In a specific embodiment, the subject has undergone surgical treatment, chemotherapy, or immunotherapy other than the treatment with immune cells that target BCMA-expressing chimeric antigen receptors before administering the immune effector cell therapy.

In a specific embodiment, before administering the immune effector cells in each course of treatment, the serum levels of a factor indicating CRS, a factor indicating neurotoxicity, a factor indicating tumor burden, and/or a factor indicating the host's anti-CAR immune response in the subject are evaluated.

In a specific embodiment, the factor indicating tumor burden is: the total number of tumor cells in the subject, or the total number of tumor cells in the organs of the subject, or the total number of tumor cells in the tissue of the subject, or the mass or volume of the tumor, or the extent of tumor metastasis, or the number of tumors.

In a specific embodiment, the factors indicating tumor burden include:
i) evaluating factors indicating tumor burden before administering the subsequent course of treatment; and
ii) based on the results of the evaluation, determining the subsequent course of treatment, and
iii) if the evaluation determines that the subject's tumor quality or volume is stable or decreasing, administering to the subject the subsequent course of treatment that comprises smaller or larger number of CAR-expressing cells than that in the previous course of treatment or about the same number of CAR-expressing cells as that in the previous course of treatment.

In a specific embodiment, the immune effector cells are administered at a dose of about 0.1×10⁶cells/kg subject's body weight to 5×10⁷ cells/kg subject's body weight, or the total dose of the immune effector cells administered is about 0.1x 10⁷ cells∼1x 10¹⁰ cells.

Preferably, it is about 0.5x 10⁶ cells/kg subject's body weight ∼1x 10⁷ cells/kg subject's body weight, or the total dose of the immune effector cells administered is about 0.1x 10⁸ cells∼1x 10⁹ cells;

More preferably, it is about 0.9×10⁶ cells/kg subject's body weight to 5×10⁶ cells/kg subject's body weight, or the total dose of the immune effector cells administered is about 0.1×10⁸ cells to 9×10⁸ cells.

In a specific embodiment, the positive rate of BCMA expression in the subject is greater than 50%, preferably, greater than 70%, or greater than 80%. More preferably, it is greater than 85%. More preferably, it is greater than 90%.

In a specific embodiment, the disease subtype of the subject is IgG κ type, or IgG λ type, or IgA λ type, or IgA κ type, or λ light chain type.

It should be understood that within the scope of the present invention, the above-mentioned technical features of the present invention and the technical features specifically described in the following (such as the embodiments) can be combined with each other to form a new or preferred technical solution. Due to space limitations, they will not be repeated here.

### BRIEF DESCRIPTION OF THE DRAWINGS

Figure 1 shows the efficacy results of different groups of patients.
Figure 2 shows the proliferation of BCMA CAR-T cells in subjects.

### DETAIL DESCRIPTION OF THE INVENTION

After in-depth clinical research, the inventors found that administering immune effector cells expressing chimeric antigen receptors (CARs) targeting BCMA to subjects in a specific amount significantly improves the efficacy of using immune effector cells for tumor treatment.

Unless otherwise defined, all professional terms, symbols, and other technical and scientific terms or specialized words used herein are intended to have the same meaning as commonly understood by those skilled in the art to which the present invention belongs. In some cases, for the purpose of clarification and/or ease of reference, terms with conventionally understood meanings are further defined herein. Such further definitions included herein shall not be construed as indicating a substantial difference from the conventional understanding in the art.

If the definition shown herein is different from the definition shown in the cited patents, published applications and other publications or is otherwise inconsistent, the definition shown herein shall prevail.

In the present invention, the description in range format should not be construed as a rigid limit to the scope of the claimed subject matter. Therefore, the description of a range should be considered to have specifically disclosed all possible subranges and individual values within the range. For example, for a specific numerical range, it should be understood that every intermediate value between the upper limit and lower limit of the range and any other stated values or intermediate values within the range are included in the claimed subject matter. The upper and lower limits of the range also belong to the scope of the claimed subject matter. The upper and lower limits of the smaller ranges can be independently included in these smaller range, and they also belong to the scope of the claimed subject matter, unless the upper and lower limits of the range are explicitly excluded. When the set range includes one or two limit values, the claimed subject matter also includes a range that excludes one or two of the limit values. This principle applies regardless of the scope.

The term "about" used herein refers to the usual error range of each value easily known by those skilled in the art. "About" a value or a parameter herein includes an embodiment that refers to the value or parameter itself. For example, description of "about X" includes description of "X". For example, "about" can mean within 1 or more than 1 according to the actual standard deviation in the art. Or "about" can mean a range of up to 10% (i.e., ±10%). For example, about 5 mg can include any number between 4.5 mg and 5.5 mg. When a specific value or composition is provided in the application and the scope of the patent application, unless otherwise indicated, "about" should be within the acceptable error range of the specific value or composition.

Any concentration range, percentage range, ratio range, or integer range described herein should be understood to include any integer within the stated range, and where appropriate, the value of the fraction (for example, one tenth of an integer and one hundredth of an integer), unless otherwise indicated.

The "administration interval" as used herein refers to the time elapsed between the administration of multiple courses of immune effector cell therapy and between the therapy and the pretreatment drugs administration to a subject. Therefore, the administration interval can be indicated as a range.

The "dose" as used herein can be expressed as a dose calculated on the basis of weight or a dose calculated on the basis of body surface area (BSA). The dose calculated on the basis of weight is the dose given to the patient calculated based on the body weight of the patient, such as mg/kg, number of immune effector cells/kg, etc. The dose calculated on the basis of BSA is the dose given to the patient calculated based on the surface area of the patient, such as mg/m2, and the number of immune effector cells/m2.

The "number of administrations" as used herein refers to the frequency of administration of immune effector cells or pretreatment drug doses within a given time. The number of administrations can be indicated as the number of doses per given time. For example, fludarabine can be administered as follows: once a day for 4 consecutive days, once a day for 3 consecutive days, once a day for 2 consecutive days, or once a day. Cyclophosphamide can be administered as follows: once a day for 4 consecutive days, once a day for 3 consecutive days, once a day for 2 consecutive days, or once a day.

This application relates to tumor treatments using adoptive cells or immune effector cells, including the administration of cells for one or more courses of treatment, and the methods, compositions and products used. Cells generally express chimeric antigen receptors such as chimeric antigen receptors (CARs) or other transgenic receptors such as T cell receptors (TCRs).

The present invention provides therapeutic methods and compositions for treating diseases (such as tumors) related to BCMA expression.

The present invention provides a method for treating tumors in a subject using adoptive cells or immune effector cells expressing genetically engineered (recombined) chimeric receptors. The method includes single treatment course-reinfusion, or multiple treatment course-reinfusion of adoptive cells or immune effector cells. As used herein, "dose" refers to the total quantity of adoptive cells or immune effector cells that are administered or reinfused in a course of treatment. In some embodiments, where multiple courses of treatment are included according to the methods described herein, the dose for each course of treatment is the same. In some embodiments, where multiple courses of treatment are included according to the methods described herein, the dosage for each course of treatment is different.

"Divided dose" refers to a single dose administered when the dose of an entire course of treatment is divided into multiple doses to the subject within a course of treatment. In some embodiments, in the case where the dose within a course of treatment is divided into multiple administrations to the subject, the divided dose for each administration is the same. In some embodiments, in the case where the dose within a course of treatment is divided into multiple administrations to the subject, the divided dose for each administration is different. For the purposes herein, unless otherwise specified, the dose refers to the total quantity of adoptive cells or immune effector cells administered or reinfused within a course of treatment.

In some embodiments, the methods described herein include reinfusion of the adoptive cells or immune effector cells in a single course of treatment. A single course of treatment refers to the reinfusion of a certain quantity of adoptive cells or immune effector cells within a certain period of time. In some embodiments, a certain total quantity of adoptive cells or immune effector cells is reinfused at one time during the course of treatment. In some embodiments, a certain total quantity of adoptive cells or immune effector cells is reinfused in two or more times. In some embodiments, a certain total quantity of adoptive cells or immune effector cells is reinfused in three or more times. In some embodiments, the adoptive cells or immune effector cells that are reinfused each time are equal divisions of the adoptive cells or immune effector cells to be reinfused. In some embodiments, the adoptive cells or immune effector cells that are reinfused each time are non-equal divisions of the adoptive cells or immune effector cells to be reinfused. In some embodiments, the quantity of adoptive cells or immune effector cells to be reinfused each time is determined by the physician according to the specific conditions of the subject. The specific conditions of the subject can be, for example, the overall health of the subject, the severity of the disease, the response to the previous dose of the same course of treatment, the response to the previous course of treatment, the condition of the subject's combined medication, the degree or possibility of toxic reaction, complications, and any other factors considered by the physician as influences on the quantity of adoptive cells or immune effector cells suitable for reinfusion to the subject. In some embodiments, for the multiple reinfusions of a certain total quantity of adoptive cells or immune effector cells, the quantity of adoptive cells or immune effector cells of each reinfusion shows an increasing trend. In some embodiments, for the multiple reinfusions of a certain total quantity of adoptive cells or immune effector cells, the quantity of adoptive cells or immune effector cells of each reinfusion shows an decreasing trend. In some embodiments, for the multiple reinfusions of a certain total quantity of adoptive cells or immune effector cells, the quantity of adoptive cells or immune effector cells of each reinfusion first shows a trend of increasing and then decreasing. In some embodiments, for the multiple reinfusions of a certain total quantity of adoptive cells or immune effector cells, the quantity of adoptive cells or immune effector cells of each reinfusion first shows a trend of decreasing and then increasing.

Multi-course reinfusion refers to having multiple said time periods, and a certain total quantity of adoptive cells or immune effector cells are reinfused in each time period. In some embodiments, the lengths of the multiple time periods are consistent. In some embodiments, the lengths of the multiple time periods are unequal. In some embodiments, the multiple treatment course refers to having at least two of the time periods. In some embodiments, the multiple treatment course refers to having at least three or more of the time periods.

In some embodiments, a certain total quantity of adoptive cells or immune effector cells is reinfused at one time in one of the multiple treatment courses. In some embodiments, a certain total quantity of adoptive cells or immune effector cells is reinfused in two or more times in one course of the multiple treatment courses. In some embodiments, a certain total quantity of adoptive cells or immune effector cells is reinfused three or more times in one course of the multiple treatment courses. In some embodiments, in one of the multiple treatment courses, the amount of the adoptive cells or immune effector cells to be reinfused each time is an equal division of the adoptive cells or immune effector cells to be reinfused. In some embodiments, in one of the multiple treatment courses, the amount of the adoptive cells or immune effector cells to be reinfused each time is a non-equal division of the adoptive cells or immune effector cells to be reinfused. In some embodiments, the quantity of adoptive cells or immune effector cells to be reinfused each time in one of the multiple treatment courses is determined by the physician according to the specific conditions of the subject. The specific conditions of the subject can be, for example, the overall health of the subject, the severity of the disease, the response to the previous dose of the same course of treatment, the response to the previous course of treatment, the condition of the subject's combined medication, the degree or probability of toxic reaction, complications, cancer metastasis, and any other factors considered by the physician as influences on the quantity of adoptive cells or immune effector cells suitable for reinfusion in the subject. In some embodiments, a certain total quantity of adoptive cells or immune effector cells are reinfused in a same number of times in each of the multiple treatment courses. In some embodiments, a certain total quantity of adoptive cells or immune effector cells are reinfused in different number of times in each of the multiple treatment courses. In some embodiments, a certain total quantity of adoptive cells or immune effector cells are reinfused in a same number of times in each of the multiple treatment courses. In some embodiments, a same certain total quantity of adoptive cells or immune effector cells are reinfused in each of the multiple treatment courses. In some embodiments, different certain total amounts of adoptive cells or immune effector cells are reinfused in each of the multiple treatment courses.

In some embodiments, the total amount of the adoptive cells or immune effector cells in each of the multiple treatment courses shows an increasing trend. In some embodiments, the total amount of the adoptive cells or immune effector cells in each of the multiple treatment courses shows a decreasing trend. In some embodiments, the total amount of the adoptive cells or immune effector cells in each of the multiple treatment courses first shows a trend of increasing and then decreasing. The total amount of the adoptive cells or immune effector cells in each of the multiple treatment courses first shows a trend of decreasing and then increasing.

In some embodiments, the method described herein includes monitoring the exposure of the subject to the adoptive cells or immune effector cells, and determining the doses and intervals for the subsequent divided administrations or the subsequent courses of treatment according to the exposure. In some embodiments, the methods described herein include monitoring the exposure of the subject to the adoptive cells or immune effector cells, and maintaining or reducing the doses for the subsequent divided administrations or subsequent courses of treatment and/or maintaining or extending the intervals between the subsequent divided administrations or between the subsequent courses of treatment, according to the exposure which reaches or exceeds a certain level. In some embodiments, the methods described herein include monitoring the exposure of the subject to the adoptive cells or immune effector cells, and maintaining or increasing the doses for the subsequent divided administration or the subsequent courses of treatment and/or maintaining or shortening the intervals between the subsequent divided administration or between the subsequent courses of treatment, according to the exposure which is below a certain level.

In some embodiments, the method described herein includes monitoring the degree of toxic reaction or risk of the subject to the adoptive cells or immune effector cells, and determining the doses and intervals for the subsequent divided administrations or the subsequent courses of treatment according to the degree of toxicity or risk. In some embodiments, the degree of toxic reaction or risk includes, but is not limited to, for example, CRS, neurotoxicity, macrophage activation syndrome, and tumor lysis syndrome, etc..

In some embodiments, immune effector cells in a subsequent course of treatment are administered when the host adaptive immune response to the cells has not been detected, has not been established, and/or has not yet reached a certain level or degree or stage.

The so-called tumor burden includes classification, staging, the proportion of abnormal plasma cells in the bone marrow at the onset of illness, cytogenetic changes, whether extramedullary infiltration exists, and the response to treatment. In some embodiments, the tumor burden is evaluated using serum, urine protein electrophoresis, bone marrow smear, bone marrow biopsy, MRD, MRI and/or CT.

In some embodiments, the dose of immune effector cells for each course of treatment includes a cell dose sufficient to reduce the tumor burden of the subject. When administering cells in the subsequent course of treatment, the serum level of factors indicating cytokine-release syndrome (CRS) in the subject does not exceed 10 or 25 times that in the subject before the administration of immune effector cell therapy, and/or at the peak level of CRS-related results in the subject and it began to decline after the administration of immune effector cells, and when the subject does not have a detectable adaptive host immune response specific to the chimeric antigen receptor expressed by the immune effector cells of the previous course of treatment.

In some embodiments, the administered dose of immune effector cells is no less than about 0.5x10⁵ cells/kg subject's body weight or the total dose is no less than 1×10⁵ cells; preferably, no less than about 0.1×10⁶ cells/kg body weight or the total dose is no less than 1x 10⁶ cells; preferably, no less than about 0.5x 10⁶ cells/kg subject's body weight or the total dose is no less than 1x 10⁷cells; preferably, no less than about 0.9x 10⁶ cells/kg subject's body weight or the total dose is no less than 1x 10⁷cells.

In a specific embodiment, the administered dose of immune effector cells is no more than about 1x 10⁹cells/kg subject's body weight or the total amount is no more than about 1x 10¹⁰ cells; preferably, no more than about 1x 10⁸ cells or the total quantity of cells is no more than about 1×10⁹ cells; preferably, no more than about 1×10⁷ cells or the total quantity of cells is no more than about 1×10⁹ cells, preferably, no more than about 5×10⁶ cells or the total number of cells is no more than about 9x10⁸ cells.

In a specific embodiment, the administration dose of the immune effector cells is about 0.1×10⁶ cells/kg subject's body weight to 5x10⁷ cells/kg subject's body weight, or the total dose of immune effector cells administered is about 0.1 x 10⁷ cells to 1x 10¹⁰ cells. Preferably, it is about 0.5×10⁶ cells/kg subject's body weight to 1×10⁷ cells/kg subject's body weight, or the total dose of the immune effector cells is about 0.1×10⁸ cells to 1×10⁹ cells. More preferably, it is about 0.9×10⁶ cells/kg subject's body weight to 5×10⁶ cells/kg subject's body weight, or the total dose of the immune effector cells is about 0.1×10⁸ cells to 9×10⁸ cells.

### Immune effector cell therapy

The method provided by the present invention includes administering at least one course of immune effector cells expressing chimeric antigen receptors (such as CAR, TCR, TFP, TAC) that recognize BCMA to tumor subjects expressing BCMA.

The "subject" as used herein is a mammal, such as a human or other animal, usually a human. In some embodiments, the subject has been treated with chemotherapy or radiotherapy before administering the immune effector cells in a previous course of treatment and/or administration of immune effector cells in a subsequent course of treatment. In some aspects, the subject is refractory or non-responsive to other therapeutic agents.

In some embodiments, the tumor is persistent or recurrent, for example, after intervention with another treatment (including chemotherapy, radiation), the tumor still progresses or recurs after being controled..

In some embodiments, the subject is responsive to other therapeutic agents, reducing tumor burden. In some aspects, the subject is initially responsive to the therapeutic agent, but over time shows tumor recurrence. In some embodiments, the subject is determined to have a risk of recurrence, for example, at a high risk of recurrence, and thus the CAR T cells of the present invention are prophylactically administered to reduce the possibility of recurrence or prevent recurrence.

In some embodiments, the dose and the reinfusion time are determined by the subject's initial tumor burden. For example, in some cases, the number of immune effector cells generally given to subjects in the previous course of treatment is low. In the case of low tumor burden (for example tumor markers can be used to assess tumor burden and/or minimal residual disease of solid tumors), the initial dose may be higher. In other cases, in subjects with higher tumor burden, cells of each course of treatment can be divided into several continuous infusions, such as divided into 1, 2, 3, 4, 5, 6, 7, 8, 9 or 10 administrations, preferably 1-5 administrations, more preferably 2-3 administrations. 1, 2, 3, 4, 5, 6 more days of interval or no interval between each administrations.

The term "treatment" as used herein refers to the complete or partial alleviation or reduction of the tumor or its related symptoms, as well as the improvement of objective indicators in relevant evaluation. The desired therapeutic effects include, but are not limited to, preventing the occurrence or recurrence of tumors, reducing symptoms, reducing any direct or indirect pathological results of tumors, preventing extramedullary lesions, reducing the rate of tumor progression, improving or reducing tumor status, and reducing or improving the prognosis. As used herein, "inhibiting" a function or activity means a reduction in function or activity when compared to the same condition in other situations or compared to another condition.

In some embodiments, the cell therapy can be administered by autologous reinfusion. Therefore, in some aspects, the cells are derived from the subject in need of treatment and are administered to the same subject after isolation and processing.

In some embodiments, the cell therapy is treated by allogeneic reinfusion, wherein the cells are isolated and/or extracted and prepared from the donor in other cases, and the donor and the subject reinfused the cells are different. In some embodiments, the donor and recipient are genetically identical. In some embodiments, the donor and recipient are genetically similar. In some embodiments, the recipient and the donor have the same HLA class or supertype.

The cells can be administered by any suitable means, for example, by injection, such as intravenous injection, intraocular injection, fundus injection, subretinal injection, intravitreal injection, reverse septal injection, subscleral injection, intrachoroidal injection, anterior chamber injection, subconjectval injection, subconjuntival injection, episcleral cavity injection, retrobulbar injection, periocular injection, or perispheric delivery. In some embodiments, the cells are administered by instillation. In some embodiments, the cells are administered by multiple instillations, for example, multiple administrations in no more than 30 days, or the cells are administered by continuous infusion.

In the prevention or treatment of diseases, the administered dose may depend on the type of disease, the chimeric antigen receptor or cell type, the severity of the disease and the course of the disease, the subject's prior treatment history, and the response to the given cells, and the judgment of the attending physician.

In some embodiments, the immune effector cells are used as a part of a combination therapy, for example, combined with another interventional therapy, such as antibodies, engineered immune effector cells, receptors or reagents, cytotoxic drugs, or other treatment methods, simultaneously or sequentially, in any order. In some embodiments, immune effector cells are used in combination with one or more other treatment methods, or in combination with another interventional treatment method, simultaneously or sequentially in any order. In some cases, the immune effector cells are co-administered with another treatment in times close enough, to produce a therapeutic effect greater than that of the above-mentioned immune effector cell population or one or more other therapeutic drugs or methods, and vice versa. In some embodiments, the immune effector cells are administered before one or more of other therapeutic agents. In some embodiments, the immune effector cells are administered after one or more of other therapeutic agents. In some embodiments, one or more other therapeutic drugs include cytokines, such as IL-2, IL-12, to enhance persistence.

In some embodiments, the method includes administering pretreatment before administering immune effector cells, for example, chemotherapy drugs (chemotherapeutic agents), whole body radiation, local radiation therapy, etc., or a combination thereof. In some embodiments, the method includes pretreatment to the subject with one or more chemotherapeutic agents. In some embodiments, the method includes pretreatment to the subject with a tubulin inhibitor and one or more other chemotherapeutic agents. Without being limited by theory, the effects of pretreatment are believed to include, but are not limited to, lymphocyte clearance and tumor burden reduction. The chemotherapeutic agent refers to drugs used in chemotherapy, for example, cyclophosphamide, fludarabine, protease inhibitors (such as bortezomib, carfilzomib, etc.), immunomodulators (such as thalidomide, lenalidomide, pomalidomide, etc.), melphalan, adriamycin, dexamethasone, prednisone, etc.

Pretreatment can improve the effect of immune effector cell therapy. The day of the first infusion of immune effector cells (such as CAR T cells) for each course of treatment is set as day 0. Pretreatment can be given at any time before the infusion of immune effector cells. In some embodiments, fludarabine or cyclophosphamide alone or in combination is used as a pretreatment, and at least 1, 2, 3, 4, 5, 6, 7, 8, 9, 10, 11, 12, 13, 14, 15, 16, 17, 18, 19, 20, 21, 22, 23, 24, 25, 26, 27, 28, 29 or 30 days before CAR-T cell infusion, preferably at least 1, 2, 3, 4, 5, 6, 7, 8, 9, 10, 11, 12, 13, 14, 15, 16, 17, 18, 19 or 20 days, more preferably at least 2, 3, 4, 5, 6, 7, or 8 days to the subject. In some embodiments, pretreatment includes administering fludarabine and cyclophosphamide 2-12 days before CAR-T cell infusion. In some embodiments, the pretreatment includes administering fludarabine and cyclophosphamide 7 days before the CAR-T cell infusion.

The timing of administration of pretreatment components can be adjusted to maximize the therapeutic effect of CAR T. Usually, fludarabine and cyclophosphamide can be given daily. In some embodiments, fludarabine and cyclophosphamide are administered daily for about 1 day, about 2 days, about 3 days, about 4 days, about 5 days, about 6 days, or about 7 days. In some embodiments, fludarabine is administered daily for 4 consecutive days, and cyclophosphamide is administered daily for 2 consecutive days. In some embodiments, fludarabine is administered daily for 2 consecutive days, and cyclophosphamide is administered daily for 2 consecutive days. In some embodiments, fludarabine is administered daily for 3 consecutive days, and cyclophosphamide is administered once, or cyclophosphamide is administered continuously for 2 days or 3 days or 5 days.

As mentioned above, the day when each round of CAR T cell therapy is given to the patient is designated as day 0. In some embodiments, fludarabine is administered to the patient on 6 days(i.e., day -6) before day 0, day -5, and day -4. In some embodiments, fludarabine is administered to the patient on days -5, -4, and -3. In some embodiments, fludarabine is administered to the patient on days -6, -5, -4, and -3. In some embodiments, fludarabine is administered to the patient on days -5, -4, -3, and -2. In some embodiments, fludarabine is administered to the patient on days -4, -3, and -2. In some embodiments, fludarabine is administered to the patient on days -3 and -2. In some embodiments, fludarabine is administered to the patient on days -3, -2, and -1. In some embodiments, the patient is administered cyclophosphamide on days -6, -5, -4, -3, and -2. In some embodiments, the patient is administered cyclophosphamide on days -6 and -5. In some embodiments, the patient is administered cyclophosphamide on days -4, -3, and -2. In some embodiments, the patient is administered cyclophosphamide on days -4 and -3. In some embodiments, the patient is administered cyclophosphamide on days -5 and -4. In some embodiments, the patient is administered cyclophosphamide on day -5. In some embodiments, the patient is administered cyclophosphamide on days -3 and -2. In some embodiments, the patient is administered cyclophosphamide on days -3, -2, and -1.

Fludarabine and cyclophosphamide can be given on the same day or on different days.

In certain embodiments, fludarabine and cyclophosphamide can be administered simultaneously or sequentially.

Fludarabine and cyclophosphamide can be administered by any route (including intravenous drip (I.V.)). In some embodiments, fludarabine and cyclophosphamide can be administered in accordance with the instructions for fludarabine and cyclophosphamide.

In some embodiments, adoptive cell therapy or immune effector cell therapy is selected from the group consisting of tumor infiltrating lymphocyte (TIL) immunotherapy, autologous cell therapy, engineered autologous cell therapy (eACT), and allogeneic T cell transplantation. In some embodiments, immune effector cell therapy is the administration of chimeric antigen receptor modified T cells that target tumor antigens (such as BCMA).

In some embodiments, pretreatment includes administering fludarabine no more than about 50, 49, 48, 47, 46, 45, 44, 43, 42, 41, 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2, 1 mg/ m²/day; and/or administering cyclophosphamide no more than about 700, 690, 680, 670, 660, 650, 640, 630, 620, 610, 600, 595, 590, 585, 580, 579, 578, 576, 575, 574, 573, 572, 571, 570, 569, 568, 567, 566, 565, 564, 563, 562, 561, 560, 559, 558, 557, 556, 555, 554, 553, 552, 551, 550, 549, 548, 547, 546, 545, 544, 543, 542, 541, 540, 539, 538, 537, 536, 535, 534, 533, 532, 531, 530, 529, 528, 527, 526, 525, 524, 523, 522, 521, 520, 519, 518, 517, 516, 515, 514, 513, 512, 511, 510, 509, 508, 507, 506, 505, 504, 503, 502, 501, 500, 490, 480, 470, 460, 450, 440, 430, 420, 410, 400, 390, 380, 370, 360, 350, 340, 330, 320, 310, 300, 290, 280, 270, 260, 250, 240, 230, 220, 210, 200, 190, 180, 170, 160, 150, 140, 130, 120, 110, or 100 mg/ m²/day.

In some embodiments, the amount of fludarabine administered is about 10-50 mg/m²/day, or about 15-40 mg/m²/day, or about 15-35 mg/m²/day, or 15-30 mg/m²/day, or about 20-30 mg/m²/day.

In some embodiments, the amount of cyclophosphamide administered is about 100-700 mg/m²/day, or about 150-600 mg/m²/day, or about 190-600 mg/m²/day, or about 190-560 mg/m²/day.

In a preferred embodiment, the administration amount of cyclophosphamide is about 150-400 mg/m²/day, preferably, about 190-350 mg/m²/day.

In a preferred embodiment, the amount of cyclophosphamide administered is about 400-600 mg/m²/day, preferably about 450-600 mg/m²/day, more preferably about 450-560 mg/m²/day.

Various other interventions can be included in the methods described herein. For example, cyclophosphamide and fludarabine may cause adverse reactions in patients after administration. The scope of the present invention includes administering the composition to the patient to reduce some of these adverse events. In certain embodiments, the method comprises administering physiological saline to the patient. The patient may be given physiological saline before or after the administration of cyclophosphamide and/or fludarabine, or before and after administration of cyclophosphamide and/or fludarabine. In some embodiments, on each day of infusion, the patient is given physiological saline before and after the administration of cyclophosphamide and/or fludarabine. In addition, adjuvants and excipients can also be administered to patients. For example, Mesna (sodium 2-mercaptoethane sulfonate). In addition, exogenous cytohormones can also be given to patients.

In some embodiments, pretreatment prior to infusion of immune effector cells in a previous course or immune effector cells in a subsequent course improves the outcome of the treatment. For example, in some aspects, pretreatment improves the efficacy of the immune effector cells used in the previous course of treatment or the immune effector cells in the subsequent course of treatment, or increases the persistence of immune effector cells (such as CAR-T cells) in the subject. In some embodiments, the pretreatment treatment increases the stable stage of the disease.

Once the immune effector cells are administered to the subject, in some aspects, the sustained survival period of the immune effector cell population in the body and its biological activity are measured by any one of the variety of known methods. For example, the sustained survival period of CAR- T cells in the body refers to the sustained survival period of CAR- T cells after being "implanted" in the body. The sustained survival period of CAR-T cells can be measured from the end of the initial implantation, at each visit point, the Q-PCR method is used to detect the copy number of CAR DNA in the peripheral blood until any two consecutive tests are negative, and it is recorded as the sustained survival period of CAR-T cells.

The biological activity of immune effector cells can be evaluated by the specific binding of engineered or natural T cells or other immune cells to antigens, for example, by ELISA or flow cytometry.

In some embodiments, the cell killing ability of engineered immune effector cells can be detected by any suitable method known in the art, such as by measuring expression and/or secretion of certain cytokines, such as CD107a, IFNγ, IL-2 and TNF, to measure the biological activity of immune effector cells. In some aspects, biological activity is assessed by clinical results, such as reduction in tumor burden or load. In some aspects, the cells are assessed for their toxicity outcome, persistence and/or proliferation, and/or the presence or absence of a host immune response.

In the case of immune effector cell therapy, the administration of a given "dose" includes a single composition and/or a single uninterrupted administration, such as a single injection or continuous infusion of a given quantity or number of immune effector cells, and also includes that within a specific time period in no more than 30, 29, 28, 27, 26, 25, 24, 23, 22, 20, 19, 18, 17, 16, 15, 14, 13, 12, 11, 10, 9, 8, 7, 6, 5, 4, 3, or 2 days, in divided doses, provided in multiple separate compositions or infusions, a certain quantity or number of immune effector cells are given. Therefore, the immune effector cells for each course of treatment are given or started to be given in a single or continuous administrations of a specified number of immune effector cells at a single time point. However, in some cases, the immune effector cells of each course of treatment are injected or infused multiple in times within a period of no more than 30, 29, 28, 27, 26, 25, 24, 23, 22, 20, 19, 18, 17, 16, 15, 14, 13., 12, 11, 10, 9, 8, 7, 6, 5, 4, 3 or 2 days, such as once a day for three or two continuous days, or multiple infusions within 1 day. In some aspects, the immune effector cells in the previous course of treatment are administered as a single pharmaceutical composition.

With respect to the administration of the first course of treatment, the term "the subsequent course of treatment" refers to the dose given to the subject without any intervening doses being given to the subject during the period after the first course of treatment. Nevertheless, the term does not include the second, third, and/or other injections or infusions in a series of infusions or injections contained in a single divided dose. Therefore, unless otherwise stated, a second infusion within one, two, or three days is not considered a "the subsequent course of treatment" as used herein. Similarly, the second, third, and other doses in the multiple dose series within the divided doses are not considered "intervening" doses in the meaning of the dose of "the subsequent course of treatment". Therefore, unless otherwise specified, even if the subject receives the infusion of the second or subsequent course of treatment after the start of the first course of treatment, the dose given for a certain period of time exceeding 30 days after the start of the first course or the previous course of treatment is considered the dose of "the subsequent course of treatment". Unless otherwise specified, multiple administrations of the same immune effector cells are considered as a single dose for a period of up to 30 days, and administration of immune effector cells within 30 days after the initial administration (the day of the first infusion of each course of treatment (i.e., the initial dosing) is the first day) is not considered to be administered in a subsequent course of treatment, and is not considered to be an intervening doses for determining whether the second dose is "continuous" with the immune effector cells in the previous course of treatment.

As used herein, "the first course of treatment" is used to describe the total dose given in the first course of treatment according to the methods described herein. The dose is equal to the total dose administered in the course of treatment in a single course-administration, or the total dose administered in the first course of treatment in a multiple course-administration. The term does not necessarily mean that the subject has never received immune effector cell therapy before, or that the subject has not previously received immune effector cell therapy that targets the same antigen.

The dose of immune effector cells in the first course of treatment and/or immune effector cells in one or more of the subsequent courses of treatment is usually designed to provide improved efficacy and/or reduced risk of toxicity. In some embodiments, the dose of immune cells in each course of treatment is higher than, lower than or equal to about 0.5×10⁶cells/kg subject's body weight to 1×10 10 cells/kg subject's body weight, such as higher than, lower than or equal to about 0.6×10⁶, 0.7×10⁶, 0.8×10⁶, 0.9×10⁶, 1.0×10⁶, 1.1×10⁶, 1.2×10⁶, 1.3×10⁶, 1.4×10⁶, 1.5×10⁶, 1.6×10⁶, 1.7×10⁶, 1.8×10⁶, 1.9×10⁶, 2.0×10⁶, 2.1×10⁶, 2.2×10⁶, 2.3×10⁶, 2.4×10⁶, 2.5×10⁶, 2.6×10⁶, 2.7×10⁶, 2.8×10⁶, 2.9×10⁶, 3.0×10⁶, 3.1×10⁶, 3.2×10⁶, 3.3×10⁶, 3.4×10⁶, 3.5×10⁶, 3.6×10⁶, 3.7×10⁶, 3.8×10⁶, 3.9×10⁶, 4.0×10⁶, 4.1×10⁶, 4.2×10⁶, 4.3×10⁶, 4.4×10⁶, 4.5×10⁶, 4.6×10⁶, 4.7×10⁶, 4.8×10⁶, 4.9×10⁶, 5.0×10⁶, 5.1×10⁶, 5.2×10⁶, 5.3×10⁶, 5.4×10⁶, 5.5×10⁶, 5.6×10⁶, 5.7×10⁶, 5.8×10⁶, 5.9×10⁶, 6.0×10⁶, 6.1×10⁶, 6.2×10⁶, 6.3×10⁶, 6.4×10⁶ 6.5×10⁶, 6.6×10⁶, 6.7×10⁶, 6.8×10⁶, 6.9×10⁶, 7.0×10⁶, 7.1×10⁶, 7.2×10⁶, 7.3×10⁶, 7.4×10⁶ 7.5×10⁶, 7.6×10⁶, 7.7×10⁶, 7.8×10⁶, 7.9×10⁶, 8.0×10⁶, 8.1×10⁶, 8.2×10⁶, 8.3×10⁶, 8.4×10⁶, 8.5×10⁶, 8.6×10⁶, 8.7×10⁶, 8.8×10⁶, 8.9×10⁶, 9.0×10⁶, 9.1×10⁶, 9.2×10⁶, 9.3×10⁶, 9.4×10⁶, 9.5×10⁶, 9.6×10⁶, 9.7×10⁶, 9.8×10⁶, 9.9×10⁶, 1.0×10⁷ cells/kg subject's body weight. In some embodiments, the total dose of immune cells administered for each course of treatment is higher than, lower than or equal to about 0.1×10⁸ cells to 1×10¹⁰cells. Preferably, it is higher than, lower than or equal to about 1x 10⁶ cells/kg subject's body weight to 1x 10⁷ cells/kg subject's body weight, or the total dose of the immune effector cells administered is higher than, lower than or equal to about 0.5x 10⁸cells to 1x 10⁹ cells. More preferably, it is higher than, lower than or equal to about 1.5x 10⁶ cells/kg subject's body weight to 3x 10⁶ cells/kg subject's body weight, or the total dose of the immune effector cells administered is about 0.5x 10⁸cells to 5x 10⁸cells.

In a specific embodiment, the number of immune effector cells refers to the number of immune effector cells expressing chimeric antigen receptors, such as the number of CAR-T cells. In other embodiments, the number of immune effector cells can also refer to the number of T cells or PBMCs or total cells administered.

In some embodiments, the dose in the subsequent course of treatment is sufficient to reduce tumor burden or its indicators, and/or one or more symptoms of the disease or disorder.

In some embodiments, compared with that at the time before the first course of immune effector cells were administered, after the immune effector cells were administered, the tumor burden, such as the ratio of plasma cells in bone marrow, blood/urine M protein, and extramedullary soft tissue plasmacytoma, is reduced by approximately 25%, 30%, 35%, 40%, 45%, 50%, 55%, 60%, 65%, 70%, 75%, 80%, 85%, 90% or 100%.

M protein is a large amount of abnormal immunoglobulin produced in the malignant proliferation of monoclonal plasma cells or B lymphocytes.

Based on the teachings of the present invention, those skilled in the art should understand that the dose specifically disclosed in the present invention is a safe and effective dose obtained by the inventor through research. Those skilled in the art, such as clinicians, can determine the dose of immune effector cells in the previous course of treatment based on various actual conditions, such as the patient's tumor burden, the patient's own physical condition and other factors; if it is necessary to further administer immune effector cells in the subsequent course of treatment, those skilled in the art can determine the dose of immune effector cells in the subsequent course of treatment base on, for example, the change in tumor burden after the administration of immune effector cells.

In some embodiments, the dose of immune effector cells in each course of treatment includes an quantity that does not cause toxicity or reduces toxicity. The toxicity may be cytokine release syndrome (CRS), severe CRS (sCRS), macrophage activation syndrome, tumor lysis syndrome, fever of at least 38 degree Celsius or about 38 degree Celsius sustained for three or more days, CRP plasma level at least equal to about 20 mg/dL, and/or neurotoxicity. In some aspects, the cells number of the immune effector cells administered in the subsequent course of treatment is determined based on the possibility that the subject shows toxicity or toxicity results after administration of the cells. For example, in some embodiments, the possibility of developing a toxicity result in a subject is predicted based on tumor burden. In some embodiments, the method includes detecting or evaluating toxicity results and/or tumor burden before administering immune effector cells.

### Administration time

In some embodiments, the administration time of the subsequent course of treatment is calculated from the completion of the previous course of treatment (the total dose infusion completion date of the previous course of treatment is set as day 0).

In some embodiments, when the serum level of the factor indicative of CRS in the subject does not exceed about 10 times, 25 times, 50 times or 100 times the serum level of the indicator in the subject after administration of the immune effector cells of the previous course of treatment, the immune effector cells are administrated in the subsequent course of treatment.

In some embodiments, when the results related to CRS (for example, serum factors related to CRS or indicative of CRS) or its clinical signs or symptoms such as fever, hypoxia, hypotension, or neurological disorders in the subject have reached their peak levels, and the immune effector cells begin to decline after the administration of immune effector cells, the immune effector cells in the subsequent course of treatment are administered. In some embodiments, the immune effector cells in the subsequent course of treatment are administered when a decrease in comparison to the highest level of such results after administration is observed, or when the maximum value or level of the results is reached after administration and the level decreases.

In some embodiments, when the level of an indicator of toxicity results (for example, a serum indicator of CRS) drops below about 25 times the level of the indicator at the time before the immune effector cell administration in the previous course of treatment, the immune effector cells in the subsequent course of treatment are administrated. In some aspects, the immune effector cells in the subsequent course of treatment are administrated when the subject does not show CRS or does not show severe CRS.

In some aspects, the immune effector cells in the subsequent course of treatment are administered at a point of time when the tumor burden in the patient decreases compared to the tumor burden before the immune effector cells in the previous course of treatment are administered. In some embodiments, the immune effector cells in the subsequent course of treatment are administered after the administration of immune effector cells in the previous course of treatment, when the tumor burden such as the proportion of plasma cells in the bone marrow, blood/urine M protein, and extramedullary soft tissue plasmacytoma has been reduced by about 25%, 30%, 35%, 40%, 45 %, 50%, 55%, 60%, 65%, 70%, 75%, 80%, 85%, 90% or more.

In some embodiments, the immune effector cells in the subsequent course of treatment are administered after a reduction in the response to the immune effector cells of the previous course of treatment or the previous dose and when the subject's disease or condition has not recurred. In some embodiments, the reduction of tumor burden is indicated by a decrease in one or more indicators, such as classification, stage, the proportion of abnormal plasma cells in the bone marrow at the onset of illness, cytogenetic changes, whether there is extramedullary infiltration, and response after treatment. In some embodiments, the immune effector cells of the subsequent course of treatment are administered when the host's adaptive immune response is not detected, has not yet been established, or has not reached a certain level, degree, or stage. In some aspects, the immune effector cells of the subsequent course of treatment are administered before the subject's memory immune response develops.

In some aspects, the time between the administration of the immune effector cells in the previous course of treatment and the administration of the immune effector cells in the subsequent course of treatment is about 4 weeks to about 24 weeks.

In some embodiments, after the immune effector cells in the subsequent course are administered, immune effector cells in the additional or further subsequent course are administered, for example, the immune effector cells in the second subsequent course of treatment (dose of the third course of treatment), immune effector cells in the third subsequent course of treatment (dose of the fourth course of treatment), and so on.

In some embodiments, compared with that at the time before the administration of the immune effector cells in the previous course of treatment or the immune effector cells in the subsequent course of treatment, after administering immune effector cells in the subsequent course of treatment, the tumor burden is reduced at least equal to about 25%, 30%, 35%, 40%, 45%, 50%, 55%, 60%, 65%, 70%, 75%, 80%, 85%, 90% or more. In some embodiments, tumor burden can be assessed using serum, urine protein electrophoresis, bone marrow smear, bone marrow biopsy, MRD, MRI, and/or CT.

### Host immune response against reinfused cells

In some embodiments, one or more doses are administered, for example when immune effector cells in a subsequent course of treatment, are not present in the subject's immune response (such as adaptive or specific immune response to transgenic receptors or cells), and cannot be detected or cannot be detected above a certain level. The presence or extent of a specific immune response to a transgene is usually related to the immunogenicity of the recipient (e.g., CAR or transgenic TCR expressed by the cell) and/or the time the subject is exposed to the cells. In some embodiments, the immunity effector cells in the subsequent course of treatment are administered prior to an immune response to a chimeric antigen receptor or cell, an adaptive or specific immune response, a detectable immune response and/or a memory response has been developed in the subject. In this regard, compared with other methods of administering immune effector cells of the subsequent course of treatment at a later time point, the immune effector cells' proliferation and/or ability to persist in the subject is improved in the subsequent course of treatment compared with that of the immune effector cells of the previous or previous course of treatment. In some embodiments, the decision of when and/or whether to administer immune effector cells in a subsequent course of treatment depends on whether the subject exhibits such an immune response or its detectable readings, for example a detectable specificity or adaptive host immune response specific to cells or chimeric antigen receptors, for example CAR expressed by immune effector cells of the previous course of treatment, and/or whether such a response is detected at a certain level. In some embodiments, when such a reaction is detected, the subject is not administered with immune effector cells in the subsequent course of treatment. When the subject does not exhibit a specific or adaptive (e.g., humoral or cell-mediated) immune response to the receptor (e.g., CAR expressed by the immune effector cells in the previous course of treatment), or does not exhibit a detectable level or higher than an acceptable level of such a response or indicator, immune effector cells in the subsequent course of treatment are administered. In some aspects, when the immune effector cells of the subsequent course of treatment are administered, the subject exhibits reduced humoral or cell-mediated immune response against the CAR expressed by the immune effector cells in the previous course of treatment compared to that when the initial dose is larger.

### Cell persistence

In some embodiments, the provided methods increase the durability of the subject to the administered immune effector cells, for example, the increased number or duration of cells over time, and/or the improvement of the efficacy and treatment results in immune cell therapy. In some aspects, the advantage of this method is that, compared with other methods, cells expressing chimeric antigen receptors (such as CAR-T cells) can improve treatment results to a greater and/or longer extent. These results can include patient survival and remission, even in subjects with severe tumor burden.

In some embodiments, the presence and/or quantity of cells expressing chimeric antigen receptors (e.g., CAR-expressing cells) in the subject after the immune effector cells in the previous course of treatment and/or the immune effector cells in the subsequent course of treatment are detected. In some aspects, quantitative PCR (qPCR) is used to assess the quantity of cells expressing chimeric antigen receptors (e.g., CAR-T) in the subject's blood or serum or organs or tissues (e.g., disease sites). In some aspects, persistence is quantified as receptors encoded in each microgram of DNA, for example the copies of DNA or plasmid of CAR, or as in each microgram of samples, for example, the expression of receptors in blood or serum, for example the quantity of cells expressing CAR, or the total number of peripheral blood mononuclear cells (PBMC) or white blood cells or T cells per microliter sample.

In some embodiments, after administration of the immune effector cells in the previous course of treatment or at least on the Day 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30, the cells are detected in the subject. In some aspects, in or at least 2, 4, or 6 weeks after administration of the immune effector cells in the first or subsequent course of treatment, or 3, 6 or 12, 18 or 24, or 30 or 36 months, or 1, 2, 3, 4, 5 or more years, the cells are detected.

In some embodiments, the method results in a maximum concentration of at least 10, 20, 30, 40, 50, 60, 70, 80, 90, 100, 500, 1000, 1500, 2000, 5000, 10000, or 15,000 copies or receptor-encoding nucleic acids in the blood or serum or other body fluids or organs or tissues of the subject, such as CAR per microgram of DNA, or at least 0.1, 0.2, 0.3, 0.4, 0.5, 0.6, 0.7, 0.8 or 0.9 receptor expression, for example total number CAR-expressing cells/peripheral blood mononuclear cells (PBMCs), total number of monocytes, total number of T cells or total microliters. In some embodiments, the receptor-expressing cells are detected as at least 10%, 20%, 30%, 40%, 50%, or 60% of the total PBMCs in the blood of the subject, and/or at this level for at least 1, 2, 3, 4, 5, 6, 7, 8, 9, 10, 11, 12, 24, 36, 48, or 52 weeks after the first or subsequent administration, or for 1, 2, 3, 4, or 5 years or more after such administration.

In some aspects, the method results in, for example, an at least 2-fold, at least 4-fold, at least 10-fold, or at least 20-fold increase in copies of a nucleic acid encoding a chimeric antigen receptor, such as a CAR, in the subject's serum per microgram of DNA.

In some embodiments, the receptor-expressing cells can be detected in the blood or serum of the subject, for example, by a designated method, such as qPCR or a detection method based on flow cytometry, after administration of the immune effector cells of the previous course of treatment or at least 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59 or 60 or more days after the administration of the immune effector cells in the subsequent course of treatment, or after the administration of immune effector cells in the previous course of treatment or the immune effector cells in the subsequent course of treatment for at least or about 2, 3, 4, 5, 6, 7, 8, 9, 10, 11, 12, 13, 14, 15, 16, 17, 18, 19, 20, 21, 22, 23, or 24 or more weeks.

In some aspects, measured by immunohistochemistry, PCR, and/or flow cytometry, the number of copies of the nucleic acid encoding the chimeric antigen receptor per 100 cells, such as the vector copy number, for example, in peripheral blood or bone marrow or other compartments, it is at least 0.01, at least 0.1, at least 1, or at least 10, after administering the cells, for example, about 1 week, about 2 weeks, about 3 weeks, about 4 weeks, about 5 weeks, or at least about 6 weeks or at least about 2, 3, 4, 5, 6, 7, 8, 9, 10, 11, or 12 months, or at least 2 or 3 years after the immune effector cells in the previous course of treatment or the immune effector cells in the subsequent course of treatment.

### Cells expressing chimeric antigen receptors

The immune effector cells express a chimeric antigen receptor that recognizes BCMA or its variants. The chimeric antigen receptor (CAR) usually includes an extracellular antigen binding domain, such as a part of an antibody molecule, which is usually a variable heavy (VH) chain region and/or a variable light (VL) chain region of an antibody, for example, scFv antibody fragments.

In some embodiments, the heavy chain variable region and the light chain variable region of the antibody comprise: the HCDR1 shown in SEQ ID NO: 1, the HCDR2 shown in SEQ ID NO: 2, and the HCDR3 shown in SEQ ID NO: 3; and the LCDR1 shown in SEQ ID NO: 6, the LCDR2 shown in SEQ ID NO: 7, and the LCDR3 shown in SEQ ID NO: 8, or an amino acid sequence having at least 85%, 86%, 87%, 88%, 89%, 90%, 91%, 92%, 93%, 94%, 95%, 96%, 97%, 98%, 99% identity to the above sequences; or the HCDR1 shown in SEQ ID NO: 1, the HCDR2 shown in SEQ ID NO: 2, and the HCDR3 shown in SEQ ID NO: 4; and the LCDR1 shown in SEQ ID NO: 6, the LCDR2 shown in SEQ ID NO: 7, and the LCDR3 shown in SEQ ID NO: 9 LCDR3, or an amino acid sequence having at least 85%, 86%, 87%, 88%, 89%, 90%, 91%, 92%, 93%, 94%, 95%, 96%, 97%, 98%, 99% identity to the above sequences; or the HCDR1 shown in SEQ ID NO: 1, the HCDR2 shown in SEQ ID NO: 2, and the HCDR3 shown in SEQ ID NO: 5; and the LCDR1 shown in SEQ ID NO: 6, the LCDR2 shown in ID NO: 7, and the LCDR3 shown in SEQ ID NO: 10, or an amino acid sequence having at least 85%, 86%, 87%, 88%, 89%, 90%, 91%, 92%, 93 %, 94%, 95%, 96%, 97%, 98%, 99% identity to the above sequences; or the HCDR1 shown in SEQ ID NO: 11, the HCDR2 shown in SEQ ID NO: 12, the HCDR3 shown in SEQ ID NO: 5; and the LCDR1 shown in SEQ ID NO: 6, the LCDR2 shown in SEQ ID NO: 7, the LCDR3 shown in SEQ ID NO: 10, or an amino acid sequence having at least 85%, 86%, 87%, 88%, 89%, 90%, 91%, 92%, 93%, 94%, 95%, 96%, 97%, 98%, 99% identity to the above sequences; or the HCDR1 shown in SEQ ID NO: 13, the HCDR2 shown in SEQ ID NO: 14, and the HCDR3 shown in SEQ ID NO: 5; and the LCDR1 shown in SEQ ID NO: 6, the LCDR2 shown in SEQ ID NO: 7 and the LCDR3 shown in SEQ ID NO: 10, or an amino acid sequence having at least 85%, 86%, 87%, 88%, 89%, 90%, 91%, 92%, 93%, 94%, 95%, 96%, 97%, 98%, 99% identity to the above sequences. In a specific embodiment, the heavy chain variable region and the light chain variable region of the antibody comprise the HCDR1 shown in SEQ ID NO: 11, the HCDR2 shown in SEQ ID NO: 12, the HCDR3 shown in SEQ ID NO: 5; and the LCDR1 shown in SEQ ID NO: 6, the LCDR2 shown in SEQ ID NO: 7, the LCDR3 shown in SEQ ID NO: 10, or an amino acid sequence having at least 85%, 86%, 87%, 88%, 89%, 90%, 91%, 92%, 93%, 94%, 95%, 96%, 97%, 98%, 99% identity to the above sequences.

In some embodiments, the light chain variable region of the antibody comprises the LCDR1 shown in SEQ ID NO: 6, the LCDR2 shown in SEQ ID NO: 7, and the LCDR3 shown in SEQ ID NO: 10, or an amino acid sequence having at least 85%, 86%, 87%, 88%, 89%, 90%, 91%, 92%, 93%, 94%, 95%, 96%, 97%, 98%, 99% identity to the above sequences. In a preferred embodiment, the light chain variable region of the antibody comprises the amino acid sequence shown in SEQ ID NO: 20, or an amino acid sequence having at least 85%, 86%, 87%, 88%, 89%, 90%, 91%, 92%, 93%, 94%, 95%, 96%, 97%, 98%, 99% identity to the above sequences. In a preferred example, the heavy chain variable region of the antibody comprises the HCDR3 shown in SEQ ID NO: 5, and the light chain variable region of the amino acid sequence shown in SEQ ID NO: 20, or an amino acid sequence having at least 85%, 86%, 87%, 88%, 89%, 90%, 91%, 92%, 93%, 94%, 95%, 96%, 97%, 98%, 99% identity to the above sequences.

In some embodiments, the heavy chain variable region of the antibody comprises the amino acid sequence shown in SEQ ID NO: 15 and the light chain variable region of the antibody comprises the amino acid sequence shown in SEQ ID NO: 16, or an amino acid sequence having at least 85%, 86%, 87%, 88%, 89%, 90%, 91%, 92%, 93%, 94%, 95%, 96%, 97%, 98%, 99% identity to the above sequences; or the heavy chain variable region of the antibody comprises the amino acid sequence shown in SEQ ID NO: 17 and the light chain variable region of the antibody comprises the amino acid sequence shown in SEQ ID NO: 18, or an amino acid sequence having at least 85%, 86%, 87%, 88%, 89%, 90%, 91%, 92%, 93%, 94%, 95%, 96%, 97%, 98%, 99% identity to the above sequences; or the heavy chain variable region of the antibody comprises the amino acid sequence shown in SEQ ID NO: 19 and the light chain variable region of the antibody comprises the amino acid sequence shown in SEQ ID NO: 20, or an amino acid sequence having at least 85%, 86%, 87%, 88%, 89%, 90%, 91%, 92%, 93%, 94%, 95%, 96%, 97%, 98%, 99% identity to the above sequences; or the heavy chain variable region of the antibody comprises the amino acid sequence shown in SEQ ID NO: 21 and the light chain variable region of the antibody comprises the amino acid sequence shown in SEQ ID NO: 20, or an amino acid sequence having at least 85%, 86%, 87%, 88%, 89%, 90%, 91%, 92%, 93%, 94%, 95%, 96%, 97%, 98%, 99% identity to the above sequences; or the heavy chain variable region of the antibody comprises the amino acid sequence shown in SEQ ID NO: 21 and the light chain variable region of the antibody comprises the amino acid sequence shown in SEQ ID NO: 20, or an amino acid sequence having at least 85%, 86%, 87%, 88%, 89%, 90%, 91%, 92%, 93%, 94%, 95%, 96%, 97%, 98%, 99% identity to the above sequences. In a specific embodiment, the heavy chain variable region of the antibody comprises the amino acid sequence shown in SEQ ID NO: 21 and the light chain variable region of the antibody comprises the amino acid sequence shown in SEQ ID NO: 20, or an amino acid sequence having at least 85%, 86%, 87%, 88%, 89%, 90%, 91%, 92%, 93%, 94%, 95%, 96%, 97%, 98%, 99% identity to the above sequences.

In some embodiments, the antibody has a sequence of the scFv shown in SEQ ID NO: 25, 27, or 29, or an amino acid sequence having at least 85%, 86%, 87%, 88%, 89%, 90%, 91%, 92%, 93%, 94%, 95%, 96%, 97%, 98%, 99% identity to the above sequences.

In some embodiments, the chimeric antigen receptor comprises the amino acid sequence shown in SEQ ID NO: 30, 31, 32, 33, 34, 35, 36, 37, 38, 39, 40, or 41, or an amino acid sequence having at least 85%, 86%, 87%, 88%, 89%, 90%, 91%, 92%, 93%, 94%, 95%, 96%, 97%, 98%, 99% identity to the above sequences. In a specific embodiment, the chimeric antigen receptor comprises an amino acid sequence shown in any one of SEQ ID NO: 36, 37, 38, or an amino acid sequence having at least 85%, 86%, 87%, 88%, 89%, 90%, 91%, 92%, 93%, 94%, 95%, 96%, 97%, 98%, 99% identity to the above sequences. In a preferred example, the chimeric antigen receptor comprises the amino acid sequence shown in SEQ ID NO: 36, or an amino acid sequence having at least 85%, 86%, 87%, 88%, 89%, 90%, 91%, 92%, 93%, 94%, 95%, 96%, 97%, 98%, 99% identity to the above sequences.

In some embodiments, the antibody portion of the chimeric antigen receptor (eg, CAR) also includes a linking sequence, which may be or include at least a portion of an immunoglobulin constant region or a variant or modified form thereof, such as a hinge region, such as, IgG4 hinge region and/or CH1/CL and/or Fc region. In some embodiments, the constant region or portion is of human IgG, such as IgG4 or IgG1.

The antigen recognition domain is generally connected to one or more intracellular signal transduction moieties, for example, in the case of CAR, the activated signal transduction moiety is simulated by the antigen receptor complex (such as TCR complex), and/or by the signal from another cell surface receptor. Therefore, in some embodiments, the antigen binding component (eg, antibody) is linked to one or more transmembrane and intracellular signal transduction domains. In some embodiments, the transmembrane domain is fused to the extracellular domain. In one embodiment, the transmembrane domain of one of the domains in the naturally associated receptor (eg, CAR) is used. In some cases, the transmembrane domain is selected or modified by amino acid substitutions to prevent the domain from binding to the transmembrane domain of the same or different surface membrane protein, so that minimizing interaction with other members of the receptor complex.

In some embodiments, the transmembrane domain is derived from natural or synthetic sources. When the source is a natural source, in some aspects, the domain is derived from any membrane-bound or transmembrane protein. The transmembrane region includes α, β or ζ chains derived from T-cell receptors, CD28, CD3ε, CD45, CD4, CD5, CD8, CD9, CD 16, CD22, CD33, CD37, CD64, CD80, CD86, CD 134, CD137, CD 154, and/or transmembrane regions include those functional variants thereof (e.g., those that substantially retain their structural parts (e.g., transmembrane structural parts), properties) (i.e., include at least their transmembrane regions). In some embodiments, the transmembrane domain is a transmembrane domain derived from CD4, CD28, or CD8, for example, CD8α or a functional variant thereof. Alternatively, in some embodiments, the transmembrane domain is synthetic. In some aspects, the synthetic transmembrane domain mainly contains hydrophobic residues such as leucine and valine. In some aspects, trimers of phenylalanine, tryptophan, and valine will present at each end of the synthetic transmembrane domain. In some embodiments, the connection occurs through linkers, spacers, and/or transmembrane domains.

The intracellular signal transduction domains include those that mimic or approximate signals through natural antigen receptors, signals through such receptors in combination with co-stimulatory receptors, and/or signals through individual co-stimulatory receptors. In some embodiments, a short oligopeptide or polypeptide linker is present, for example, a linker with a length of 2-10 amino acids, such as a linker containing glycine and serine, for example, a glycine-serine doublet, and a connection between the cytoplasmic signal transduction domain and the transmembrane domain of CAR is formed.

The receptor, for example, CAR, generally includes at least one of one or more intracellular signal transduction moieties. In some embodiments, the receptor includes the intracellular component of the TCR complex, such as the TCRCD3+ chain that mediates T-cell activation and cytotoxicity, for example, the CD3ζ chain. Therefore, in some aspects, the antigen binding moiety is connected to one or more cell signal transduction modules. In some embodiments, the cell signal transduction module includes a CD3 transmembrane domain, a CD3 intracellular signal transduction domain, and/or other CD transmembrane domains. In some embodiments, the receptor, for example, CAR, also includes one or more portions of other molecules, such as Fc receptor gamma, CD8, CD4, CD25, or CD16. For example, in some aspects, CAR or other chimeric antigen receptors include chimeric molecules between CD3ζ (CDS-ζ) or Fc receptor γ and CD8, CD4, CD25, or CD16.

In some embodiments, when the CAR or other chimeric antigen receptors are bound, the cytoplasmic domain or intracellular signal transduction domain of the receptor activates at least one of the normal effector function or response of immune cells, such as engineered T cells modified to express CAR. For example, in some cases, CARs induce T cell functions, such as cytolytic activity or helper T cell activity, such as the secretion of cytokines or other factors. In some embodiments, a truncated portion of the intracellular signal transduction domain of an antigen receptor portion or a costimulatory molecule is used to replace the complete immunostimulatory chain, for example, if it transduces effector function signals. In some embodiments, the intracellular signal transduction domain includes the cytoplasmic sequence of the T cell receptor (TCR), and in some aspects, it also includes those co-receptors that exist in nature that act in concert with these receptors to initiate signal transduction after antigen-receptor binding.

In other embodiments, the CAR does not include components used to generate costimulatory signals. In some aspects, other CARs are expressed in the same cell and provide components for generating a second or costimulatory signal.

In some aspects, T cell activation is described as being mediated by two types of cytoplasmic signal transduction sequences: those that initiate antigen-dependent first activation via TCR (the first cytoplasmic signal transduction sequence), and those that act in an antigen-independent manner to provide a second or costimulatory signal (second cytoplasmic signal transduction sequence). In some aspects, the CAR includes one or both of such signal transduction components.

In some embodiments, the antibody portion of the chimeric antigen receptor (e.g., CAR) also includes a signal peptide, such as a signal peptide including CD8 or a variant thereof, for example, including the amino acid sequence shown in SEQ ID NO: 35, or an amino acid sequence having at least 85%, 86%, 87%, 88%, 89%, 90%, 91%, 92%, 93%, 94%, 95%, 96%, 97%, 98%, 99% or more sequence identity to SEQ ID NO: 35.

In some aspects, the CAR includes a major cytoplasmic signal transduction sequence that regulates the initial activation of the TCR complex. The first cytoplasmic signal transduction sequence that acts in a stimulating manner may contain a signal transduction motif, which is known as activation motif of an immune receptor based on tyrosine or an ITAM. Examples of ITAM include first cytoplasmic signal transduction sequences, which include those derived from TCRζ, FcRγ, FcRβ, CD3γ, CD3δ, CD3ε, CDS, CD22, CD79a, CD79b, and CD66d. In some embodiments, the cytoplasmic signal transduction molecule in the CAR comprises a cytoplasmic signal transduction domain, partly derived or derived from CD3ζ sequence.

In some embodiments, the CAR includes the transmembrane portion and/or signal transduction domain of a costimulatory receptor, such as CD28, CD137, OX40, DAP10, and ICOS. In some aspects, the same CAR includes both activation and costimulatory components.

In some embodiments, the activation domain is included in one CAR, and the costimulatory component is provided by another CAR that recognizes another antigen. In some embodiments, the CAR includes activating or stimulating CAR and co-stimulating CAR, all of which are expressed on the same cell (see WO2014/055668). In some aspects, the cell includes one or more stimulating or activating CAR and/or costimulating CAR. In some embodiments, the cell also includes an inhibitory CAR (iCAR, see Fedorov et al., Sci. Transl. Medicine, 5(215) (December 2013)), for example, a CAR identifying that other than those related and/or specific to diseases or conditions, thereby, the activation signal delivered by the disease-targeted CAR is reduced or inhibited by the binding of the inhibitory CAR to its ligand, such as reducing off-target effects.

In some embodiments, the intracellular signal transduction portion of the chimeric antigen receptor, such as a CAR, comprises a CD3ζ intracellular domain and a costimulatory signal transduction region. In certain embodiments, the intracellular signal transduction domain comprises a CD28 transmembrane and signal transduction domain, which is connected to the CD3 (e.g., CDS-ζ) intracellular domain. In some embodiments, the intracellular signal transduction domain comprises a chimeric CD28 and/or CD137 (4-1BB, TNFRSF9) costimulatory domain, which is linked to the CD3ζ intracellular domain.

In some embodiments, the CAR encompasses one or more, for example, two or more, costimulatory domains and activation domains, for example, the initial activation domain in the cytoplasmic portion. Exemplary CARs include the intracellular portion of CDS-ζ, CD28, and CD137.

In some cases, CARs are referred to as first, second and/or third generation CARs. In some aspects, the first-generation CAR is a CAR that only provides CD3 chain inducing signals upon antigen binding; in some aspects, the second-generation CAR is a CAR that provides such signals and co-stimulatory signals, for example, including those derived from the intracellular signal transduction domain of co-stimulatory receptors (for example, CD28 or CD137); in some aspects, the third-generation CAR is a CAR that includes multiple costimulatory domains of different costimulatory receptors.

In some embodiments, the chimeric antigen receptor includes an extracellular portion containing an antibody or antibody fragment. In some aspects, the chimeric antigen receptor includes an extracellular portion containing an antibody or fragment and an intracellular signal transduction domain. In some embodiments, the antibody or fragment includes scFv, and the intracellular domain includes ITAM. In some aspects, the intracellular signal transduction domain includes the signal transduction domain of the zeta chain of the CDS-ζ chain. In some embodiments, the chimeric antigen receptor includes a transmembrane domain that connects the extracellular domain and the intracellular signal transduction domain. In some aspects, the transmembrane domain comprises the transmembrane portion of CD28. In some embodiments, the chimeric antigen receptor contains the intracellular domain of a T cell costimulatory molecule. The extracellular domain and the transmembrane domain can be directly or indirectly connected. In some embodiments, the extracellular domain and transmembrane are connnected by a linking sequence. In some embodiments, the receptor comprises the extracellular portion of the molecule from which the transmembrane domain is derived, such as the CD28 extracellular portion. In some embodiments, the chimeric antigen receptor comprises an intracellular domain derived from a T cell costimulatory molecule or a functional variant thereof, for example between a transmembrane domain and an intracellular signal transduction domain. In some aspects, the T cell costimulatory molecule is CD28 or 41BB. For example, in some embodiments, the CAR contains an antibody, such as an antibody fragment, is or contains a transduction domain containing a transmembrane portion of CD28 or functional variant thereof, and the intracellular signal transduction domain containing the signal transduction portion of CD28 or functional variant thereof, and the signal transduction portion of CD3ζ or functional variant thereof. In some embodiments, the CAR contains an antibody, such as an antibody fragment, which comprises or contains a transduction domain containing the transmembrane portion of CD28 or a functional variant thereof, and an intracellular signal transduction domain containing a signal transduction portion of CD137 or a functional variant thereof, and the signal transduction portion of CD3ζ or functional variants thereof. In some of the embodiments, the receptor further includes a linking sequence that includes a portion of an Ig molecule (e.g., a human Ig molecule), such as an Ig hinge, such as an IgG4 hinge, such as only an hinge linking sequence. In some embodiments, the chimeric antigen receptor comprises: (i) antibodies that specifically recognize tumor antigens, CD28 or CD8 transmembrane regions, CD28 costimulatory signal domains, and CD3ζ; or (ii) antibodies that specifically recognize tumor antigens, the transmembrane region of CD28 or CD8, the costimulatory signal domain of CD137, and CD3ζ; or (iii) antibodies that specifically recognize tumor antigens, the transmembrane region of CD28 or CD8, and the costimulatory signal domain of CD28, the costimulatory signal domain of CD137 and CD3ζ.

For example, in some embodiments, the CAR includes antibodies, such as antibody fragments, including scFv, linking sequence, for example a linking sequence containing a portion of an immunoglobulin molecule, such as a hinge region and/or one or more heavy chain constant regions, for example, a linking sequence containing an Ig-hinge, a transmembrane domain containing all or part of a CD28-derived transmembrane domain, a CD28-derived intracellular signal domain and a CD3ζ signal transduction domain. In some embodiments, the CAR includes antibodies or fragments, such as scFv, linking sequences, such as any linking sequence containing Ig-hinge, CD28-derived transmembrane domain, CD137-derived intracellular signal transduction domain and CD3ζ-derived signal transduction domain.

In some embodiments, the methods described herein include administering an adoptive cell or immune effector cell to the subject. In some embodiments, the methods described herein include administering to the subject two or more adoptive cells or immune effector cells directed against the same tumor antigen in different courses of treatment. In some embodiments, the methods described herein include administering two or more kinds of adoptive cells or immune effector cells against different tumor antigens to the subject in different courses of treatment. In some embodiments, the methods described herein include administering two or more types of adoptive cells or immune effector cells against the same epitope of the same tumor antigen to the subject in different courses of treatment. In some embodiments, the methods described herein include administering to the subject two or more adoptive cells or immune effector cells against different epitopes of the same tumor antigen in different courses of treatment. In some embodiments, the methods described herein include administering two or more types of adoptive cells or immune effector cells to the subject in different courses of treatment to treat tumors at the same site. In some embodiments, the methods described herein include administering two or more kinds of adoptive cells or immune effector cells to the subject in different courses of treatment to treat tumors at different sites. In some embodiments, at least one of the adoptive cells or immune effector cells used in the methods described herein is BCMA-targeting CAR-T cells. In some embodiments, at least one of the adoptive cells or immune effector cells used in the methods described herein is the BCMA-targeting CAR-T cells described herein. In the above cases, those skilled in the art, such as clinicians, can decide the number of administrations and doses of the BCMA-CAR-T cells of the present invention according to the previous treatment.

### Immune effector cells

The cells provided in the methods described herein are immune effector cells expressing chimeric antigen receptors. The cells are generally mammalian cells, and generally human cells. In some embodiments, the cells are derived from the blood, bone marrow, lymph, or lymphoid organs, and are cells of the immune system, such as cells of innate or adaptive immunity, for example, bone marrow or lymphoid cells, including lymphocytes, generally T cells and / or NK cells. Other exemplary cells include stem cells, such as multipotent and pluripotent stem cells, including induced pluripotent stem cells (iPSCs). The cells are generally primary cells, such as those cells isolated directly from the subject and/or frozen and isolated from the subject. In some embodiments, the cells include one or more subgroups of T cells or other cell types, such as whole T cell populations, CD4+ cells, CD8+ cells and subgroups thereof, such as those defined by function, activation state, maturation, differentiation potential, expansion, recycling, localization, and/or persistence, antigen specificity, antigen receptor type, presence in a specific organ or compartment, marker or cytokine secretion profile, and/or degree of differentiation. As to the subject to be treated, the cells may be allogeneic and/or autologous. Methods include existing methods. In some aspects, as with existing technologies, the cells are pluripotent and/or multipotent, such as stem cells, such as induced pluripotent stem cells (iPSCs). In some embodiments, the method includes isolating cells from the subject, preparing, processing, culturing, and/or engineering them, and reintroducing them into the same patient before or after cryopreservation.

The subtypes and subgroups of T cells and/or CD4+ and/or CD8+ T cells include: naive T (TN) cells, effector T cells (TEFF), memory T cells and their subtypes, such as stem cell-like memory T (TSCM), central memory T (TCM), effector memory T (TEM), or terminally differentiated effector memory T cells, tumor-infiltrating lymphocytes (TILs), immature T cells, mature T cells, helper T cells, cytotoxic T cells, mucosal-associated non-variant T (MAIT) cells, naturally occurring and adoptive regulatory T (Treg) cells, helper T cells, such as TH1 cells, TH2 cells, TH3 cells, TH17 cells, TH9 cells, TH22 cells, follicular helper T cells, α/β T cells, and δ/γ T cells.

In some embodiments, the cell is a natural killer (NK) cell. In some embodiments, the cells are monocytes or granulocytes, for example, bone marrow cells, macrophages, neutrophils, dendritic cells, mast cells, eosinophils, and/or basophils granulocyte.

In some embodiments, the cell includes one or more nucleic acids introduced by genetic engineering, and thereby expresses a recombinant or genetically engineered product of the nucleic acid. In some embodiments, the nucleic acid is heterologous, that is, it is not normally present in a cell or a sample obtained from the cell, such as a sample obtained from another organism or cell, such as those not normally found in the engineered cells and/or the organisms in which the cells are derived. In some embodiments, the nucleic acid is not naturally occurring, for example, the nucleic acid is not found in nature, including chimeric combinations comprising nucleic acids encoding various domains from multiple different cell types.

### Methods and vectors for genetic engineering

The invention also provides methods, compositions and kits for producing genetically engineered cells expressing chimeric antigen receptors. Genetic engineering generally involves introducing the nucleic acid encoding the recombinant or engineered part into a cell, such as introducing into the cell through virus transduction, electroporation, or the like.

In some embodiments, gene transfer is performed by: first, stimulating the cell, for example, by combining it with a stimulus that induces a response, such as proliferation, survival, and/or activation, for example, detected by cytokine or the expression of activation markers, and then transducing the activated cells and expanding them in culture to a sufficient number for clinical application.

In some aspects, the cells are also engineered to promote the expression of cytokines or other factors. Various methods for introducing genetically engineered components, for example, antigen receptors (e.g., CARs) are well known, and the methods and compositions provided herein can be used. Exemplary methods include those used to transfer the nucleic acid encoding the receptor, including via virus, for example, retrovirus or lentivirus, transduction, transposon, and electroporation.

In some embodiments, recombinant infectious virus particles are used to transfer recombinant nucleic acid into cells, for example, vectors derived from simian virus 40 (SV40), adenovirus, adeno-associated virus (AAV). In some embodiments, recombinant lentiviral vectors or retroviral vectors, such as gamma-retroviral vectors, are used to transfer recombinant nucleic acids into T cells.

### Preparation of immune effector cells

In some embodiments, the preparation of engineered cells includes one or more types of cultures and/or one or more preparation steps. Cells used to be introduced with nucleic acid encoding a transgenic receptor (e.g., CAR) can be isolated from a sample (e.g., a biological sample, such as a sample obtained or derived from a subject). In some embodiments, the subject from which the cells are isolated is a subject suffering from a certain disease or condition or in need of cell therapy or to be given cell therapy. In some embodiments, the subject is a person in need of specific therapeutic intervention, for example, a person in need of adoptive cell therapy or effector cell therapy, the cells used for the therapy are isolated, processed, and/or engineered . In some embodiments, the cell is a primary cell, for example, a primary human cell. The sample includes tissues, body fluids and other samples taken directly from the subject, as well as samples obtained from one or more processing steps, such as separation, centrifugation, genetic engineering (such as transduction with viral vectors), washing and/or incubating. The biological sample may be a sample obtained directly from a biological source or a processed sample. The biological samples include, but are not limited to, body fluids such as blood, plasma, serum, cerebrospinal fluid, synovial fluid, urine and sweat, tissue and organ samples, including processed samples derived from them.

Exemplary samples include whole blood, peripheral blood mononuclear cells (PBMCs), white blood cells, bone marrow, thymus, tissue biopsy, tumor, leukemia, lymphoma, lymph nodes, intestinal-associated lymphoid tissue, mucosal-associated lymphoid tissue, spleen, other lymphoid tissues, liver, lung, stomach, intestine, colon, kidney, pancreas, breast, bone, prostate, cervix, testis, ovary, tonsil or other organs, and/or cells derived therefrom. In the case of cell therapy (e.g., adoptive cell therapy or immune effector cell therapy), samples include samples from autologous and allogeneic sources.

In some embodiments, the separation of cells includes one or more preparation and/or non-affinity-based cell separation steps. In some examples, cells are washed, centrifuged and/or incubated in the presence of one or more substances, for example, to remove unwanted components, enrich required components, lyse or remove cells that are sensitive to specific substances. In some examples, cells are separated based on one or more properties, such as density, adhesion properties, size, sensitivity and/or resistance to specific components. In some examples, cells from the circulating blood of the subject are obtained, for example, by apheresis or leukapheresis. In some aspects, the sample includes lymphocytes, including T cells, monocytes, granulocytes, B cells, other nucleated white blood cells, red blood cells, and/or platelets.

In some embodiments, the blood cells collected from the subject are washed, for example, to remove the plasma fraction, and the cells are placed in a suitable buffer or medium for subsequent processing steps. In some embodiments, the cells are washed with phosphate buffered saline (PBS). In some embodiments, the cleaning solution lacks calcium and/or magnesium and/or many or all divalent cations. In some aspects, the washing step is performed by semi-automated "flow-through" centrifugation (for example, COBE 2991 cell processor, BaXter) according to the manufacturer's instructions. In some aspects, the cleaning step is performed by tangential flow filtration (TFF) according to the manufacturer's instructions. In some embodiments, after washing, the cells are resuspended in various biocompatible buffers, for example, Ca++/Mg++-free PBS. In some embodiments, blood cell sample components are removed and the cells are directly resuspended in the culture medium.

In some embodiments, the method includes a density-based cell separation method, for example, peripheral blood mononuclear cells (PBMCs) are obtained by lysing red blood cells or not lysing red blood cells and gradient centrifuging peripheral blood or single samples or leukapheresis samples by Percoll or Ficoll.

In some embodiments, the separation method includes separating different cell types based on the expression or presence of one or more specific molecules in the cell, such as surface markers, for example, surface proteins, intracellular markers, or nucleic acids. In some embodiments, any known method of separation based on such markers can be used. In some embodiments, the separation is based on affinity or based on immunoaffinity. For example, in some aspects, the separation includes separating cells and cell populations based on the expression or expression levels of one or more markers of the cells (usually cell surface markers), for example, by incubating with antibodies or binding partners that specifically bind to such markers,, usually followed by a washing step, and cells that have bound the antibody or binding partner are separated from those cells that have not yet bound to the antibody or binding partner.

Such separation steps may be based on positive selection, where cells that have bound the reagents are retained for further use, and/or, negative selection, where cells that have not yet bound to the antibody or binding partner are retained. In some examples, both parts are reserved for further applications. In some aspects, negative selection may be particularly useful when there are no antibodies that can be used to specifically identify cell types in a heterogeneous population, so that separation is most preferably based on markers expressed by cells different from the desired population..

The separation need not result in 100% enrichment or removal of specific cell populations or cells expressing specific markers. For example, positive selection or enrichment of specific types of cells, such as those expressing a marker, refers to increasing the number or percentage of said cells, but does not need to result in the complete absence of cells that do not express the marker. Likewise, negative selection, removal or depletion of specific types of cells, such as those expressing markers, refers to reducing the number or percentage of said cells, but need not result in the complete removal of all such cells.

In some examples, multiple rounds of separation steps are performed, wherein the positive or negative selection from one step is subjected to another separation step, such as subsequent positive or negative selection. In some examples, a single isolation step simultaneously depletes cells expressing multiple markers, such as by incubating the cells with multiple antibodies or binding partners that are each specific for the marker targeted by negative selection. Likewise, multiple cell types can be positively selected simultaneously by incubating the cells with multiple antibodies or binding partners expressed on various cell types.

For example, in some aspects, a specific subpopulation of T cells, such as one or more surface marker positive cells or cells expressing high levels of one or more surface markers, for example, CD3+, CD28+, CD62L+, CCR7+, CD27+, CD127+, CD4+, CD8+, CD45RA+ and/or CD45RO+ T cells, are separated by positive or negative selection techniques.

For example, CD3+ and CD28+ T cells can be positively selected using CD3/CD28-linked magnetic beads (for example, DYNA bead M-450 CD3/CD28 T cell expander).

In some embodiments, separation is performed by enriching specific cell populations by positive selection or depleting specific cell populations by negative selection. In some embodiments, positive or negative selection is accomplished by incubating cells with one or more antibodies or other binding reagents that specifically bind to one or more surface markers. The one or more surface markers are expressed on positively or negatively selected cells (marker+) or at a relatively high level (marker high).

In some embodiments, T cells are isolated from a PBMC sample by negative selection of markers (such as CD14) expressed on non-T cells (such as B cells, monocytes, or other blood leukocytes). In some aspects, a CD4+ or CD8+ selection step is used to isolate helper CD4+ and CD8+ cytotoxic T cells. By positively or negatively selecting markers expressed on one or more primary, memory and/or effector T cell subpopulations or at a relatively high degree, such CD4+ and CD8+ populations can be further sorted into subpopulations.

In some embodiments, CD8+ cells are further enriched or depleted for primary, central memory, effector memory, and/or central memory stem cells, for example, by positive or negative selection based on surface antigens associated with the corresponding subpopulation. In some embodiments, enrichment for central memory T (TCM) cells is performed to increase efficacy, for example, to improve long-term survival, proliferation, and/or engraftment after administration. In some aspects, it is particularly robust in this subgroup. See Terakura et al. (2012) Blood. 1:72-82; Wang et al. (2012) J Immunother. 35(9):689-701. In some embodiments, TCM-enriched CD8+ T cells are combined with CD4+ T cells to further enhance efficacy.

In an embodiment, memory T cells are present in the CD62L+ and CD62L- subgroups of CD8+ peripheral blood lymphocytes. PBMCs can be enriched or depleted for the CD62L-CD8+ and/or CD62L+CD8+ part, for example, using anti-CD8 and anti-CD62L antibodies.

In some embodiments, the enrichment for central memory T (TCM) cells is based on CD45RO, CD62L, CCR7, CD28, CD3, and/or CD127 positive or high surface expression; in some aspects, it performs negative selection based on cells expressing or highly expressing CD45RA and/or granule enzyme B. In some aspects, the isolation of the CD8+ population enriched for TCM cells is performed by depleting cells expressing CD4, CD14, CD45RA, and positive selection or enrichment for cells expressing CD62L. On the one hand, the enrichment for central memory T (TCM) cells is performed as follows: starting from the negative part of cells selected based on CD4 expression, which is negatively selected based on the expression of CD14 and CD45RA, and positively selected based on CD62L. In some aspects, such selections are performed simultaneously, and in other aspects, such selections are performed sequentially in a certain order. In some aspects, the same CD4 expression-based selection step is used to prepare CD8+ cell populations or subpopulations, and also to generate CD4+ cell populations or subpopulations, thereby retaining the positive and negative portions from the CD4-based separation, and optionally after one or more further positive or negative selection steps, it is used in the subsequent steps of the method.

In a specific example, CD4+ cell selection is performed on PBMC samples or other blood leukocyte samples, wherein negative and positive parts are retained. Then, negative selection is performed on the negative part based on the expression of CD14 and CD45RA or CD19, and positive selection is performed based on central memory T cell characteristic markers, such as CD62L or CCR7, wherein the positive and negative selections are performed in a certain order.

By identifying cell populations with cell surface antigens, helper CD4+ T cells are sorted into primary, central memory and effector cells. CD4+ lymphocytes can be obtained by standard methods. In some embodiments, the primary CD4+ T lymphocytes are CD45RO-, CD45RA+, CD62L+, CD4+ T cells. In some embodiments, the central memory CD4+ cells are CD62L+ and CD45RO+. In some embodiments, the effector CD4+ cells are CD62L- and CD45RO-.

In one example, in order to enrich CD4+ cells by negative selection, the monoclonal antibody mixture usually includes antibodies against CD14, CD20, CD11b, CD16, HLA-DR, and CD8. In some embodiments, the antibody or binding partner is bound to a solid support or matrix, such as magnetic beads or paramagnetic beads, to allow separation of cells for positive and/or negative selection.

In some embodiments, the preparation method includes a freezing step, for example, freezing the cells before or after isolation, incubation, and/or engineering. In some embodiments, the freezing and subsequent thawing steps remove granulocytes and, to some extent, remove monocytes in the cell population. In some embodiments, for example, after a washing step to remove plasma and platelets, the cells are suspended in a freezing medium. In some aspects, any of a variety of known freezing mediums and parameters can be used. One example involves the use of PBS or other suitable cell freezing medium containing 20% DMSO and 8% human serum albumin (HAS). Then, it was diluted 1:1 with medium, so that the final concentrations of DMSO and HSA were 10% and 4%, respectively. Then, the cells are generally frozen to -80°C or -90°C according to a predetermined procedure or principle, such as a rate of l°/min, with a programmable cooling device, and stored in the vapor phase of a liquid nitrogen storage tank.

In some embodiments, the provided methods include cultivation, incubation, culturing, and/or genetic engineering steps. For example, in some embodiments, methods are provided for incubating and/or engineering the depleted cell population and the culture starting composition.

In some embodiments, the cell population is incubated in the culture starting composition. Incubation and/or engineering can be carried out in culture vessels, such as units, chambers, wells, columns, tubes, tube sets, valves, vials, petri dishes, bags, or other containers used to culture or grow cells.

In some embodiments, cells are incubated and/or cultured before or with genetic engineering. The incubation step may include culture, incubation, stimulation, activation, and/or proliferation. In some embodiments, the cells or compositions are incubated in the presence of stimulating conditions or stimulating agents. Such conditions include those designed to induce cell proliferation, reproduction, activation, and/or survival in a population to mimic antigen contact, and/or trigger cells for genetic engineering, such as those used to introduce recombinant antigen receptors.

The conditions may include one or more of the following: specific medium, temperature, oxygen content, carbon dioxide content, time, reagents, for example, nutrients, amino acids, antibiotics, ions, and/or stimulating factors, such as cytokines, chemokines, antigens, binding partners, fusion proteins, recombinant soluble receptors, and any other substances designed to maintain an activated cell state.

In some embodiments, the stimulating condition or agent includes one or more substances, for example, a ligand, which can activate the intracellular signal transduction domain of the TCR complex. In some aspects, the substance turns on or initiates the TCR/CD3 intracellular signal transduction cascade in T cells. Such substances may include antibodies, such as those specific for TCR components and/or costimulatory receptors, such as anti-CD3, anti-CD28, for example, which are bound to a solid support, such as beads, and/or one or more cytokines. Optionally, the amplification method may further include the step of adding anti-CD3 and/or anti-CD28 antibodies to the culture medium (for example, at a concentration of at least about 0.5 ng/ml). In some embodiments, the stimulant includes 1L-2 and/or IL-15 and/or IL-7 and/or IL-21, for example, IL-2 at a concentration of at least about 10 units/mL.

In some aspects, the incubation is performed in accordance with some techniques, such as U.S. Patent No. 6,040,177 granted to Riddell et al., Klebanoff et al., (2012) J Immunother. 35(9):651-660, Terakura et al., (2012) Blood. 1:72- 82 and/or Wang et al. (2012) J Immunother. 35(9): 689-701.

In some embodiments, the T cell population is expanded as follows: adding feeder cells to culture starting composition, such as non-dividing peripheral blood mononuclear cells (PBMCs), (for example, therefore, for each T lymphocyte in the initial population to be expanded, the resulting cell population contains at least about 5, 10, 20, or 40 or more PBMC feeder cells); and incubating the culture (e.g., for a time sufficient to expand the number of T cells). In some aspects, the non-dividing feeder cells may include gamma ray-irradiated PBMC feeder cells. In some embodiments, the PBMCs is irradiated with gamma rays in the range of about 3000-3600 rads to prevent cell division. In some aspects, the feeder cells are added to the culture medium before adding the population of T cells.

In some embodiments, the stimulation conditions include a temperature suitable for the growth of human T lymphocytes, for example, at least about 25 degrees Celsius, generally at least about 30 degrees Celsius, and generally at or about 37 degrees Celsius. Optionally, the incubation may also include adding non-dividing EBV-transformed lymphoblastoid cells (LCL) as feeder cells. LCL can be irradiated with gamma rays in the range of about 6000-10000 rads. In some aspects, the LCL feeder cells are provided in any suitable amount, for example, the ratio of LCL feeder cells to primary T lymphocytes is at least about 10:1.

In a certain embodiment, antigen-specific T cells, such as antigen-specific CD4+ and/or CD8+ T cells, are obtained by stimulating natural or antigen-specific T lymphocytes with antigen. For example, antigen-specific T cell lines or clones can be generated against cytomegalovirus antigens by isolating T cells from an infected subject and stimulating the cells *in vitro* with the same antigen.

### Composition and formulation

The present invention also provides compositions comprising cells for administration, including pharmaceutical compositions and preparations, such as a composition from a unit dosage form containing the number of cells for administration in a given dose or part thereof. The pharmaceutical compositions and formulations generally include one or more optional pharmaceutically acceptable carriers or excipients. In some embodiments, the composition includes at least one of the other therapeutic agents.

The term "pharmaceutical preparation" refers to a form of preparation that allows the biological activity of the active ingredient contained therein to be effective, and does not contain additional ingredients with unacceptable toxicity to the subject to whom the preparation is to be administered.

"Pharmaceutically acceptable carrier" refers to an ingredient in a pharmaceutical preparation that is not an active ingredient and is not toxic to a subject. Pharmaceutically acceptable carriers include, but are not limited to, buffers, excipients, stabilizers or preservatives.

In some aspects, the choice of carrier is determined in part by the particular cell and/or method of administration. Therefore, there are many suitable formulations. For example, the pharmaceutical composition may include a preservative. Suitable preservatives may include, for example, methyl paraben, propyl paraben, sodium benzoate, and benzalkonium chloride. In some aspects, a mixture of two or more preservatives are used. The preservative or mixture thereof is usually present in an amount of from about 0.0001% to about 2% (based on the total weight of the composition). The carrier is described in, for example, "Remington's Pharmaceutical Sciences", 16th edition, Osol, A. Ed. (1980). At the dosage and concentration used, pharmaceutically acceptable carriers are usually non-toxic to the recipient, including but not limited to: buffers such as phosphate, citrate and other organic acid buffers; antioxidants, including ascorbic acid and methionine; preservatives (such as octadecyl dimethyl benzyl ammonium chloride; hexaalkyl quaternary ammonium chloride; benzalkonium chloride, benzethonium chloride; phenol, butyl or benzyl alcohol; p-hydroxy alkyl benzoate, such as methyl or propyl p-hydroxybenzoate; catechol; resorcinol; cyclohexanol; 3-pentanol; and m-cresol); low molecular weight (less than about 10 residues)) polypeptide; protein, such as serum albumin, gelatin or immunoglobulin; hydrophilic polymer, such as polyvinylpyrrolidone; amino acid, such as glycine, glutamine, asparagine, histidine, arginine or lysine; monosaccharides, disaccharides, and other sugars, including glucose, mannose, or dextrin; chelating agents, such as EDTA; sugars, such as sucrose, mannitol, trehalose, or sorbitol; salt-forming counter ions, such as sodium; metal complexes (such as Zn-protein complexes); and/or non-ionic surfactants, such as polyethylene glycol (PEG).

In some aspects, the composition includes a buffer. Suitable buffering agents include, for example, citric acid, sodium citrate, phosphoric acid, potassium phosphate, and various other acids and salts. In some aspects, a mixture of two or more buffers is used. The buffering agent or mixture thereof is usually present in an amount of about 0.001 % to about 4% (based on the total weight of the composition). Methods for preparing administrable pharmaceutical compositions are known. Exemplary methods are specifically described in, for example, "Remington: The Science and Practice of Pharmacy" (Lippincott Williams & Wilkins); 21st edition (May 1, 2005).

The formulation may comprise an aqueous solution. The preparation or composition may also contain more than one active ingredient, which can be used for the specific indication, disease or condition to be treated with the cell, preferably those with complementary activity to the cell, wherein the corresponding activity agents do not negatively affect each other. Such active ingredients are suitable to be present in combination in an amount effective for the desired purpose. Therefore, in some embodiments, the pharmaceutical composition further comprises other pharmaceutically active substances or drugs, such as chemotherapeutics, for example, asparaginase, busulfan, carboplatin, cisplatin, daunorubicin, doxorubicin, fluorouracil, gemcitabine, hydroxyurea, methotrexate, paclitaxel, rituximab, vinblastine, and/or vincristine.

In some embodiments, the pharmaceutical composition includes an amount effective to treat or prevent a disease or condition, such as a therapeutically effective or preventive effective quantity of cells. In some embodiments, the efficacy of treatment or prophylaxis is monitored by regularly evaluating the treated subjects. The required dose can be delivered by administering the cells by a single pill, by administering the cells by multiple pills, or by administering the cells by continuous infusion.

In some embodiments, the composition comprises cells in a quantity effective to reduce the burden of a disease or condition, and/or a quantity that does not cause CRS or severe CRS in the subject and/or a quantity that achieves any other results of the methods described herein.

The cells and compositions can be administered using standard administration techniques, formulations and/or devices. The administration of the cells can be autologous or heterologous. For example, immunosuppressive cells or progenitor cells can be obtained from one subject and administered to the same subject or different compatible subjects. The immunosuppressive cells derived from peripheral blood or their progeny (e.g., derived in vivo, ex vivo, or in vitro) can be administered by local injection, including catheter administration, systemic injection, local injection, intravenous injection, or parenteral administration. When a therapeutic composition (for example, a pharmaceutical composition containing genetically modified immunosuppressive cells) is administered, it is usually formulated in a unit-dose injectable form (solution, suspension, emulsion).

Formulations include those for oral, intravenous, intraperitoneal, subcutaneous, pulmonary, transdermal, intramuscular, intranasal, mucosal, sublingual or suppository administration. In some embodiments, the cell population is administered parenterally. The term "parenteral" as used herein includes intravenous, intramuscular, subcutaneous, rectal, vaginal and intraperitoneal administration. In some embodiments, the cells are administered to the subject by intravenous, intraperitoneal or subcutaneous injection using peripheral systemic delivery.

In some embodiments, the composition is provided in the form of a sterile liquid formulation, for example, an isotonic aqueous solution, suspension, emulsion, dispersion, or viscous composition, which in some aspects can be buffered to a selected pH. Liquid formulations are generally easier to prepare than gels, other viscous compositions, and solid compositions. In addition, liquid compositions are somewhat easier to administer, especially by injection. In another aspect, the viscous composition can be formulated in a suitable viscosity range to provide a longer contact time with a specific tissue. The liquid or viscous composition may include a carrier, which may be a solvent or dispersion medium, which contains, for example, water, saline, phosphate buffered saline, polyhydroxy compounds (for example, glycerol, propylene glycol, liquid polyethylene glycol), and suitable mixture thereof. Sterile injectable solutions can be prepared by incorporating the cells in a solvent, such as with a suitable carrier, diluent, or excipient such as sterile water, physiological saline, glucose, dextrose, and the like. The composition may contain auxiliary substances such as wetting, dispersing, or emulsifying agents (for example, methyl cellulose), pH buffering agents, gelling or viscosity enhancing additives, preservatives, flavoring agents, and/or pigments, depending on the desired route of administration and preparation. In some aspects, standard textbooks can be consulted to prepare suitable preparations.

### Articles of manufacture

The present invention also provides an article of manufacture, such as kits and devices, for administering cells to subjects according to the provided methods for adoptive cell therapy or immune effector cell therapy, and for storing and administering the cells and compositions.

The article of manufacture includes one or more containers, generally multiple containers, packaging materials, and a label or package insert combined with or on one or more containers and/or packaging, and generally includes instructions for administering cells to a subject.

The container generally contains the cells to be administered, for example, one or more unit doses thereof. Articles of manufacture generally include multiple containers, each containing a single unit dose of cells. The unit dose may be the quantity or number of cells to be administered to the subject of the immune effector cells of the previous course of treatment or twice (or more) the number of cells to be administered to the immune effector cells of the first or subsequent course of treatment. It may be the lowest dose or lowest possible dose of cells given to the subject in relation to the method of administration. In some embodiments, the unit dose is the number of cells or the minimum number of cells to be administered to any subject or any subject with a specific disease or disorder in a unit dose according to the method of the present invention. For example, in some aspects, the unit dose may include the minimum quantity of cells that will be administered to a subject with a lower body weight and/or a lower disease burden, such as administering one or in some cases more than one unit doses to a given subject with the immune effector cells of the previous course of treatment and administering one or more than one unit dose of immune effector cells to the given subject in one or more subsequent courses of treatment, for example, in accordance with the methods provided. In some embodiments, the number of cells in a unit dose is the number of immune effector cells that need to be administered to a specific subject in a previous course of treatment, such as the number of chimeric antigen receptor-expression or CAR-expression or number of cells in a cell-derived subject. In some embodiments, the cells are derived from the subject to be treated by the methods provided herein.

In some embodiments, each container individually contains a unit dose of cells, for example, including the same or substantially the same number of cells. Therefore, in some embodiments, each container contains the same or approximately or substantially the same number of cells or chimeric antigen receptor-expressing cells. In some embodiments, the unit dose includes less than about 1x 10¹⁰, less than about 1x 10⁹, less than about 1x 10⁸, or less than about 1x 10⁷ engineered cells, total cells, T cells or PBMCs per kilogram of the subject to be treated and/or cell-derived .

Suitable containers include, for example, bottles, vials, syringes, and flexible bags such as freezer bags. In specific embodiments, the container is a bag, for example, a flexible bag, such as those suitable for infusing cells into a subject, for example, a flexible plastic or PVC bag or EVA or ULPDE, and/or an IV solution bag. In some embodiments, the bag is sealable and/or sterilizable to provide sterile solutions and delivery of cells and compositions. In some embodiments, for the container, for example, the volume of the bag is equal to or about or at least or about 10, 20, 30, 40, 50, 60, 70, 80, 90, 100, 200, 300, 400, 500, or 1000 ml volume, such as equal to or about 10 to equal to or about 100 mL or equal to or about 10 to equal to or about 500 mL volume. In some embodiments, the container, for example, the bag is and/or made of a material that is stable at one or more different temperatures and/or provides stable storage and/or maintenance of cells, such as at low temperatures, for example, lower than at or about or equal to or about -20°C, -80°C, -120°C, 135°C, -196°C and/or a temperature suitable for cryopreservation, and/or other temperatures, such as a temperature suitable for freezing and thawing cells and body temperature, such as equal to or about 37°C or -38°C, or -39°C, or -40°C, to allow freezing and thawing prior to treatment, for example, at the subject's site or treatment site.

The container can be made of various materials such as glass or plastic. In some embodiments, the container has one or more ports, for example, a sterile immersion port, for example, for connecting to one or more tubes by a tube or catheter, for example, for intravenous or other infusion and/or connect for the purpose of transferring from and to other containers, such as cell culture and/or storage bags or other containers. Exemplary containers include freezer bags, intravenous solution bags, vials, including those with stoppers that can be pierced by an injection needle.

The article of manufactor may also include a package insert or label, one or more pieces of which display usage information and/or instructions. In some embodiments, the information or instructions show the content that can or should be used to treat a particular disease or condition, and/or provide instructions for it. The label or package insert may show the contents of the article to be used to treat the disease or condition. In some embodiments, the label or package insert provides instructions for treating a subject, e.g., a subject whose cells have been derived, by including cells that administer the first and one or more immune effector cells for subsequent courses of treatment, e.g., the method according to any one of the provided method embodiments. In some embodiments, the instructions specify that in the previous course of treatment, a unit dose of the immune effector cells, for example, the contents of a single container of the article of manufactor, is administered, and then at a specified time point or within a specified time window and/or after the presence or absence or amount or extent of one or more factors or results is detected in the subject, immune effector cells of one or more subsequent courses of treatment are then administered.

In some embodiments, the instructions specify that multiple unit doses are administered to the subject by performing the first administration and continuous administration. In some embodiments, the first administration includes delivering one of the unit doses to the subject and subsequent administration includes administering one or more of the unit doses to the subject.

In some embodiments, the label or package insert or package contains an identifier to indicate the identity of the subject from which the cells are derived and/or the subject to be administered. In the case of autologous transplantation, the cells are derived from the subject to whom the cells are to be administered. Therefore, the identification information may specify that the cells are to be given to a specific patient. Such information may be present in the packaging material and/or label in the form of a barcode or other coded identifier, or may indicate the subject's name and/or other identifying characteristics.

In some embodiments, the article of manufacture includes one or more, usually multiple containers containing a composition containing cells, such as a separate unit dosage form thereof, and also includes one or more other containers containing the composition therein. The composition includes other agents, such as cytotoxic agent or other therapeutic agents, which, for example, will be combined with the cells, for example, being administered simultaneously or in any order at one time. Alternatively or in addition, the article of manufacture may also include another or the same container containing a pharmaceutically acceptable buffer. It may also include other materials, such as other buffers, diluents, filters, tubes, needles, and/or syringes.

The term "package insert" refers to the instructions often included in the commercial packaging of therapeutic products, the instructions containing information about the description, usage, dosage, administration, combination therapy, contraindications, and/or warnings of such therapeutic products.

The method of the present invention can be summarized as:
Peripheral blood mononuclear cells (PBMC) or T cells from human subjects with cancer are isolated based on "monocyte-removal separation", and viral vectors encoding chimeric antigen receptors (CARs) are used to culture and transduce cells. The chimeric antigen receptor (CAR) specifically binds to an antigen expressed by cancer in a subject, which is a tumor-related or tumor-specific antigen. The cells are cryopreserved in an infusion medium in separate flexible freezing bags, each containing a single unit dose of cells, which is about 1×10⁵ cells to 1×10⁹ cells. Prior to infusion, the cells are maintained at a temperature of about below -130°C or about below -175°C.

Before starting cell therapy, blood is obtained from the subject, and the levels of one or more serum factors indicative of cytokine release syndrome (CRS) in serum are optionally evaluated by ELISA and/or MSD and/or CBA, such as tumor necrosis factor alpha (TNFα) interferon gamma (IFNγ), IL-10 and IL-6. Before treatment starts, the tumor burden can optionally be assessed by measuring the size or quality of the solid tumor, for example by PET or CT scan.

The resuscitation was performed by raising the temperature to about 38°C, and the subject was administered with the cells of the immune effector cells of the previous course of treatment by multiple infusions. The infusion is continuous intravenous (IV) administration within about 1-20ml/min.

After administering the immune effector cells of the previous course of treatment, the subject undergoes a physical examination and os monitored for any symptoms of toxicity or toxicity result, such as fever, hypotension, hypoxia, neurological disorders, or inflammatory cytokines or increased serum levels of C-reactive protein (CRP). Optionally, after administering the immune effector cells of the previous course of treatment, in one or more cases, blood is obtained from the patient, and the levels of serum factors indicative of CRS are evaluated by ELISA and/or MSD and/or CBA methods . The serum factor level is compared with the serum factor level obtained just before the immune effector cells in the previous course of treatment are administered. If necessary, anti-IL6 or other CRS treatments is administered to reduce the symptoms of CRS.

After administering the immune effector cells of the previous course of treatment, for example 1, 2, 3 and/or 4 weeks after the start of administration, optionally detecting the presence or absence of an anti-CAR immune response in the subject, for example, by qPCR, ELISA, ELISPOT, cell-based antibody assay and/or mixed lymphocyte reaction.

The percent reduction in tumor burden achieved by the immune effector cells of the previous course of treatment can optionally be measured one or more times after the immune effector cells in the previous course of treatment are administered in solid tumor patients by scanning (e.g., PET and CT scans), and / or by quantifying disease-positive cells in the blood or tumor site.

From the first course of treatment with immune effector cells and lasting up for several years, the subjects are monitored regularly. During the follow-up period, the tumor burden is measured, and/or CAR-expressing cells are detected by flow cytometry and quantitative polymerase chain reaction (qPCR), in order to measure the in vivo proliferation and persistence of the administered cells, and/or evaluate the development of the anti-CAR immune response.

The present invention will be further explained below in conjunction with specific embodiments. It should be understood that these embodiments are only used to illustrate the present invention and not to limit the scope of the present invention. The experimental methods without specific conditions in the following examples usually follow the conventional conditions as described in J. Sambrook et al., Molecular Cloning Experiment Guide, Third Edition, Science Press, 2002, or follow the suggested conditions described in the manufacturer.

### Materials and Methods:

Various materials used in the present invention, including reagents, can be purchased from commercial channels.

For the preparation method of immune effector cells expressing chimeric antigen receptors, refer to, the full content disclosed for example, Chinese Patent Application Publication Nos. CN107058354A, CN107460201A, CN105194661A, CN105315375A, CN105713881A, CN106146666A, CN106519037A, CN106554414A, CN105331585A, CN106397593A, CN106467573A, International Patent Application Publication Nos. WO2018006882A1, WO2015172339A8.

As an example, in the following embodiments of the present invention, the amino acid sequence of the scFv portion of the chimeric antigen receptor is shown in SEQ ID NO: 27, the nucleotide sequence is shown in SEQ ID NO: 26, and the scFv comprisees the heavy chain variable region shown in SEQ ID NO: 21 and the light chain variable region shown in SEQ ID NO: 20, the chimeric antigen receptor comprises the amino acid sequence shown in SEQ ID NO: 36. It should be understood that the CAR containing the aforementioned scFv may also have other intracellular domains. Therefore, the sequence of the CAR may also be the sequence shown in SEQ ID NO: 37 or 38.

The scFv shown in SEQ ID NO:27 comprises the HCDR1 shown in SEQ ID NO:11, the HCDR2 shown in SEQ ID NO:12, the HCDR3 shown in SEQ ID NO:5, and the LCDR1 shown in SEQ ID NO:6, the LCDR2 shown in SEQ ID NO:7, the LCDR3 shown in SEQ ID NO:10.

### EXAMPLE

### Example 1. Treatment of patients with multiple myeloma

Patients with BCMA-positive multiple myeloma were administered autologous T cells expressing anti-BCMA chimeric antigen receptor (CAR). Before administering the cells, the patient receives the apheresis technique of "monocyte-removal separation" and was treated with pretreatment. In order to obtain autologous CAR-T cells, T cells were obtained by isolating PBMCs from the subject's peripheral blood, transduced with viral vectors encoding CARs targeting BCMA, and extensively amplified. Then CAR-T cells targeting BCMA were obtained by preparation with frozen medium agents then subpackaged into freezer bags and stored in liquid nitrogen below -175°C. They were thawed and resuscitated before infusion, and then autologously reinfused into the subject..

Before starting cell therapy, obtain blood from the subject, and optionally evaluate the level of one or more factors indicative of cytokine release syndrome (CRS) in the serum by ELISA and/or MSD and/or CBA methods, for example tumor necrosis factor alpha (TNFα), interferon gamma (IFNγ), IL-10, IL-2 and IL-6. Before treatment starts, the tumor burden can be assessed by evaluating the number of cells associated with cancer in the bone marrow or peripheral blood of the patient. By assessing the bone marrow, the tumor burden before treatment was evaluated and the percentage of bone marrow blasts was determined. A subject with at least 5% of blast cells in the bone marrow was considered to have a morphological disease (MD).

The cryopreserved anti-BCMA CAR-T cells were resuscitated by warming to about 38°C, and the patient was given by a single infusion, a continuous intravenous (IV) infusion within about 2-30 minutes, the median infusion duration of which was 5 minutes .

After administering anti-BCMA CAR-T cells, the subject undergoes a physical examination and was monitored for any signs of toxicity or toxicity results, such as fever, hypotension, hypoxia, neurological disorders, or inflammatory cytokines or increased serum levels C-reactive protein (CRP). Optionally, after administration of anti-BCMA CAR-T cells for one or more times, blood was obtained from the patient, and the levels of serum factors indicative of CRS were evaluated by ELISA and/or MSD and/or CBA methods. The serum factor level was compared with the serum factor level obtained just before the first dose. If necessary, give anti-IL6 or other CRS treatments to reduce the symptoms of CRS.

The subjects were monitored regularly from after the administration of anti-BCMA CAR-T cells until several years later. During follow-up, tumor burden was measured, and/or anti-BCMA CAR-expressing cells were detect by flow cytometry and quantitative polymerase chain reaction (qPCR) to measure the in vivo proliferation and persistence of the administered cells, and/or evaluate the development of anti-CAR immune response.

The response of anti-BCMA CAR-T cells to relapsed/refractory myeloma (refer to IMWG2016 version, treatment evaluation was CR, sCR, ICR, MCR or VGPR):
a. Refer to the "Diagnostic Criteria for Multiple Myeloma" (IMWG2016 Edition) response criteria for efficacy evaluation;
b. Statistical indicators for efficacy evaluation:
   Progression-free survival (PFS), disease control rate (DCR) and objective response rate (ORR), overall survival (OS).

In the present invention, subjects are multiple myeloma patients with positive BCMA expression, relapsed or refractory multiple myeloma, and are divided into 4 groups to participate. All subjects expressed high levels of BCMA (the positive rate of BCMA expression was 53%-100%), and the median duration of multiple myeloma was between 0.3 and 10.7 years, mostly about 5.2 years (range: 0.4 to 10.7 years). The median number of previous chemotherapy was 6 (3-20), and some subjects had also received stem cell transplantation before. The average proportion of plasma cells in the bone marrow of the subjects was about 25% (range: <5% ∼ 85%). The subject's disease types include IgG κ type, IgA λ type, IgG λ type, λ light chain type, and IgA κ type. So far, the median follow-up time for subjects is about 135 days (range: 11 to 260 days).

The first group was the low-dose cyclophosphamide group, the pretreatment dose of cyclophosphamide was not higher than 400mg/m²/d (about 190-310mg/m2/d), and about 20mg/m²/d fludarabin. After the pretreatment agent was administered, approximately 2×10⁶ to 2.7×10⁶ cells/kg of BCMA CAR-T cells were administered to each subject.

The second group was the cyclophosphamide high-dose group. The pretreatment dose of cyclophosphamide was higher than 400mg/m²/d (about 450-560mg/m²/d), and about 20-30mg/m²/d fludarabine. After administration of the pretreatment agent, each subject was adminisered an infusion of BCMA CAR-T cells of approximately 2×10⁶ to 2.5×10⁶ cells/kg.

The third group was the CAR-T low-dose group. The anti-BCMA CAR-T cells infused to subjects of this group was less than 1.5×10⁶ cells/kg, and the anti-BCMA CAR-T cells infused to subjects of this group was about 0.9×10⁶ cells/kg by infusion.

The fourth group was the CAR-T high-dose group, which includes 3 patients. About 2.7×10⁶ to 3.3×10⁶ cells/kg anti-BCMA CAR-T cells were infused to subjects of this group.

The day when the CAR T cell therapy is administered to the subject is designated as day 0. Fludarabine and cyclophosphamide can be administered on the same day or on different days. In group 1, fludarabine was administered to subjects on days -6, -5, and -4, and cyclophosphamide was administered on days -6, -5, -4, -3, and -2; or, fludarabine and cyclophosphamide were administered to subjects on days -4, -3, -2; or fludarabine and cyclophosphamide were administered to the subject on days -3, -2, and -1; or fludarabine and cyclophosphamide were administered to the subject on days -3, and -2. In the second group, fludarabine was administered to subjects on days -5, -4, -3, and -2, and cyclophosphamide was administered on days -5 and -4; or fludarabine was administered to subjects on days -5, -4 and -3, and cyclophosphamide was administered on days -5 and -4; or fludarabine was administered to subjects on days -5, -4, and -3, and cyclophosphamide was administered on day -5; or fludarabine was administered to the subject on day -4, -3, and -2, and cyclophosphamide was administered on day -4 and -3; or fludarabine and cyclophosphamide were administered to the subject on days -3 and -2. In Group 3, fludarabine was administered to the subject on days -5, -4, and -3, and cyclophosphamide was was administered on days -5 and -4. In group 4, fludarabine was administered to subjects on days -6, -5, -4, and -3, and cyclophosphamide was administered on days -6 and -5; or fludarabine and cyclophosphamide were administered to the subject on days -3 and -2; or fludarabine and cyclophosphamide were administered to the subject on the days -3, -2 and -1.

The dosage of each group is shown in Table 1. The dosage of fludarabine, cyclophosphamide, and CAR-T cells indicated in the table is the total dosage.

**Table 1.**

| **Grouping situation** | **Patient number** | **Drug** | **Dose** | **Number of previous chemotherapies** | **Whether stem cell transplantation has been received** |
|---|---|---|---|---|---|
| Group 1 | **Patient 1** | Fludarabine | 21mg/m²/d×3 | 6 | yes |
| | | Cyclophosphamide | 210mg/m²/d×5 | | |
| | | CAR-Tcell | 1.5^{∗}10⁸cell | | |
| | **Patient 2** | Fludarabine | 19mg/m²/d×2 | 3 | no |
| | | Cyclophosphamide | 192mg/m²/d×2 | | |
| | | CAR-Tcell | 1.5^{∗}10⁸cell | | |
| | **Patient 3** | Fludarabine | 21mg/m²/d×3 | 8 | no |
| | | Cyclophosphamide | 306mg/m²/d×3 | | |
| | | CAR-Tcell | 1.5^{∗}10⁸cell | | |
| | **Patient 4** | Fludarabine | 20mg/m²/d×3 | 9 | no |
| | | Cyclophosphamide | 304mg/m2/dx3 | | |
| | | CAR-Tcell | 1.5 ^{∗}10⁸cell | | |
| Group 2 | **Patient 5** | Fludarabine | 24mg/m²/d×3 | 8 | yes |
| | | Cyclophosphamide | 482mg/m²/d×2 | | |
| | | CAR-Tcell | 1.8^{∗}10⁸cell | | |
| | **Patient6** | Fludarabine | 25mg/m²/d×3 | 4 | yes |
| | | Cyclophosphamide | 524mg/m²/d×2 | | |
| | | CAR-Tcell | 1.5^{∗}10⁸cell | | |
| | **Patient 7** | Fludarabine | 20 mg/m2/dx4 | 5 | yes |
| | | Cyclophosphamide | 508 mg/m²/d×2 | | |
| | | CAR-Tcell | 1.5^{∗}10⁸cell | | |
| | **Patient 8** | Fludarabine | 19 mg/m2/d×3 | 6 | yes |
| | | Cyclophosphamide | 457 mg/m²/d×1 | | |
| | | CAR-Tcell | 1.5^{∗}10⁸cell | | |
| | **Patient 9** | Fludarabine | 25 mg/m²/d×3 | 9 | yes |
| | | Cyclophosphamide | 543 mg/m²/d×2 | | |
| | | CAR-Tcell | 1.5^{∗}10⁸cell | | |
| | **Patient 10** | Fludarabine | 26 mg/m²/d×2 | 6 | no |
| | | Cyclophosphamide | 517 mg/m²/d×2 | | |
| | | CAR-Tcell | 1.5 ^{∗}10⁸cell | | |
| | **Patient 11** | Fludarabine | 25 mg/m²/d×3 | 12 | no |
| | | Cyclophosphamide | 519 mg/m²/d×2 | | |
| | | CAR-Tcell | 1.5 ^{∗}10⁸cell | | |
| | **Patient 12** | Fludarabine | 26 mg/m²/d×3 | 7 | yes |
| | | Cyclophosphamide | 504mg/m2/d×2 | | |
| | | CAR-Tcell | 1.5^{∗}10⁸cell | | |
| Group 3 | **Patient 13** | Fludarabine | 21mg/m²/d×3 | 6 | no |
| | | Cyclophosphamide | 519mg/m²/d×2 | | |
| | | CAR-Tcell | 0.5^{∗}10⁸cell | | |
| Group 4 | **Patient 14** | Fludarabine | 21mg/m2/d×4 | 2 | no |
| | | Cyclophosphamide | 525mg/m²/d×2 | | |
| | | CAR-Tcell | 1.5^{∗}10⁸cell | | |
| | **Patient 15** | Fludarabine | 21mg/m²/d×2 | 7 | no |
| | | Cyclophosphamide | 312mg/m²/d×2 | | |
| | | CAR-Tcell | 1.5^{∗}10⁸cell | | |
| | **Patient 16** | Fludarabine | 21mg/m²/d×3 | 2 | no |
| | | Cyclophosphamide | 311mg/m²/d×3 | | |
| | | CAR-Tcell | 1.5^{∗}10⁸cell | | |

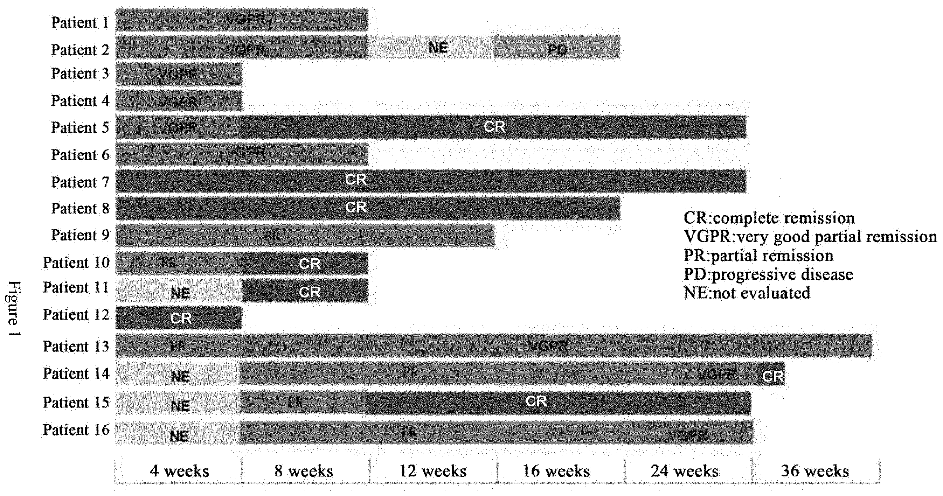

The therapeutic effects of each group of patients are shown in Figure 1, ORR was 100%; CR was (8 cases) about 50%. Among them, the CR rate of the second group was 75%. In order to further evaluate the efficacy of administering anti-BCMA CAR-T cells, the continuous survival period of anti-BCMA CAR-T cells in the body was detected, that is, the continuous survival period of CAR-T cells "engrafted" in the body. From the end of the first infusion (day 0), Q-PCR was used at each visit point, the probe used was Probe783-P1 (see SEQ ID NO: 42 for nucleotide sequence); the upstream primer sequence was: Primer783P1-F1 (see SEQ ID NO: 43 for nucleotide sequence); the downstream primer sequence was: Primer783P1-R3 (see SEQ ID NO: 44 for nucleotide sequence), and the copy number of anti-BCMA CAR DNA in peripheral blood was measured until any 2 consecutive tests are negative and recorded as the continuous survival period of anti-BCMA CAR-T cells.

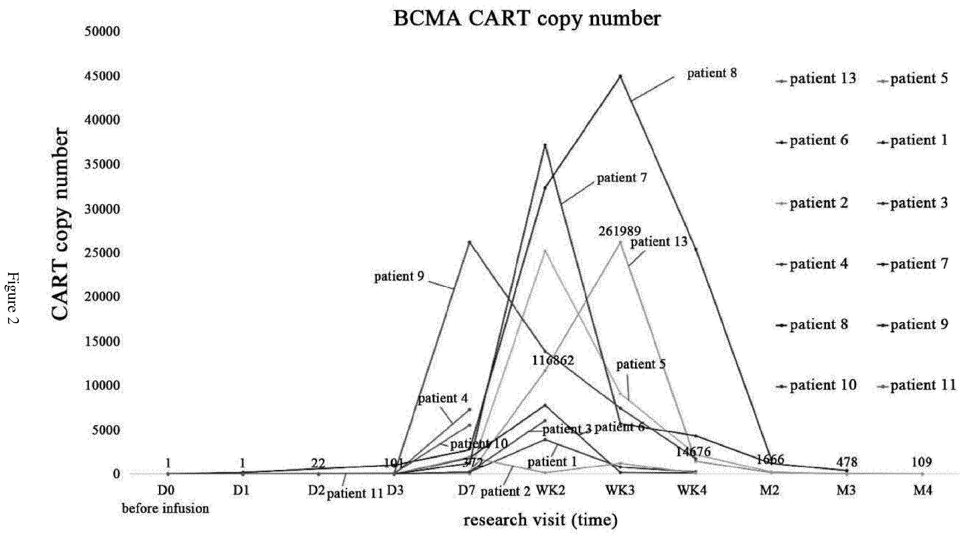

Patients No. 1-13 were tested, the results are shown in Table 2 and Figure 2. BCMA CAR-T cells proliferated in all subjects. The number of cell copies was detected on the third day, and the peak time was about the first 7∼21 days, maintained until the second to third months.

The number of copies of anti-BCMA CAR DNA contained in the peripheral blood of patients No. 14, 15, and 16 were tested. The results showed that the number of CAR-T cells in patient No. 14 reached a peak on the 14th day after CAR-T administration, and the number of CAR-T cells was undetectable on day 56; the number of CAR-T cells in patient No. 15 reached a peak on day 14 after CAR-T administration, and was undetectable on day 56; the number of CAR-T cells in patient No. 16 reached a peak on the 7th day after CAR-T administration, and it was undetectable after 6 months.

**Table 2. BCMA CART copy number**

| Rese arch visit (tim e) | Patien t No. 1 | Patien t No. 2 | Patien t No. 3 | Patien t No. 4 | Patient No. 5 | Patien t No. 6 | Patient No. 7 | Patient No. 8 | Patie nt No. 9 | Pati ent No . 10 | Pat ient No . 11 | Patie nt No. 13 |
|---|---|---|---|---|---|---|---|---|---|---|---|---|
| D0 befo re infus ion | 1 | 1 | 1 | 1 | 1 | 1 | 1 | 1 | 1 | 1 | 1 | 1 |
| D1 | 1 | 1 | 1 | | 1 | 1 | 1 | 1841 | 44 | 130 | 276 | 1 |
| D2 | 48 | 1 | 1 | 1 | 1 | 1 | | | | | | 22 |
| D3 | 249 | 264 | 50 | 106 | 1 | 46 | 108.59 34753 | 10277 | 464 | 102 2 | 758 | 104 |
| D7 | 2335 | 19554 | 2555 | 72919 | 338 | 18484 | 12450. 17578 | 27471 | 2620 95 | 554 50 | | 372 |
| WK 2 | 38988 | 1518 | 60547 | | 252289 | 77760 | 372111 .7188 | 323300 | 1388 95 | | | 11686 2 |
| WK 3 | 7919 | 12351 | | | 90920 | 1786 | 57189. 09375 | 449369 | 7446 6 | | | 2619 89 |
| WK 4 | 2561 | 1146 | | | 22168 | 1265 | 43151. 21484 | 254071 | 1748 7 | | | 1467 6 |
| M2 | | | | | 2455 | | 12107 | 17003 | | | | 1656 |
| M3 | | | | | 31 | | 4040 | | | | | 478 |
| M4 | | | | | 1 | | | | | | | 109 |

After administration of anti-BCMA CAR-T cells, the disease state of the subject is evaluated to assess the response to treatment. After treatment, subjects were evaluated and monitored for neurotoxicity (neurological complications, including confusion symptoms, aphasia, seizures, convulsions, drowsiness and/or altered mental state), graded according to severity (use 1-5 scale for example, Guido Cavaletti and Paola Marmiroli Nature Reviews Neurology 6, 657-666 (December 2010), wherein grade 3 (severe symptoms), 4 (life-threatening symptoms) or 5 (death) are considered serious neurotoxicity.

### (1) Determine and monitor cytokine release syndrome (CRS):

Grade 1 (mild)-not life threatening, only systemic treatments such as antipyretics and antiemetics are required (for example, fever, nausea, fatigue, headache, myalgia, malaise);
Grade 2 (Medium)-Need and respond to moderate intervention:
   Oxygen demand <40%, or hypotension requiring corresponding body fluids or low-dose single vasopressor drugs, or Grade 2 organ toxicity (through CTCAE v4.0);
Grade 3 (severe)-requires and responds to active intervention:
   Oxygen demand ≥40%, or hypotension requiring high-dose single vasopressor drugs (for example, norepinephrine ≥20ug/kg/min, dopamine ≥10ug/kg/min, phenylephrine ≥200ug/kg/min, or adrenal gland ≥10ug/kg/min), or hypotension requiring multiple vasopressor drugs (for example, antidiuretic + one of the above reagents, or combination of vasopressor drugs equal to ≥ 20ug/kg/min norepinephrine), or grade 3 organ toxicity, or grade 4 transaminase inflammation (through CTCAE v4.0);
Grade 4 (life threatening)-ventilator support is required, or organ toxicity of grade 4 (excludes transaminase inflammation);
Grade 5 (Fatal)-Death.

### (2) Exemplary classification standards for neurotoxicity:

Grade 1 (no clinical symptoms or mild)-mild or no clinical symptoms;
Grade 2 (moderate)-there are symptoms that restrict active daily activities (ADL), such as cooking, shopping for vegetables or clothes, using the phone, and managing money;
Grade 3 (Severe)-There are restrictive self-management ADL, such as symptoms of bathing, dressing or undressing, eating, using the toilet, and taking medication;
Grade 4 (life threatening)-life-threatening symptoms requiring urgent intervention;
Grade 5 (Fatal)-Death.

None of the subjects had serious neurotoxicity, but they all had at least one adverse event; the most common adverse event related to the anti-BCMA CAR-T cells of the present invention was fever, followed by decreased platelets and decreased white blood cells. After symptomatic and supportive treatment, they were alleviated or in remission. For example, in the second group, one subject had grade 2 CRS and one subject had grade 3 CRS, all recovered after antipyretic, IL-6R monoclonal antibody (tocilizumab) and antibiotic anti-infective treatment; one subject had a grade 4 thrombocytopenia SAE and recovered after symptomatic and supportive treatment.

In the above embodiments, the CAR shown in SEQ ID NO: 36 is selected as an example. However, it should be understood that other CARs targeting BCMA may also be applied to the technical solutions described in this application, such as the CAR shown in SEQ ID NO: 30, 31, 32, 33, 34, 35, 39, 40, or 41.

Among them, the scFv of the CAR shown in SEQ ID NO: 30, 31, or 32 comprises the VH shown in SEQ ID NO: 15 and the VL shown in SEQ ID NO: 16, and the CDR regions are as follows: the HCDR1 shown in SEQ ID NO: 1, the HCDR2 shown in SEQ ID NO: 2, the HCDR3 shown in SEQ ID NO: 3, and the LCDR1 shown in SEQ ID NO: 6, the LCDR2 shown in SEQ ID NO: 7, and the LCDR3 shown in SEQ ID NO: 8.

Among them, the scFv of CAR shown in SEQ ID NO: 33, 34, or 35 comprises the VH shown in SEQ ID NO: 17, and the VL shown in SEQ ID NO: 18, and the CDR regions are respectively: the HCDR1 shown in SEQ ID NO: 1, the HCDR2 shown in SEQ ID NO: 2, the HCDR3 shown in SEQ ID NO: 4, and the LCDR1 shown in SEQ ID NO: 6, the LCDR2 shown in SEQ ID NO: 7, and the LCDR3 shown in SEQ ID NO: 9.

Among them, the scFv of CAR shown in SEQ ID NO: 39, 40, or 41 (amino acid sequence is shown in SEQ ID NO: 29, nucleotide sequence is shown in SEQ ID NO: 28) comprises the VH shown in SEQ ID NO: 23, and the VL shown in SEQ ID NO: 20, the CDR regions are the HCDR1 shown in SEQ ID NO: 13, the HCDR2 shown in SEQ ID NO: 14, the HCDR3 shown in SEQ ID NO: 5; and the LCDR1 shown in SEQ ID NO: 6, the LCDR2 shown in SEQ ID NO: 7, the LCDR3 shown in SEQ ID NO: 10.

All documents mentioned in the present invention are cited as references in this application, as if each document was individually cited as a reference. In addition, it should be understood that after reading the above teaching content of the present invention, those skilled in the art can make various changes or modifications to the present invention, and these equivalent forms also fall within the scope defined by the appended claims of the present application.

The following are the sequences involved in this patent text:

| Sequence name | SEQ ID NO | Sequence |
|---|---|---|
| HCDR1-1 | 1 | SYAMS |
| HCDR2-1 | 2 | AISGSGGSTYYADSVKG |
| HCDR3-1 | 3 | YPYLAFDY |
| HCDR3-2 | 4 | LSGDAAMDY |
| HCDR3-3 | 5 | VRPFWGTFDY |
| LCDR1-1 | 6 | RASQSVSSSYLA |
| LCDR2-1 | 7 | GASSRAT |
| LCDR3-1 | 8 | QQYGYPPSY |
| LCDR3-2 | 9 | QQYGYPPRY |
| LCDR3-3 | 10 | QQYFNPPEY |
| HCDR1-2 | 11 | gnams |
| HCDR2-2 | 12 | aisgnggstfyadsvkg |
| HCDR1-3 | 13 | syams |
| HCDR2-3 | 14 | aisggggntfyadsvkg |
| VH-1 amino acid sequence | 15 | EVQLLESGGGLVQPGGSLRLSCAASGFTFSSYAMSWVRQAPGKGLEWVSAISG SGGSTYYADSVKGRFTISRDNSKNTLYLQMNSLRAEDTAVYYCARYPYLAFDY WGQGTLVTVSS |
| VL-1 amino acid sequence | 16 | EIVLTQSPGTLSLSPGERATLSCRASQSVSSSYLAWYQQKPGQAPRLLIYGASSR ATGIPDRFSGSGSGTDFTLTISRLEPEDFAVYYCQQYGYPPSYTFGQGTKVEIK |
| VH-2 amino acid sequence | 17 | EVQLLESGGGLVQPGGSLRLSCAASGFTFSSYAMSWVRQAPGKGLEWVSAISG SGGSTYYADSVKGRFTISRDNSKNTLYLQMNSLRAEDTAVYYCAKLSGDAAMD YWGQGTLVTVSS |
| VL-2 amino acid sequence | 18 | EIVLTQSPGTLSLSPGERATLSCRASQSVSSSYLAWYQQKPGQAPRLLIYGASSR ATGIPDRFSGSGSGTDFTLTISRLEPEDFAVYYCQQYGYPPRYTFGQGTKVEIK |
| VH-3 amino acid sequence | 19 | EVQLLESGGGLVQPGGSLRLSCAASGFTFSSYAMSWVRQAPGKGLEWVSAISG SGGSTYYADSVKGRFTISRDNSKNTLYLQMNSLRAEDTAVYYCAKVRPFWGTF DYWGQGTLVTVSS |
| VL-3 amino | 20 | EIVLTQSPGTLSLSPGERATLSCRASQSVSSSYLAWYQQKPGQAPRLLIYGASSR |
| acid sequence | | ATGIPDRFSGSGSGTDFTLTISRLEPEDFAVYYCQQYFNPPEYTFGQGTKVEIK |
| VH-4(AA) | 21 | evqllesggglvqpggslrlscaasgftfggnamswvrqapgkglewvsaisgnggstfyadsvkgrftisrdnskntlyl qmnslraedtavyycakvrpfwgtfdywgqgtlvtvss |
| VH-4 nucleic acid sequence | 22 | gaggtgcaattgctggagtctgggggaggcttggtacagcctggggggtccctgagactctcctgtgcagcctccggattca cctttggcggtaatgccatgtcctgggtccgccaggctccagggaaggggctggagtgggtctcagcaattagtggtaatggt ggtagtacattctacgcagactccgtgaagggccggttcaccatctccagagacaattccaagaacacgctgtatctgcagat gaacagcctgagagccgaggacacggccgtatattactgtgcgaaagttcgtccattctggggtactttcgactactggggcc aaggaaccctggtcaccgtctcgagt |
| VH-5(AA) | 23 | evqllesggglvqpggslrlscaasgftfrsyamswvrqapgkglewvsaisggggntfyadsvkgrftisrdnskntlyl qmnslraedtavyycakvrpfwgtfdywgqgtlvtvss |
| VH-5 nucleic acid sequence | 24 | gaggtgcaattgctggagtctgggggaggcttggtacagcctggggggtccctgagactctcctgtgcagcctccggattca cctttaggagctatgccatgagctgggtccgccaggctccagggaaggggctggagtgggtctcagctattagtggcggtgg tggtaacacattctacgcagactccgtgaagggccggttcaccatctccagagacaattccaagaacacgctgtatctgcaga tgaacagcctgagagccgaggacacggccgtatattactgtgcgaaagttcgtccattctggggtactttcgactactggggc caaggaaccctggtcaccgtctcgagt |
| scFv-1 | 25 | Evqllesggglvqpggslrlscaasgftfssyamswvrqapgkglewvsaisgsggstyyadsvkgrftisrdnskntlyl qmnslraedtavyycakvrpfwgtfdywgqgtlvtvssggggsggggsggggseivltqspgtlslspgeratlscrasqs vsssylawyqqkpgqaprlliygassratgipdrfsgsgsgtdftltisrlepedfavyycqqyfnppeytfgqgtkveikr |
| scFv-2 nucleic acid sequence | 26 | gaggtgcaattgctggagtctgggggaggcttggtacagcctggggggtccctgagactctcctgtgcagcctccggattca cctttggcggtaatgccatgtcctgggtccgccaggctccagggaaggggctggagtgggtctcagcaattagtggtaatggt ggtagtacattctacgcagactccgtgaagggccggttcaccatctccagagacaattccaagaacacgctgtatctgcagat gaacagcctgagagccgaggacacggccgtatattactgtgcgaaagttcgtccattctggggtactttcgactactggggcc aaggaaccctggtcaccgtctcgagtggtggaggcggttcaggcggaggtggttctggcggtggcggatcggaaatcgtgt taacgcagtctccaggcaccctgtctttgtctccaggggaaagagccaccctctcttgcagggccagtcagagtgttagcagc agctacttagcctggtaccagcagaaacctggccaggctcccaggctcctcatctatggagcatccagcagggccactggca tcccagacaggttcagtggcagtggatccgggacagacttcactctcaccatcagcagactggagcctgaagattttgcagtg tattactgtcagcagtacttcaacccaccagaatacacgttcggccaggggaccaaagtggaaatcaaacgt |
| scFv-2(AA) | 27 | evqllesggglvqpggslrlscaasgftfggnamswvrqapgkglewvsaisgnggstfyadsvkgrftisrdnskntlyl qmnslraedtavyycakvrpfwgtfdywgqgtlvtvssggggsggggsggggseivltqspgtlslspgeratlscrasqs vsssylawyqqkpgqaprlliygassratgipdrfsgsgsgtdftltisrlepedfavyycqqyfnppeytfgqgtkveikr |
| scFv-3 nucleic acid sequence | 28 | gaggtgcaattgctggagtctgggggaggcttggtacagcctggggggtccctgagactctcctgtgcagcctccggattca cctttaggagctatgccatgagctgggtccgccaggctccagggaaggggctggagtgggtctcagctattagtggcggtgg tggtaacacattctacgcagactccgtgaagggccggttcaccatctccagagacaattccaagaacacgctgtatctgcaga tgaacagcctgagagccgaggacacggccgtatattactgtgcgaaagttcgtccattctggggtactttcgactactggggc caaggaaccctggtcaccgtctcgagtggtggaggcggttcaggcggaggtggttctggcggtggcggatcggaaatcgtg ttaacgcagtctccaggcaccctgtctttgtctccaggggaaagagccaccctctcttgcagggccagtcagagtgttagcag cagctacttagcctggtaccagcagaaacctggccaggctcccaggctcctcatctatggagcatccagcagggccactggc atcccagacaggttcagtggcagtggatccgggacagacttcactctcaccatcagcagactggagcctgaagattttgcagt gtattactgtcagcagtacttcaacccaccagaatacacgttcggccaggggaccaaagtggaaatcaaacgt |
| scFv-3(AA) | 29 | evqllesggglvqpggslrlscaasgftfrsyamswvrqapgkglewvsaisggggntfyadsvkgrftisrdnskntlyl qmnslraedtavyycakvrpfwgtfdywgqgtlvtvssggggsggggsggggseivltqspgtlslspgeratlscrasqs vsssylawyqqkpgqaprlliygassratgipdrfsgsgsgtdftltisrlepedfavyycqqyfnppeytfgqgtkveikr |
| CAR-1 | 30 | EVQLLESGGGLVQPGGSLRLSCAASGFTFSSYAMSWVRQAPGKGLEWVSAISG |
| | | SGGSTYYADSVKGRFTISRDNSKNTLYLQMNSLRAEDTAVYYCARYPYLAFDY WGQGTLVTVSSGGGGSGGGGSGGGGSEIVLTQSPGTLSLSPGERATLSCRASQS VSSSYLAWYQQKPGQAPRLLIYGASSRATGIPDRFSGSGSGTDFTLTISRLEPEDF AVYYCQQYGYPPSYTFGQGTKVEIKRTTTPAPRPPTPAPTIASQPLSLRPEACRPA AGGAVHTRGLDFACDIYIWAPLAGTCGVLLLSLVITLYCKRGRKKLLYIFKQPF MRPVQTTQEEDGCSCRFPEEEEGGCELRVKFSRSADAPAYQQGQNQLYNELNL GRREEYDVLDKRRGRDPEMGGKPQRRKNPQEGLYNELQKDKMAEAYSEIGM KGERRRGKGHDGLYQGLSTATKDTYDALHMQALPPR |
| CAR-2 | 31 | Evqllesggglvqpggslrlscaasgftfssyamswvrqapgkglewvsaisgsggstyyadsvkgrftisrdnskntlyl qmnslraedtavyycarypylafDywgqgtlvtvssggggsggggsggggseivltqspgtlslspgeratlscrasqsvs ssylawyqqkpgqaprlliygassratgipdrfsgsgsgtdftltisrlepedfavyycqqygyppsytfgqgtkveikrTT TPAPRPPTPAPTIASQPLSLRPEACRPAAGGAVHTRGLDFACDFWVLVVVGGVL ACYSLLVTVAFIIFWVRSKRSRLLHSDYMNMTPRRPGPTRKHYQPYAPPRDFAA YRSRVKFSRSADAPAYQQGQNQLYNELNLGRREEYDVLDKRRGRDPEMGGKP QRRKNPQEGLYNELQKDKMAEAYSEIGMKGERRRGKGHDGLYQGLSTATKDT YDALHMQALPPR |
| CAR-3 | 32 | Evqllesggglvqpggslrlscaasgftfssyamswvrqapgkglewvsaisgsggstyyadsvkgrftisrdnskntlyl qmnslraedtavyycarypylafDywgqgtlvtvssggggsggggsggggseivltqspgtlslspgeratlscrasqsvs ssylawyqqkpgqaprlliygassratgipdrfsgsgsgtdftltisrlepedfavyycqqygyppsytfgqgtkveikrTT TPAPRPPTPAPTIASQPLSLRPEACRPAAGGAVHTRGLDFACDFWVLVVVGGVL ACYSLLVTVAFIIFWVRSKRSRLLHSDYMNMTPRRPGPTRKHYQPYAPPRDFAA YRSKRGRKKLLYIFKQPFMRPVQTTQEEDGCSCRFPEEEEGGCELRVKFSRSAD APAYQQGQNQLYNELNLGRREEYDVLDKRRGRDPEMGGKPQRRKNPQEGLYN ELQKDKMAEAYSEIGMKGERRRGKGHDGLYQGLSTATKDTYDALHMQALPPR |
| CAR-4 | 33 | EVQLLESGGGLVQPGGSLRLSCAASGFTFSSYAMSWVRQAPGKGLEWVSAISG SGGSTYYADSVKGRFTISRDNSKNTLYLQMNSLRAEDTAVYYCAKLSGDAAMD YWGQGTLVTVSSGGGGSGGGGSGGGGSEIVLTQSPGTLSLSPGERATLSCRASQ SVSSSYLAWYQQKPGQAPRLLIYGASSRATGIPDRFSGSGSGTDFTLTISRLEPED FAVYYCQQYGYPPRYTFGQGTKVEIKRTTTPAPRPPTPAPTIASQPLSLRPEACRP AAGGAVHTRGLDFACDIYIWAPLAGTCGVLLLSLVITLYCKRGRKKLLYIFKQPF MRPVQTTQEEDGCSCRFPEEEEGGCELRVKFSRSADAPAYQQGQNQLYNELNL GRREEYDVLDKRRGRDPEMGGKPQRRKNPQEGLYNELQKDKMAEAYSEIGM KGERRRGKGHDGLYQGLSTATKDTYDALHMQALPPR |
| CAR-5 | 34 | Evqllesggglvqpggslrlscaasgftfssyamswvrqapgkglewvsaisgsggstyyadsvkgrftisrdnskntlyl qmnslraedtavyycaklsgdaAmdywgqgtlvtvssggggsggggsggggseivltqspgtlslspgeratlscrasq svsssylawyqqkpgqaprlliygassratgipdrfsgsgsgtdftltisrlepedfavyycqqygypprytfgqgtkveikr TTTPAPRPPTPAPTIASQPLSLRPEACRPAAGGAVHTRGLDFACDFWVLVVVGG VLACYSLLVTVAFIIFWVRSKRSRLLHSDYMNMTPRRPGPTRKHYQPYAPPRDF AAYRSRVKFSRSADAPAYQQGQNQLYNELNLGRREEYDVLDKRRGRDPEMGG KPQRRKNPQEGLYNELQKDKMAEAYSEIGMKGERRRGKGHDGLYQGLSTATK DTYDALHMQALPPR |
| CAR-6 | 35 | Evqllesggglvqpggslrlscaasgftfssyamswvrqapgkglewvsaisgsggstyyadsvkgrftisrdnskntlyl qmnslraedtavyycaklsgdaamdywgqgtlvtvssggggsggggsggggseivltqspgtlslspgeratlscrasqs vsssylawyqqkpgqaprlliygassratgipdrfsgsgsgtdftltisrlepedfavyycqqygypprytfgqgtkveikr |
| | | TTTPAPRPPTPAPTIASQPLSLRPEACRPAAGGAVHTRGLDFACDFWVLVVVGG VLACYSLLVTVAFIIFWVRSKRSRLLHSDYMNMTPRRPGPTRKHYQPYAPPRDF AAYRSKRGRKKLLYIFKQPFMRPVQTTQEEDGCSCRFPEEEEGGCELRVKFSRS ADAPAYQQGQNQLYNELNLGRREEYDVLDKRRGRDPEMGGKPQRRKNPQEGL YNELQKDKMAEAYSEIGMKGERRRGKGHDGLYQGLSTATKDTYDALHMQAL PPR |
| CAR-7 | 36 | EVQLLESGGGLVQPGGSLRLSCAASGFTFGGNAMSWVRQAPGKGLEWVSAIS GNGGSTFYADSVKGRFTISRDNSKNTLYLQMNSLRAEDTAVYYCAKVRPFWGT FDYWGQGTLVTVSSGGGGSGGGGSGGGGSEIVLTQSPGTLSLSPGERATLSCRA SQSVSSSYLAWYQQKPGQAPRLLIYGASSRATGIPDRFSGSGSGTDFTLTISRLEP EDFAVYYCQQYFNPPEYTFGQGTKVEIKRTTTPAPRPPTPAPTIASQPLSLRPEAC RPAAGGAVHTRGLDFACDIYIWAPLAGTCGVLLLSLVITLYCKRGRKKLLYIFKQ PFMRPVQTTQEEDGCSCRFPEEEEGGCELRVKFSRSADAPAYQQGQNQLYNEL NLGRREEYDVLDKRRGRDPEMGGKPQRRKNPQEGLYNELQKDKMAEAYSEIG MKGERRRGKGHDGLYQGLSTATKDTYDALHMQALPPR |
| CAR-8 | 37 | EVQLLESGGGLVQPGGSLRLSCAASGFTFGGNAMSWVRQAPGKGLEWVSAIS GNGGSTFYADSVKGRFTISRDNSKNTLYLQMNSLRAEDTAVYYCAKVRPFWGT FDYWGQGTLVTVSSGGGGSGGGGSGGGGSEIVLTQSPGTLSLSPGERATLSCRA SQSVSSSYLAWYQQKPGQAPRLLIYGASSRATGIPDRFSGSGSGTDFTLTISRLEP EDFAVYYCQQYFNPPEYTFGQGTKVEIKRTTTPAPRPPTPAPTIASQPLSLRPEAC RPAAGGAVHTRGLDFACDFWVLVVVGGVLACYSLLVTVAFIIFWVRSKRSRLL HSDYMNMTPRRPGPTRKHYQPYAPPRDFAAYRSRVKFSRSADAPAYQQGQNQL YNELNLGRREEYDVLDKRRGRDPEMGGKPQRRKNPQEGLYNELQKDKMAEA YSEIGMKGERRRGKGHDGLYQGLSTATKDTYDALHMQALPPR |
| CAR-9 | 38 | EVQLLESGGGLVQPGGSLRLSCAASGFTFGGNAMSWVRQAPGKGLEWVSAIS GNGGSTFYADSVKGRFTISRDNSKNTLYLQMNSLRAEDTAVYYCAKVRPFWGT FDYWGQGTLVTVSSGGGGSGGGGSGGGGSEIVLTQSPGTLSLSPGERATLSCRA SQSVSSSYLAWYQQKPGQAPRLLIYGASSRATGIPDRFSGSGSGTDFTLTISRLEP EDFAVYYCQQYFNPPEYTFGQGTKVEIKRTTTPAPRPPTPAPTIASQPLSLRPEAC RPAAGGAVHTRGLDFACDFWVLVVVGGVLACYSLLVTVAFIIFWVRSKRSRLL HSDYMNMTPRRPGPTRKHYQPYAPPRDFAAYRSKRGRKKLLYIFKQPFMRPVQ TTQEEDGCSCRFPEEEEGGCELRVKFSRSADAPAYQQGQNQLYNELNLGRREEY DVLDKRRGRDPEMGGKPQRRKNPQEGLYNELQKDKMAEAYSEIGMKGERRR GKGHDGLYQGLSTATKDTYDALHMQALPPR |
| CAR-10 | 39 | evqllesggglvqpggslrlscaasgftfrsyamswvrqapgkglewvsaisggggntfyadsvkgrftisrdnskntlyl qmnslraedtavyycakvrpfwgtfdywgqgtlvtvssggggsggggsggggseivltqspgtlslspgeratlscrasqs vsssylawyqqkpgqaprlliygassratgipdrfsgsgsgtdftltisrlepedfavyycqqyfnppeytfgqgtkveikr TTTPAPRPPTPAPTIASQPLSLRPEACRPAAGGAVHTRGLDFACDIYIWAPLAGTC GVLLLSLVITLYCKRGRKKLLYIFKQPFMRPVQTTQEEDGCSCRFPEEEEGGCEL RVKFSRSADAPAYKQGQNQLYNELNLGRREEYDVLDKRRGRDPEMGGKPRRK NPQEGLYNELQKDKMAEAYSEIGMKGERRRGKGHDGLYQGLSTATKDTYDAL HMQALPPR |
| CAR-11 | 40 | evqllesggglvqpggslrlscaasgftfrsyamswvrqapgkglewvsaisggggntfyadsvkgrftisrdnskntlyl qmnslraedtavyycakvrpfwgtfdywgqgtlvtvssggggsggggsggggseivltqspgtlslspgeratlscrasqs |
| | | vsssylawyqqkpgqaprlliygassratgipdrfsgsgsgtdftltisrlepedfavyycqqyfnppeytfgqgtkveikr TTTPAPRPPTPAPTIASQPLSLRPEACRPAAGGAVHTRGLDFACDFWVLVVVGG VLACYSLLVTVAFIIFWVRSKRSRLLHSDYMNMTPRRPGPTRKHYQPYAPPRDF AAYRSRVKFSRSADAPAYQQGQNQLYNELNLGRREEYDVLDKRRGRDPEMGG KPQRRKNPQEGLYNELQKDKMAEAYSEIGMKGERRRGKGHDGLYQGLSTATK DTYDALHMQALPPR |
| CAR-12 | 41 | evqllesggglvqpggslrlscaasgftfrsyamswvrqapgkglewvsaisggggntfyadsvkgrftisrdnskntlyl qmnslraedtavyycakvrpfwgtfdywgqgtlvtvssggggsggggsggggseivltqspgtlslspgeratlscrasqs vsssylawyqqkpgqaprlliygassratgipdrfsgsgsgtdftltisrlepedfavyycqqyfnppeytfgqgtkveikr TTTPAPRPPTPAPTIASQPLSLRPEACRPAAGGAVHTRGLDFACDFWVLVVVGG VLACYSLLVTVAFIIFWVRSKRSRLLHSDYMNMTPRRPGPTRKHYQPYAPPRDF AAYRSKRGRKKLLYIFKQPFMRPVQTTQEEDGCSCRFPEEEEGGCELRVKFSRS ADAPAYQQGQNQLYNELNLGRREEYDVLDKRRGRDPEMGGKPQRRKNPQEGL YNELQKDKMAEAYSEIGMKGERRRGKGHDGLYQGLSTATKDTYDALHMQAL PPR |
| Probe783-P1 | 42 | CTGAGACCCACTCCAGCCCCTTC |
| Primer783P 1-F1 | 43 | GCGGTAATGCCATGTCCTG |
| Primer783P 1-R3 | 44 | GCAGATACAGCGTGTTCTTGG |

## Claims

1. A method for treating BCMA-positive tumors, wherein the method comprises administering to a subject at least one course of treatment of immune effector cells expressing chimeric antigen receptors (CARs), and the immune effector cells specifically recognize BCMA.

2. The method of claim 1, wherein the dose of the immune effector cells in each course of treatment does not exceed about 1x 10⁹ cells/kg subject's body weight or does not exceed about 1x 10¹⁰ cells in total;
preferably, the dose of the immune effector cells in each course of treatment does not exceed about 1x 10⁸ cells/kg subject's body weight or does not exceed about 1x 10⁹ cells in total,
preferably, the dose of the immune effector cells in each course of treatment does not exceed about 1×10⁷ cells/kg subject's body weight or does not exceed about 5×10⁸ cells in total.

3. The method of claim 1, wherein the total dose of the immune effector cells in each course of treatment is no less than 1x10⁵;
preferably, the total dose of the immune effector cells in each course of treatment is no less than 1x 10⁶;
preferably, the total dose of the immune effector cells in each course of treatment is no less than 1×10⁷.

4. The method of claim 1, wherein the immune effector cells are administered to the subject for 2-5 courses of treatment,
more preferably, the immune effector cells of each course of treatment are divided into N administrations within 15 days, and N is a natural number no less than 1, in a preferable embodiment, N is 1, 2, 3, or 4.

5. The method of claim 4, wherein the immune effector cells of a subsequent course of treatment are administered after the immune effector cells administered previously are not detected in the body; or
the immune effector cells of the subsequent course of treatment are administered at a time point of about 4 to 24 weeks after the administration of the previous course of treatment.

6. The method of claim 4, wherein the dose of the immune effector cells administered in the subsequent course of treatment is lower than, equal to, or higher than that of the immune effector cells administered in the previous course of treatment,
preferably, the dose of the immune effector cells administered in the subsequent course of treatment is higher than that of the immune effector cells administered in the previous course of treatment,
more preferably, the dose of the immune effector cells administered in the subsequent course of treatment is 2 times, 5 times, 7 times or 10 times the dose of the immune effector cells administered in the previous course of treatment.

7. The method of claim 4, wherein the subject has any one of the following characteristics when administering the immune effector cells of the subsequent course of treatment:
(i) the serum level of the factor indicating cytokine release syndrome (CRS) in the subject is about 10 times lower, about 25 times lower, and/or about 50 times lower than that of the factor in the subject immediately before the administration of the immune effector cells in the previous course of treatment;
(ii) a grade 3 or higher neurotoxicity is not shown;
(iii) the neurotoxicity or CRS level is reduced compared to the peak level of neurotoxicity or CRS level after the administration of the immune effector cells of the previous course of treatment; or
(iv) the subject shows no detectable humoral or cell-mediated immune response against CAR expressed by cells of the previous course of treatment.

8. The method of claim 7, wherein in the (iii), the CRS level is reduced by at least 50% compared with the peak level of CRS after administering the immune effector cells in the previous course of treatment, preferably, reduced by at least 20%, more preferably, by at least 5%, or the CRS level is comparable to the CRS level before administering the immune effector cells in the previous course of treatment.

9. The method of claim 1, wherein the method further comprises pretreatment before administering the immune effector cells, and the pretreatment comprises administering a chemotherapeutic agent or radiation therapy to the subject, or a combination thereof, preferably the pretreatment is carried out 2-12 days before administering the immune effector cells,
more preferably, the pretreatment is carried out 2-7 days before administering the immune effector cells.

10. The method of claim 9, wherein the chemotherapeutic agent comprises any one or a combination of the following: cyclophosphamide, fludarabine.

11. The method of claim 10, wherein when fludarabine is used, the administration amount of fludarabine is about 10-50 mg/m²/day, or about 15-40 mg/m²/days, or about 15-35mg/m²/day, or 15-30mg/m²/day, or about 20-30mg/m²/day;
preferably, the administration amount of fludarabine is about 20-30 mg/m²/day;
preferably, the administration amount of fludarabine is about 20-26 mg/m²/day;
when cyclophosphamide is used, the administration amount of cyclophosphamide is about 100-700 mg/m²/day, or about 150-600 mg/m²/day, or about 190-600 mg/m²/day, or about 190-560 mg/m²/day;
preferably, the administration amount of cyclophosphamide is about 150-400 mg/m²/day, preferably, about 190-350 mg/m²/day;
preferably, the administration amount of cyclophosphamide is about 400-600 mg/m²/day, preferably, about 450-600 mg/m²/day, more preferably, about 450-560 mg/m²/day.

12. The method of claim 10, wherein the chemotherapeutic agent is continuously used for no more than 6 days; when cyclophosphamide is used, the cyclophosphamide is preferably used continuously for 1-5 days, and when fludarabine is used, the fludarabine is preferably used continuously for 2-4 days.

13. The method of claim 1, wherein the tumor is multiple myeloma.

14. The method of claim 1, wherein the chimeric antigen receptor comprises an antibody specifically binding to BCMA, a transmembrane domain, and an intracellular domain, and the heavy variable region and the light chain variable region of the antibody comprise:
The HCDR1 shown in SEQ ID NO: 1, the HCDR2 shown in SEQ ID NO: 2, the HCDR3 shown in SEQ ID NO: 3; and The LCDR1 shown in SEQ ID NO: 6, the LCDR2 shown in SEQ ID NO: 7, the LCDR3 shown in SEQ ID NO: 8; or
The HCDR1 shown in SEQ ID NO: 1, the HCDR2 shown in SEQ ID NO: 2, and the HCDR3 shown in SEQ ID NO: 4; and the LCDR1 shown in SEQ ID NO: 6, the LCDR2 shown in SEQ ID NO: 7, and the LCDR3 shown in SEQ ID NO: 9; or
The HCDR1 shown in SEQ ID NO: 1, the HCDR2 shown in SEQ ID NO: 2, the HCDR3 shown in SEQ ID NO: 5; and the LCDR1 shown in SEQ ID NO: 6, the LCDR2 shown in SEQ ID NO: 7, the LCDR3 shown in SEQ ID NO: 10; or
The HCDR1 shown in SEQ ID NO: 11, the HCDR2 shown in SEQ ID NO: 12, the HCDR3 shown in SEQ ID NO: 5; and the LCDR1 shown in SEQ ID NO: 6, the LCDR2 shown in SEQ ID NO: 7, the LCDR3 shown in SEQ ID NO: 10; or
The HCDR1 shown in SEQ ID NO: 13, the HCDR2 shown in SEQ ID NO: 14, the HCDR3 shown in SEQ ID NO: 5; and the LCDR1 shown in SEQ ID NO: 6, the LCDR2 shown in SEQ ID NO: 7, the LCDR3 shown in SEQ ID NO: 10.

15. The method of claim 1, wherein the chimeric antigen receptor comprises an antibody specifically binding to BCMA, a transmembrane domain, and an intracellular domain, and the light chain variable region of the antibody comprises the LCDR1 shown in SEQ ID NO: 6, the LCDR2 shown in SEQ ID NO: 7, and the LCDR3 shown in SEQ ID NO: 10.

16. The method of claim 15, wherein the light chain variable region of the antibody comprises the amino acid sequence shown in SEQ ID NO: 20; or
the heavy chain variable region of the antibody comprises the HCDR3 shown in SEQ ID NO:5.

17. The method of claim 14, wherein:
the heavy chain variable region of the antibody comprises the amino acid sequence shown in SEQ ID NO: 15 and the light chain variable region of the antibody comprises the amino acid sequence shown in SEQ ID NO: 16; or
the heavy chain variable region of the antibody comprises the amino acid sequence shown in SEQ ID NO: 17 and the light chain variable region of the antibody comprises the amino acid sequence shown in SEQ ID NO: 18; or
the heavy chain variable region of the antibody comprises the amino acid sequence shown in SEQ ID NO: 19 and the light chain variable region of the antibody comprises the amino acid sequence shown in SEQ ID NO: 20; or
the heavy chain variable region of the antibody comprises the amino acid sequence shown in SEQ ID NO: 21 and the light chain variable region of the antibody comprises the amino acid sequence shown in SEQ ID NO: 20; or
the heavy chain variable region of the antibody comprises the amino acid sequence shown in SEQ ID NO: 23 and the light chain variable region of the antibody comprises the amino acid sequence shown in SEQ ID NO: 20.

18. The method of claim 17, wherein the antibody comprises a sequence of scFv shown in SEQ ID NO: 25, 27, or 29.

19. The method of claim 14, wherein the chimeric antigen receptor comprises an amino acid sequence shown in SEQ ID NO: 30, 31, 32, 33, 34, 35, 36, 37, 38, 39, 40, or 41;
preferably, the chimeric antigen receptor comprises an amino acid sequence shown in any one of SEQ ID NO: 36, 37 and 38;
more preferably, the chimeric antigen receptor comprises the amino acid sequence shown in SEQ ID NO:36.

20. The method of any one of claims 1-19, wherein the immune effector cells are T cells, NK cells or NKT cells;
preferably, the immune effector cells are T cells, and more preferably, the immune effector cells are derived from the subject's own body.

21. The method of claim 1, wherein the administration interval of the immune effector cells in each course of treatment is about 4 to 24 weeks;
preferably, the number of immune cells in each course of treatment is basically the same;
preferably, the number of immune effector cells administered in the subsequent course of treatment is larger than the number of immune cells administered in the previous course of treatment;
preferably, the number of immune effector cells administered in the subsequent course of treatment is smaller than the number of immune cells administered in the previous course of treatment.

22. The method of claim 1, wherein the subject has not received treatment with immune cells expressing chimeric antigen receptors targeting BCMA before administering the immune effector cells; or
the subject has undergone surgical treatment, chemotherapy, or immunotherapy other than that described in claim 1 before administering the immune effector cell therapy.

23. The method of claim 1, wherein before administering the immune effector cells in each course of treatment, the serum levels of a factor indicating CRS, a factor indicating neurotoxicity, a factor indicating tumor burden, and/or a factor indicating the host's anti-CAR immune response are evaluated;
wherein, the factor indicating tumor burden is: the total number of tumor cells in the subject, or the total number of tumor cells in the organs of the subject, or the total number of tumor cells in the tissues of the subject, or the mass or volume of the tumor, or the extent of tumor metastasis, or the number of tumors.

24. The method of claim 23, whereing it comprises:
i) evaluating factors indicating tumor burden before administering the subsequent course of treatment; and
ii) based on the results of the evaluation, determining the subsequent course of treatment, and
iii) if the evaluation determines that the subject's tumor quality or volume is stable or decreasing, administering to the subject the subsequent course of treatment that comprises smaller or larger number of CAR-expressing cells than that in the previous course of treatment or about the same number of CAR-expressing cells as that in the previous course of treatment.

25. The method of claim 1, wherein the immune effector cells are administered at a dose of about 0.1×10⁶cells/kg subject's body weight to 5×10⁷ cells/kg subject's body weight, or the total dose of the immune effector cells administered is about 0.1x 107 cells∼1x 10¹⁰ cells;
preferably, it is about 0.5x 10⁶ cells/kg subject's body weight ∼1x 10⁷ cells/kg subject's body weight, or the total dose of the immune effector cells administered is about 0.1x 10⁸ cells∼1x 10⁹ cells;
more preferably, it is about 0.9×10⁶ cells/kg subject's body weight to 5×10⁶ cells/kg subject's body weight, or the total dose of the immune effector cells administered is about 0.1×10⁸ cells to 9×10⁸ cells.

26. The method of claim 1, wherein the positive rate of BCMA expression in the subject is greater than 50%, preferably, greater than 70%, or greater than 80%;
more preferably, greater than 85%;
more preferably, greater than 90%.

27. The method of claim 1, wherein the disease subtype of the subject is IgG κ type, or IgG λ type, or IgA λ type, or IgA κ type, or λ light chain type.


SEQUENCE LISTING

<110>  CARSGEN THERAPEUTICS CO., LTD.

<120>  METHODS FOR TUMOR TREATMENT WITH IMMUNE EFFECTOR CELLS

<130>  BET 21M0380

<140>  EP 19841265.2
<141>  2019-07-24

<150>  CN 201810821044.9
<151>  2018-07-24

<150>  CN 201910267885 .4
<151>  2019-04-03

<160>  44

<170>  PatentIn version 3.5

<210>  1
<211>  5
<212>  PRT
<213>  Artificial Sequence

<220>
<223>  Synthesized sequence

<400>  1

Ser Tyr Ala Met Ser
1               5


<210>  2
<211>  17
<212>  PRT
<213>  Artificial Sequence

<220>
<223>  Synthesized sequence

<400>  2

Ala Ile Ser Gly Ser Gly Gly Ser Thr Tyr Tyr Ala Asp Ser Val Lys
1               5                   10                  15


Gly



<210>  3
<211>  8
<212>  PRT
<213>  Artificial Sequence

<220>
<223>  Synthesized sequence

<400>  3

Tyr Pro Tyr Leu Ala Phe Asp Tyr
1               5

<210>  4
<211>  9
<212>  PRT
<213>  Artificial Sequence

<220>
<223>  Synthesized sequence

<400>  4

Leu Ser Gly Asp Ala Ala Met Asp Tyr
1               5

<210>  5
<211>  10
<212>  PRT
<213>  Artificial Sequence

<220>
<223>  Synthesized sequence

<400>  5

Val Arg Pro Phe Trp Gly Thr Phe Asp Tyr
1               5                   10

<210>  6
<211>  12
<212>  PRT
<213>  Artificial Sequence

<220>
<223>  Synthesized sequence

<400>  6

Arg Ala Ser Gln Ser Val Ser Ser Ser Tyr Leu Ala
1               5                   10

<210>  7
<211>  7
<212>  PRT
<213>  Artificial Sequence

<220>
<223>  Synthesized sequence

<400>  7

Gly Ala Ser Ser Arg Ala Thr
1               5

<210>  8
<211>  9
<212>  PRT

<213>  Artificial Sequence

<220>
<223>  Synthesized sequence

<400>  8

Gln Gln Tyr Gly Tyr Pro Pro Ser Tyr
1               5


<210>  9
<211>  9
<212>  PRT
<213>  Artificial Sequence

<220>
<223>  Synthesized sequence

<400>  9

Gln Gln Tyr Gly Tyr Pro Pro Arg Tyr
1               5


<210>  10
<211>  9
<212>  PRT
<213>  Artificial Sequence

<220>
<223>  Synthesized sequence

<400>  10

Gln Gln Tyr Phe Asn Pro Pro Glu Tyr
1               5


<210>  11
<211>  5
<212>  PRT
<213>  Artificial Sequence

<220>
<223>  Synthesized sequence

<400>  11

Gly Asn Ala Met Ser
1               5


<210>  12
<211>  17
<212>  PRT
<213>  Artificial Sequence

<220>
<223>  Synthesized sequence

<400>  12

Ala Ile Ser Gly Asn Gly Gly Ser Thr Phe Tyr Ala Asp Ser Val Lys
1               5                   10                  15

Gly

<210>  13
<211>  5
<212>  PRT
<213>  Artificial Sequence

<220>
<223>  Synthesized sequence

<400>  13

Ser Tyr Ala Met Ser
1               5

<210>  14
<211>  17
<212>  PRT
<213>  Artificial Sequence

<220>
<223>  Synthesized sequence

<400>  14

Ala Ile Ser Gly Gly Gly Gly Asn Thr Phe Tyr Ala Asp Ser Val Lys
1               5                   10                  15

Gly

<210>  15
<211>  117
<212>  PRT
<213>  Artificial Sequence

<220>
<223>  Synthesized sequence

<400>  15

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val

50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Tyr Pro Tyr Leu Ala Phe Asp Tyr Trp Gly Gln Gly Thr Leu
            100                 105                 110

Val Thr Val Ser Ser
        115

<210>  16
<211>  109
<212>  PRT
<213>  Artificial Sequence

<220>
<223>  Synthesized sequence

<400>  16

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Ser Ser
            20                  25                  30

Tyr Leu Ala Trp Tyr Gln Gln Lys Pro Gly Gln Ala Pro Arg Leu Leu
        35                  40                  45

Ile Tyr Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Gly Tyr Pro Pro
                85                  90                  95

Ser Tyr Thr Phe Gly Gln Gly Thr Lys Val Glu Ile Lys
            100                 105

<210>  17
<211>  118
<212>  PRT
<213>  Artificial Sequence

<220>

<223>  Synthesized sequence

<400>  17

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Leu Ser Gly Asp Ala Ala Met Asp Tyr Trp Gly Gln Gly Thr
            100                 105                 110

Leu Val Thr Val Ser Ser
        115

<210>  18
<211>  109
<212>  PRT
<213>  Artificial Sequence

<220>
<223>  Synthesized sequence

<400>  18

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Ser Ser
            20                  25                  30

Tyr Leu Ala Trp Tyr Gln Gln Lys Pro Gly Gln Ala Pro Arg Leu Leu
        35                  40                  45

Ile Tyr Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu

65                  70                  75                  80

Pro Glu Asp Phe Ala Val Tyr Tyr Cys Gln Gln Tyr Gly Tyr Pro Pro
                85                  90                  95

Arg Tyr Thr Phe Gly Gln Gly Thr Lys Val Glu Ile Lys
            100                 105

<210>  19
<211>  119
<212>  PRT
<213>  Artificial Sequence

<220>
<223>  Synthesized sequence

<400>  19

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Val Arg Pro Phe Trp Gly Thr Phe Asp Tyr Trp Gly Gln Gly
            100                 105                 110

Thr Leu Val Thr Val Ser Ser
        115

<210>  20
<211>  109
<212>  PRT
<213>  Artificial Sequence

<220>
<223>  Synthesized sequence

<400>  20

Glu Ile Val Leu Thr Gln Ser Pro Gly Thr Leu Ser Leu Ser Pro Gly
1               5                   10                  15

Glu Arg Ala Thr Leu Ser Cys Arg Ala Ser Gln Ser Val Ser Ser Ser
            20                  25                  30

Tyr Leu Ala Trp Tyr Gln Gln Lys Pro Gly Gln Ala Pro Arg Leu Leu
        35                  40                  45

Ile Tyr Gly Ala Ser Ser Arg Ala Thr Gly Ile Pro Asp Arg Phe Ser
    50                  55                  60

Gly Ser Gly Ser Gly Thr Asp Phe Thr Leu Thr Ile Ser Arg Leu Glu
65                  70                  75                  80

Pro Glu Asp Phe Ala Val Tyr Tyr Cys Gln Gln Tyr Phe Asn Pro Pro
                85                  90                  95

Glu Tyr Thr Phe Gly Gln Gly Thr Lys Val Glu Ile Lys
            100                 105

<210>  21
<211>  119
<212>  PRT
<213>  Artificial Sequence

<220>
<223>  Synthesized sequence

<400>  21

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Gly Gly Asn
            20                  25                  30

Ala Met Ser Trp Val Arg Gln Ala Pro Gly Lys Gly Leu Glu Trp Val
        35                  40                  45

Ser Ala Ile Ser Gly Asn Gly Gly Ser Thr Phe Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Val Arg Pro Phe Trp Gly Thr Phe Asp Tyr Trp Gly Gln Gly

            100                 105                 110

Thr Leu Val Thr Val Ser Ser
        115

<210> 22
<211> 357
<212> DNA
<213> Artificial Sequence

<220>
<223> Synthesized sequence

<400> 22
gaggtgcaat tgctggagtc tgggggaggc ttggtacagc ctggggggtc cctgagactc 60

tcctgtgcag cctccggatt cacctttggc ggtaatgcca tgtcctgggt ccgccaggct 120

ccagggaagg ggctggagtg ggtctcagca attagtggta atggtggtag tacattctac 180

gcagactccg tgaagggccg gttcaccatc tccagagaca attccaagaa cacgctgtat 240

ctgcagatga acagcctgag agccgaggac acggccgtat attactgtgc gaaagttcgt 300

ccattctggg gtactttcga ctactggggc caaggaaccc tggtcaccgt ctcgagt 357

<210> 23
<211> 119
<212> PRT
<213> Artificial Sequence

<220>
<223> Synthesized sequence

<400> 23

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Arg Ser Tyr
            20                  25                  30

Ala Met Ser Trp Val Arg Gln Ala Pro Gly Lys Gly Leu Glu Trp Val
        35                  40                  45

Ser Ala Ile Ser Gly Gly Gly Gly Asn Thr Phe Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Val Arg Pro Phe Trp Gly Thr Phe Asp Tyr Trp Gly Gln Gly
            100                 105                 110

Thr Leu Val Thr Val Ser Ser
        115

<210>  24
<211>  357
<212>  DNA
<213>  Artificial Sequence

<220>
<223>  Synthesized sequence

<400>  24
gaggtgcaat tgctggagtc tgggggaggc ttggtacagc ctggggggtc cctgagactc      60

tcctgtgcag cctccggatt cacctttagg agctatgcca tgagctgggt ccgccaggct     120

ccagggaagg ggctggagtg ggtctcagct attagtggcg gtggtggtaa cacattctac     180

gcagactccg tgaagggccg gttcaccatc tccagagaca attccaagaa cacgctgtat     240

ctgcagatga acagcctgag agccgaggac acggccgtat attactgtgc gaaagttcgt     300

ccattctggg gtactttcga ctactggggc caaggaaccc tggtcaccgt ctcgagt        357

<210>  25
<211>  244
<212>  PRT
<213>  Artificial Sequence

<220>
<223>  Synthesized sequence

<400>  25

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Val Arg Pro Phe Trp Gly Thr Phe Asp Tyr Trp Gly Gln Gly
            100                 105                 110

Thr Leu Val Thr Val Ser Ser Gly Gly Gly Gly Ser Gly Gly Gly Gly
        115                 120                 125

Ser Gly Gly Gly Gly Ser Glu Ile Val Leu Thr Gln Ser Pro Gly Thr
    130                 135                 140

Leu Ser Leu Ser Pro Gly Glu Arg Ala Thr Leu Ser Cys Arg Ala Ser
145                 150                 155                 160

Gln Ser Val Ser Ser Ser Tyr Leu Ala Trp Tyr Gln Gln Lys Pro Gly
                165                 170                 175

Gln Ala Pro Arg Leu Leu Ile Tyr Gly Ala Ser Ser Arg Ala Thr Gly
            180                 185                 190

Ile Pro Asp Arg Phe Ser Gly Ser Gly Ser Gly Thr Asp Phe Thr Leu
        195                 200                 205

Thr Ile Ser Arg Leu Glu Pro Glu Asp Phe Ala Val Tyr Tyr Cys Gln
    210                 215                 220

Gln Tyr Phe Asn Pro Pro Glu Tyr Thr Phe Gly Gln Gly Thr Lys Val
225                 230                 235                 240

Glu Ile Lys Arg

<210>  26
<211>  732
<212>  DNA
<213>  Artificial Sequence

<220>
<223>  Synthesized sequence

<400>  26
gaggtgcaat tgctggagtc tgggggaggc ttggtacagc ctggggggtc cctgagactc      60

tcctgtgcag cctccggatt cacctttggc ggtaatgcca tgtcctgggt ccgccaggct     120

ccagggaagg ggctggagtg ggtctcagca attagtggta atggtggtag tacattctac     180

gcagactccg tgaagggccg gttcaccatc tccagagaca attccaagaa cacgctgtat     240

ctgcagatga acagcctgag agccgaggac acggccgtat attactgtgc gaaagttcgt     300

ccattctggg gtactttcga ctactggggc caaggaaccc tggtcaccgt ctcgagtggt     360

ggaggcggtt caggcggagg tggttctggc ggtggcggat cggaaatcgt gttaacgcag     420

tctccaggca ccctgtcttt gtctccaggg gaaagagcca ccctctcttg cagggccagt 480

cagagtgtta gcagcagcta cttagcctgg taccagcaga aacctggcca ggctcccagg 540

ctcctcatct atggagcatc cagcagggcc actggcatcc cagacaggtt cagtggcagt 600

ggatccggga cagacttcac tctcaccatc agcagactgg agcctgaaga ttttgcagtg 660

tattactgtc agcagtactt caacccacca gaatacacgt tcggccaggg gaccaaagtg 720

gaaatcaaac gt 732

<210> 27
<211> 244
<212> PRT
<213> Artificial Sequence

<220>
<223> Synthesized sequence

<400> 27

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Gly Gly Asn
            20                  25                  30

Ala Met Ser Trp Val Arg Gln Ala Pro Gly Lys Gly Leu Glu Trp Val
        35                  40                  45

Ser Ala Ile Ser Gly Asn Gly Gly Ser Thr Phe Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Val Arg Pro Phe Trp Gly Thr Phe Asp Tyr Trp Gly Gln Gly
            100                 105                 110

Thr Leu Val Thr Val Ser Ser Gly Gly Gly Gly Ser Gly Gly Gly Gly
        115                 120                 125

Ser Gly Gly Gly Gly Ser Glu Ile Val Leu Thr Gln Ser Pro Gly Thr
    130                 135                 140

Leu Ser Leu Ser Pro Gly Glu Arg Ala Thr Leu Ser Cys Arg Ala Ser
145                 150                 155                 160

Gln Ser Val Ser Ser Ser Tyr Leu Ala Trp Tyr Gln Gln Lys Pro Gly
                165                 170                 175

Gln Ala Pro Arg Leu Leu Ile Tyr Gly Ala Ser Ser Arg Ala Thr Gly
            180                 185                 190

Ile Pro Asp Arg Phe Ser Gly Ser Gly Ser Gly Thr Asp Phe Thr Leu
        195                 200                 205

Thr Ile Ser Arg Leu Glu Pro Glu Asp Phe Ala Val Tyr Tyr Cys Gln
    210                 215                 220

Gln Tyr Phe Asn Pro Pro Glu Tyr Thr Phe Gly Gln Gly Thr Lys Val
225                 230                 235                 240

Glu Ile Lys Arg

<210>  28
<211>  732
<212>  DNA
<213>  Artificial Sequence

<220>
<223>  Synthesized sequence

<400>  28
gaggtgcaat tgctggagtc tgggggaggc ttggtacagc ctggggggtc cctgagactc      60

tcctgtgcag cctccggatt cacctttagg agctatgcca tgagctgggt ccgccaggct     120

ccagggaagg ggctggagtg ggtctcagct attagtggcg gtggtggtaa cacattctac     180

gcagactccg tgaagggccg gttcaccatc tccagagaca attccaagaa cacgctgtat     240

ctgcagatga acagcctgag agccgaggac acggccgtat attactgtgc gaaagttcgt     300

ccattctggg gtactttcga ctactggggc caaggaaccc tggtcaccgt ctcgagtggt     360

ggaggcggtt caggcggagg tggttctggc ggtggcggat cggaaatcgt gttaacgcag     420

tctccaggca ccctgtcttt gtctccaggg gaaagagcca ccctctcttg cagggccagt     480

cagagtgtta gcagcagcta cttagcctgg taccagcaga aacctggcca ggctcccagg     540

ctcctcatct atggagcatc cagcagggcc actggcatcc cagacaggtt cagtggcagt     600

ggatccggga cagacttcac tctcaccatc agcagactgg agcctgaaga ttttgcagtg     660

tattactgtc agcagtactt caacccacca gaatacacgt tcggccaggg gaccaaagtg     720

gaaatcaaac gt                                                         732

<210>  29
<211>  244

<212>  PRT
<213>  Artificial Sequence

<220>
<223>  Synthesized sequence

<400>  29

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Arg Ser Tyr
            20                  25                  30

Ala Met Ser Trp Val Arg Gln Ala Pro Gly Lys Gly Leu Glu Trp Val
        35                  40                  45

Ser Ala Ile Ser Gly Gly Gly Gly Asn Thr Phe Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Val Arg Pro Phe Trp Gly Thr Phe Asp Tyr Trp Gly Gln Gly
            100                 105                 110

Thr Leu Val Thr Val Ser Ser Gly Gly Gly Gly Ser Gly Gly Gly Gly
        115                 120                 125

Ser Gly Gly Gly Gly Ser Glu Ile Val Leu Thr Gln Ser Pro Gly Thr
    130                 135                 140

Leu Ser Leu Ser Pro Gly Glu Arg Ala Thr Leu Ser Cys Arg Ala Ser
145                 150                 155                 160

Gln Ser Val Ser Ser Ser Tyr Leu Ala Trp Tyr Gln Gln Lys Pro Gly
                165                 170                 175

Gln Ala Pro Arg Leu Leu Ile Tyr Gly Ala Ser Ser Arg Ala Thr Gly
            180                 185                 190

Ile Pro Asp Arg Phe Ser Gly Ser Gly Ser Gly Thr Asp Phe Thr Leu
        195                 200                 205

Thr Ile Ser Arg Leu Glu Pro Glu Asp Phe Ala Val Tyr Tyr Cys Gln
    210                 215                 220

Gln Tyr Phe Asn Pro Pro Glu Tyr Thr Phe Gly Gln Gly Thr Lys Val
225                 230                 235                 240

Glu Ile Lys Arg

<210>  30
<211>  466
<212>  PRT
<213>  Artificial Sequence

<220>
<223>  Synthesized sequence

<400>  30

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Tyr Pro Tyr Leu Ala Phe Asp Tyr Trp Gly Gln Gly Thr Leu
            100                 105                 110

Val Thr Val Ser Ser Gly Gly Gly Gly Ser Gly Gly Gly Gly Ser Gly
        115                 120                 125

Gly Gly Gly Ser Glu Ile Val Leu Thr Gln Ser Pro Gly Thr Leu Ser
    130                 135                 140

Leu Ser Pro Gly Glu Arg Ala Thr Leu Ser Cys Arg Ala Ser Gln Ser
145                 150                 155                 160

Val Ser Ser Ser Tyr Leu Ala Trp Tyr Gln Gln Lys Pro Gly Gln Ala
                165                 170                 175

Pro Arg Leu Leu Ile Tyr Gly Ala Ser Ser Arg Ala Thr Gly Ile Pro
        180                 185                 190

Asp Arg Phe Ser Gly Ser Gly Ser Gly Thr Asp Phe Thr Leu Thr Ile
    195                 200                 205

Ser Arg Leu Glu Pro Glu Asp Phe Ala Val Tyr Tyr Cys Gln Gln Tyr
    210                 215                 220

Gly Tyr Pro Pro Ser Tyr Thr Phe Gly Gln Gly Thr Lys Val Glu Ile
225                 230                 235                 240

Lys Arg Thr Thr Thr Pro Ala Pro Arg Pro Pro Thr Pro Ala Pro Thr
                245                 250                 255

Ile Ala Ser Gln Pro Leu Ser Leu Arg Pro Glu Ala Cys Arg Pro Ala
            260                 265                 270

Ala Gly Gly Ala Val His Thr Arg Gly Leu Asp Phe Ala Cys Asp Ile
        275                 280                 285

Tyr Ile Trp Ala Pro Leu Ala Gly Thr Cys Gly Val Leu Leu Leu Ser
    290                 295                 300

Leu Val Ile Thr Leu Tyr Cys Lys Arg Gly Arg Lys Lys Leu Leu Tyr
305                 310                 315                 320

Ile Phe Lys Gln Pro Phe Met Arg Pro Val Gln Thr Thr Gln Glu Glu
                325                 330                 335

Asp Gly Cys Ser Cys Arg Phe Pro Glu Glu Glu Glu Gly Gly Cys Glu
            340                 345                 350

Leu Arg Val Lys Phe Ser Arg Ser Ala Asp Ala Pro Ala Tyr Gln Gln
        355                 360                 365

Gly Gln Asn Gln Leu Tyr Asn Glu Leu Asn Leu Gly Arg Arg Glu Glu
    370                 375                 380

Tyr Asp Val Leu Asp Lys Arg Arg Gly Arg Asp Pro Glu Met Gly Gly
385                 390                 395                 400

Lys Pro Gln Arg Arg Lys Asn Pro Gln Glu Gly Leu Tyr Asn Glu Leu
                405                 410                 415

Gln Lys Asp Lys Met Ala Glu Ala Tyr Ser Glu Ile Gly Met Lys Gly
            420                 425                 430

Glu Arg Arg Arg Gly Lys Gly His Asp Gly Leu Tyr Gln Gly Leu Ser
        435                 440                 445

Thr Ala Thr Lys Asp Thr Tyr Asp Ala Leu His Met Gln Ala Leu Pro
    450                 455                 460

Pro Arg
465

<210> 31
<211> 468
<212> PRT
<213> Artificial Sequence

<220>
<223> Synthesized sequence

<400> 31

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Tyr Pro Tyr Leu Ala Phe Asp Tyr Trp Gly Gln Gly Thr Leu
            100                 105                 110

Val Thr Val Ser Ser Gly Gly Gly Gly Ser Gly Gly Gly Gly Ser Gly
        115                 120                 125

Gly Gly Gly Ser Glu Ile Val Leu Thr Gln Ser Pro Gly Thr Leu Ser
    130                 135                 140

Leu Ser Pro Gly Glu Arg Ala Thr Leu Ser Cys Arg Ala Ser Gln Ser
145                 150                 155                 160

Val Ser Ser Ser Tyr Leu Ala Trp Tyr Gln Gln Lys Pro Gly Gln Ala
                165                 170                 175

Pro Arg Leu Leu Ile Tyr Gly Ala Ser Ser Arg Ala Thr Gly Ile Pro
            180                 185                 190

Asp Arg Phe Ser Gly Ser Gly Ser Gly Thr Asp Phe Thr Leu Thr Ile
        195                 200                 205

Ser Arg Leu Glu Pro Glu Asp Phe Ala Val Tyr Tyr Cys Gln Gln Tyr
    210                 215                 220

Gly Tyr Pro Pro Ser Tyr Thr Phe Gly Gln Gly Thr Lys Val Glu Ile
225                 230                 235                 240

Lys Arg Thr Thr Thr Pro Ala Pro Arg Pro Pro Thr Pro Ala Pro Thr
                245                 250                 255

Ile Ala Ser Gln Pro Leu Ser Leu Arg Pro Glu Ala Cys Arg Pro Ala
            260                 265                 270

Ala Gly Gly Ala Val His Thr Arg Gly Leu Asp Phe Ala Cys Asp Phe
        275                 280                 285

Trp Val Leu Val Val Val Gly Gly Val Leu Ala Cys Tyr Ser Leu Leu
    290                 295                 300

Val Thr Val Ala Phe Ile Ile Phe Trp Val Arg Ser Lys Arg Ser Arg
305                 310                 315                 320

Leu Leu His Ser Asp Tyr Met Asn Met Thr Pro Arg Arg Pro Gly Pro
                325                 330                 335

Thr Arg Lys His Tyr Gln Pro Tyr Ala Pro Pro Arg Asp Phe Ala Ala
            340                 345                 350

Tyr Arg Ser Arg Val Lys Phe Ser Arg Ser Ala Asp Ala Pro Ala Tyr
        355                 360                 365

Gln Gln Gly Gln Asn Gln Leu Tyr Asn Glu Leu Asn Leu Gly Arg Arg
    370                 375                 380

Glu Glu Tyr Asp Val Leu Asp Lys Arg Arg Gly Arg Asp Pro Glu Met
385                 390                 395                 400

Gly Gly Lys Pro Gln Arg Arg Lys Asn Pro Gln Glu Gly Leu Tyr Asn
                405                 410                 415

Glu Leu Gln Lys Asp Lys Met Ala Glu Ala Tyr Ser Glu Ile Gly Met
            420                 425                 430

Lys Gly Glu Arg Arg Arg Gly Lys Gly His Asp Gly Leu Tyr Gln Gly
        435                 440                 445

Leu Ser Thr Ala Thr Lys Asp Thr Tyr Asp Ala Leu His Met Gln Ala
    450                 455                 460

Leu Pro Pro Arg
465

<210>  32
<211>  510
<212>  PRT
<213>  Artificial Sequence

<220>
<223>  Synthesized sequence

<400>  32

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Tyr Pro Tyr Leu Ala Phe Asp Tyr Trp Gly Gln Gly Thr Leu
            100                 105                 110

Val Thr Val Ser Ser Gly Gly Gly Gly Ser Gly Gly Gly Gly Ser Gly
        115                 120                 125

Gly Gly Gly Ser Glu Ile Val Leu Thr Gln Ser Pro Gly Thr Leu Ser
    130                 135                 140

Leu Ser Pro Gly Glu Arg Ala Thr Leu Ser Cys Arg Ala Ser Gln Ser
145                 150                 155                 160

Val Ser Ser Ser Tyr Leu Ala Trp Tyr Gln Gln Lys Pro Gly Gln Ala
                165                 170                 175

Pro Arg Leu Leu Ile Tyr Gly Ala Ser Ser Arg Ala Thr Gly Ile Pro
            180                 185                 190

Asp Arg Phe Ser Gly Ser Gly Ser Gly Thr Asp Phe Thr Leu Thr Ile
        195                 200                 205

Ser Arg Leu Glu Pro Glu Asp Phe Ala Val Tyr Tyr Cys Gln Gln Tyr
    210                 215                 220

Gly Tyr Pro Pro Ser Tyr Thr Phe Gly Gln Gly Thr Lys Val Glu Ile
225                 230                 235                 240

Lys Arg Thr Thr Thr Pro Ala Pro Arg Pro Pro Thr Pro Ala Pro Thr
                245                 250                 255

Ile Ala Ser Gln Pro Leu Ser Leu Arg Pro Glu Ala Cys Arg Pro Ala
            260                 265                 270

Ala Gly Gly Ala Val His Thr Arg Gly Leu Asp Phe Ala Cys Asp Phe
        275                 280                 285

Trp Val Leu Val Val Val Gly Gly Val Leu Ala Cys Tyr Ser Leu Leu
    290                 295                 300

Val Thr Val Ala Phe Ile Ile Phe Trp Val Arg Ser Lys Arg Ser Arg
305                 310                 315                 320

Leu Leu His Ser Asp Tyr Met Asn Met Thr Pro Arg Arg Pro Gly Pro
                325                 330                 335

Thr Arg Lys His Tyr Gln Pro Tyr Ala Pro Pro Arg Asp Phe Ala Ala
            340                 345                 350

Tyr Arg Ser Lys Arg Gly Arg Lys Lys Leu Leu Tyr Ile Phe Lys Gln
        355                 360                 365

Pro Phe Met Arg Pro Val Gln Thr Thr Gln Glu Glu Asp Gly Cys Ser
    370                 375                 380

Cys Arg Phe Pro Glu Glu Glu Glu Gly Gly Cys Glu Leu Arg Val Lys
385                 390                 395                 400

Phe Ser Arg Ser Ala Asp Ala Pro Ala Tyr Gln Gln Gly Gln Asn Gln
                405                 410                 415

Leu Tyr Asn Glu Leu Asn Leu Gly Arg Arg Glu Glu Tyr Asp Val Leu
            420                 425                 430

Asp Lys Arg Arg Gly Arg Asp Pro Glu Met Gly Gly Lys Pro Gln Arg
        435                 440                 445

Arg Lys Asn Pro Gln Glu Gly Leu Tyr Asn Glu Leu Gln Lys Asp Lys
    450                 455                 460

Met Ala Glu Ala Tyr Ser Glu Ile Gly Met Lys Gly Glu Arg Arg Arg
465                 470                 475                 480

Gly Lys Gly His Asp Gly Leu Tyr Gln Gly Leu Ser Thr Ala Thr Lys
                485                 490                 495

Asp Thr Tyr Asp Ala Leu His Met Gln Ala Leu Pro Pro Arg
            500                 505                 510

<210> 33
<211> 467
<212> PRT
<213> Artificial Sequence

<220>
<223> Synthesized sequence

<400> 33

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Leu Ser Gly Asp Ala Ala Met Asp Tyr Trp Gly Gln Gly Thr
            100                 105                 110

Leu Val Thr Val Ser Ser Gly Gly Gly Gly Ser Gly Gly Gly Gly Ser
        115                 120                 125

Gly Gly Gly Gly Ser Glu Ile Val Leu Thr Gln Ser Pro Gly Thr Leu
    130                 135                 140

Ser Leu Ser Pro Gly Glu Arg Ala Thr Leu Ser Cys Arg Ala Ser Gln
145                 150                 155                 160

Ser Val Ser Ser Ser Tyr Leu Ala Trp Tyr Gln Gln Lys Pro Gly Gln
                165                 170                 175

Ala Pro Arg Leu Leu Ile Tyr Gly Ala Ser Ser Arg Ala Thr Gly Ile
            180                 185                 190

Pro Asp Arg Phe Ser Gly Ser Gly Ser Gly Thr Asp Phe Thr Leu Thr
        195                 200                 205

Ile Ser Arg Leu Glu Pro Glu Asp Phe Ala Val Tyr Tyr Cys Gln Gln
    210                 215                 220

Tyr Gly Tyr Pro Pro Arg Tyr Thr Phe Gly Gln Gly Thr Lys Val Glu
225                 230                 235                 240

Ile Lys Arg Thr Thr Thr Pro Ala Pro Arg Pro Pro Thr Pro Ala Pro
                245                 250                 255

Thr Ile Ala Ser Gln Pro Leu Ser Leu Arg Pro Glu Ala Cys Arg Pro
            260                 265                 270

Ala Ala Gly Gly Ala Val His Thr Arg Gly Leu Asp Phe Ala Cys Asp
        275                 280                 285

Ile Tyr Ile Trp Ala Pro Leu Ala Gly Thr Cys Gly Val Leu Leu Leu
    290                 295                 300

Ser Leu Val Ile Thr Leu Tyr Cys Lys Arg Gly Arg Lys Lys Leu Leu
305                 310                 315                 320

Tyr Ile Phe Lys Gln Pro Phe Met Arg Pro Val Gln Thr Thr Gln Glu
                325                 330                 335

Glu Asp Gly Cys Ser Cys Arg Phe Pro Glu Glu Glu Glu Gly Gly Cys
            340                 345                 350

Glu Leu Arg Val Lys Phe Ser Arg Ser Ala Asp Ala Pro Ala Tyr Gln
        355                 360                 365

Gln Gly Gln Asn Gln Leu Tyr Asn Glu Leu Asn Leu Gly Arg Arg Glu
    370                 375                 380

Glu Tyr Asp Val Leu Asp Lys Arg Arg Gly Arg Asp Pro Glu Met Gly
385                 390                 395                 400

Gly Lys Pro Gln Arg Arg Lys Asn Pro Gln Glu Gly Leu Tyr Asn Glu
                405                 410                 415

Leu Gln Lys Asp Lys Met Ala Glu Ala Tyr Ser Glu Ile Gly Met Lys
            420                 425                 430

Gly Glu Arg Arg Arg Gly Lys Gly His Asp Gly Leu Tyr Gln Gly Leu
        435                 440                 445

Ser Thr Ala Thr Lys Asp Thr Tyr Asp Ala Leu His Met Gln Ala Leu
    450                 455                 460

Pro Pro Arg
465

<210>  34
<211>  469
<212>  PRT
<213>  Artificial Sequence

<220>
<223>  Synthesized sequence

<400>  34

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Leu Ser Gly Asp Ala Ala Met Asp Tyr Trp Gly Gln Gly Thr
            100                 105                 110

Leu Val Thr Val Ser Ser Gly Gly Gly Gly Ser Gly Gly Gly Gly Ser
        115                 120                 125

Gly Gly Gly Gly Ser Glu Ile Val Leu Thr Gln Ser Pro Gly Thr Leu
    130                 135                 140

Ser Leu Ser Pro Gly Glu Arg Ala Thr Leu Ser Cys Arg Ala Ser Gln
145                 150                 155                 160

Ser Val Ser Ser Ser Tyr Leu Ala Trp Tyr Gln Gln Lys Pro Gly Gln
                165                 170                 175

Ala Pro Arg Leu Leu Ile Tyr Gly Ala Ser Ser Arg Ala Thr Gly Ile
            180                 185                 190

Pro Asp Arg Phe Ser Gly Ser Gly Ser Gly Thr Asp Phe Thr Leu Thr
        195                 200                 205

Ile Ser Arg Leu Glu Pro Glu Asp Phe Ala Val Tyr Tyr Cys Gln Gln
    210                 215                 220

Tyr Gly Tyr Pro Pro Arg Tyr Thr Phe Gly Gln Gly Thr Lys Val Glu
225                 230                 235                 240

Ile Lys Arg Thr Thr Thr Pro Ala Pro Arg Pro Pro Thr Pro Ala Pro
                245                 250                 255

Thr Ile Ala Ser Gln Pro Leu Ser Leu Arg Pro Glu Ala Cys Arg Pro
            260                 265                 270

Ala Ala Gly Gly Ala Val His Thr Arg Gly Leu Asp Phe Ala Cys Asp
        275                 280                 285

Phe Trp Val Leu Val Val Val Gly Gly Val Leu Ala Cys Tyr Ser Leu
    290                 295                 300

Leu Val Thr Val Ala Phe Ile Ile Phe Trp Val Arg Ser Lys Arg Ser
305                 310                 315                 320

Arg Leu Leu His Ser Asp Tyr Met Asn Met Thr Pro Arg Arg Pro Gly
                325                 330                 335

Pro Thr Arg Lys His Tyr Gln Pro Tyr Ala Pro Pro Arg Asp Phe Ala
            340                 345                 350

Ala Tyr Arg Ser Arg Val Lys Phe Ser Arg Ser Ala Asp Ala Pro Ala
        355                 360                 365

Tyr Gln Gln Gly Gln Asn Gln Leu Tyr Asn Glu Leu Asn Leu Gly Arg
    370                 375                 380

Arg Glu Glu Tyr Asp Val Leu Asp Lys Arg Arg Gly Arg Asp Pro Glu
385                 390                 395                 400

Met Gly Gly Lys Pro Gln Arg Arg Lys Asn Pro Gln Glu Gly Leu Tyr
                405                 410                 415

Asn Glu Leu Gln Lys Asp Lys Met Ala Glu Ala Tyr Ser Glu Ile Gly
            420                 425                 430

Met Lys Gly Glu Arg Arg Arg Gly Lys Gly His Asp Gly Leu Tyr Gln
        435                 440                 445

Gly Leu Ser Thr Ala Thr Lys Asp Thr Tyr Asp Ala Leu His Met Gln
    450                 455                 460

Ala Leu Pro Pro Arg
465

<210> 35
<211> 511
<212> PRT
<213> Artificial Sequence

<220>
<223> Synthesized sequence

<400> 35

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Leu Ser Gly Asp Ala Ala Met Asp Tyr Trp Gly Gln Gly Thr
            100                 105                 110

Leu Val Thr Val Ser Ser Gly Gly Gly Gly Ser Gly Gly Gly Gly Ser
        115                 120                 125

Gly Gly Gly Gly Ser Glu Ile Val Leu Thr Gln Ser Pro Gly Thr Leu
    130                 135                 140

Ser Leu Ser Pro Gly Glu Arg Ala Thr Leu Ser Cys Arg Ala Ser Gln
145                 150                 155                 160

Ser Val Ser Ser Ser Tyr Leu Ala Trp Tyr Gln Gln Lys Pro Gly Gln
                165                 170                 175

Ala Pro Arg Leu Leu Ile Tyr Gly Ala Ser Ser Arg Ala Thr Gly Ile
            180                 185                 190

Pro Asp Arg Phe Ser Gly Ser Gly Ser Gly Thr Asp Phe Thr Leu Thr
        195                 200                 205

Ile Ser Arg Leu Glu Pro Glu Asp Phe Ala Val Tyr Tyr Cys Gln Gln
    210                 215                 220

Tyr Gly Tyr Pro Pro Arg Tyr Thr Phe Gly Gln Gly Thr Lys Val Glu
225                 230                 235                 240

Ile Lys Arg Thr Thr Thr Pro Ala Pro Arg Pro Pro Thr Pro Ala Pro
                245                 250                 255

Thr Ile Ala Ser Gln Pro Leu Ser Leu Arg Pro Glu Ala Cys Arg Pro
            260                 265                 270

Ala Ala Gly Gly Ala Val His Thr Arg Gly Leu Asp Phe Ala Cys Asp
        275                 280                 285

Phe Trp Val Leu Val Val Val Gly Gly Val Leu Ala Cys Tyr Ser Leu
    290                 295                 300

Leu Val Thr Val Ala Phe Ile Ile Phe Trp Val Arg Ser Lys Arg Ser
305                 310                 315                 320

Arg Leu Leu His Ser Asp Tyr Met Asn Met Thr Pro Arg Arg Pro Gly
                325                 330                 335

Pro Thr Arg Lys His Tyr Gln Pro Tyr Ala Pro Pro Arg Asp Phe Ala
            340                 345                 350

Ala Tyr Arg Ser Lys Arg Gly Arg Lys Lys Leu Leu Tyr Ile Phe Lys
        355                 360                 365

Gln Pro Phe Met Arg Pro Val Gln Thr Thr Gln Glu Glu Asp Gly Cys
    370                 375                 380

Ser Cys Arg Phe Pro Glu Glu Glu Glu Gly Gly Cys Glu Leu Arg Val
385                 390                 395                 400

Lys Phe Ser Arg Ser Ala Asp Ala Pro Ala Tyr Gln Gln Gly Gln Asn
                405                 410                 415

Gln Leu Tyr Asn Glu Leu Asn Leu Gly Arg Arg Glu Glu Tyr Asp Val
            420                 425                 430

Leu Asp Lys Arg Arg Gly Arg Asp Pro Glu Met Gly Gly Lys Pro Gln
        435                 440                 445

Arg Arg Lys Asn Pro Gln Glu Gly Leu Tyr Asn Glu Leu Gln Lys Asp
    450                 455                 460

Lys Met Ala Glu Ala Tyr Ser Glu Ile Gly Met Lys Gly Glu Arg Arg
465                 470                 475                 480

Arg Gly Lys Gly His Asp Gly Leu Tyr Gln Gly Leu Ser Thr Ala Thr
                485                 490                 495

Lys Asp Thr Tyr Asp Ala Leu His Met Gln Ala Leu Pro Pro Arg
            500                 505                 510

<210>  36
<211>  468
<212>  PRT
<213>  Artificial Sequence

<220>
<223>  Synthesized sequence

<400>  36

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Gly Gly Asn
            20                  25                  30

Ala Met Ser Trp Val Arg Gln Ala Pro Gly Lys Gly Leu Glu Trp Val
        35                  40                  45

Ser Ala Ile Ser Gly Asn Gly Gly Ser Thr Phe Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Val Arg Pro Phe Trp Gly Thr Phe Asp Tyr Trp Gly Gln Gly
            100                 105                 110

Thr Leu Val Thr Val Ser Ser Gly Gly Gly Gly Ser Gly Gly Gly Gly
        115                 120                 125

Ser Gly Gly Gly Gly Ser Glu Ile Val Leu Thr Gln Ser Pro Gly Thr
    130                 135                 140

Leu Ser Leu Ser Pro Gly Glu Arg Ala Thr Leu Ser Cys Arg Ala Ser
145                 150                 155                 160

Gln Ser Val Ser Ser Ser Tyr Leu Ala Trp Tyr Gln Gln Lys Pro Gly
                165                 170                 175

Gln Ala Pro Arg Leu Leu Ile Tyr Gly Ala Ser Ser Arg Ala Thr Gly
            180                 185                 190

Ile Pro Asp Arg Phe Ser Gly Ser Gly Ser Gly Thr Asp Phe Thr Leu
        195                 200                 205

Thr Ile Ser Arg Leu Glu Pro Glu Asp Phe Ala Val Tyr Tyr Cys Gln
    210                 215                 220

Gln Tyr Phe Asn Pro Pro Glu Tyr Thr Phe Gly Gln Gly Thr Lys Val
225                 230                 235                 240

Glu Ile Lys Arg Thr Thr Thr Pro Ala Pro Arg Pro Pro Thr Pro Ala
                245                 250                 255

Pro Thr Ile Ala Ser Gln Pro Leu Ser Leu Arg Pro Glu Ala Cys Arg
            260                 265                 270

Pro Ala Ala Gly Gly Ala Val His Thr Arg Gly Leu Asp Phe Ala Cys
        275                 280                 285

Asp Ile Tyr Ile Trp Ala Pro Leu Ala Gly Thr Cys Gly Val Leu Leu
    290                 295                 300

Leu Ser Leu Val Ile Thr Leu Tyr Cys Lys Arg Gly Arg Lys Lys Leu
305                 310                 315                 320

Leu Tyr Ile Phe Lys Gln Pro Phe Met Arg Pro Val Gln Thr Thr Gln
                325                 330                 335

Glu Glu Asp Gly Cys Ser Cys Arg Phe Pro Glu Glu Glu Glu Gly Gly
            340                 345                 350

Cys Glu Leu Arg Val Lys Phe Ser Arg Ser Ala Asp Ala Pro Ala Tyr
        355                 360                 365

Gln Gln Gly Gln Asn Gln Leu Tyr Asn Glu Leu Asn Leu Gly Arg Arg
    370                 375                 380

Glu Glu Tyr Asp Val Leu Asp Lys Arg Arg Gly Arg Asp Pro Glu Met
385                 390                 395                 400

Gly Gly Lys Pro Gln Arg Arg Lys Asn Pro Gln Glu Gly Leu Tyr Asn
                405                 410                 415

Glu Leu Gln Lys Asp Lys Met Ala Glu Ala Tyr Ser Glu Ile Gly Met
            420                 425                 430

Lys Gly Glu Arg Arg Arg Gly Lys Gly His Asp Gly Leu Tyr Gln Gly
        435                 440                 445

Leu Ser Thr Ala Thr Lys Asp Thr Tyr Asp Ala Leu His Met Gln Ala
    450                 455                 460

Leu Pro Pro Arg
465

<210>  37
<211>  470
<212>  PRT
<213>  Artificial Sequence

<220>
<223>  Synthesized sequence

<400>  37

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Gly Gly Asn
            20                  25                  30

Ala Met Ser Trp Val Arg Gln Ala Pro Gly Lys Gly Leu Glu Trp Val
        35                  40                  45

Ser Ala Ile Ser Gly Asn Gly Gly Ser Thr Phe Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Val Arg Pro Phe Trp Gly Thr Phe Asp Tyr Trp Gly Gln Gly
            100                 105                 110

Thr Leu Val Thr Val Ser Ser Gly Gly Gly Gly Ser Gly Gly Gly Gly
        115                 120                 125

Ser Gly Gly Gly Gly Ser Glu Ile Val Leu Thr Gln Ser Pro Gly Thr
    130                 135                 140

Leu Ser Leu Ser Pro Gly Glu Arg Ala Thr Leu Ser Cys Arg Ala Ser
145                 150                 155                 160

Gln Ser Val Ser Ser Ser Tyr Leu Ala Trp Tyr Gln Gln Lys Pro Gly
                165                 170                 175

Gln Ala Pro Arg Leu Leu Ile Tyr Gly Ala Ser Ser Arg Ala Thr Gly
            180                 185                 190

Ile Pro Asp Arg Phe Ser Gly Ser Gly Ser Gly Thr Asp Phe Thr Leu
        195                 200                 205

Thr Ile Ser Arg Leu Glu Pro Glu Asp Phe Ala Val Tyr Tyr Cys Gln
    210                 215                 220

Gln Tyr Phe Asn Pro Pro Glu Tyr Thr Phe Gly Gln Gly Thr Lys Val
225                 230                 235                 240

Glu Ile Lys Arg Thr Thr Thr Pro Ala Pro Arg Pro Pro Thr Pro Ala
                245                 250                 255

Pro Thr Ile Ala Ser Gln Pro Leu Ser Leu Arg Pro Glu Ala Cys Arg
            260                 265                 270

Pro Ala Ala Gly Gly Ala Val His Thr Arg Gly Leu Asp Phe Ala Cys
        275                 280                 285

Asp Phe Trp Val Leu Val Val Val Gly Gly Val Leu Ala Cys Tyr Ser
    290                 295                 300

Leu Leu Val Thr Val Ala Phe Ile Ile Phe Trp Val Arg Ser Lys Arg
305                 310                 315                 320

Ser Arg Leu Leu His Ser Asp Tyr Met Asn Met Thr Pro Arg Arg Pro
                325                 330                 335

Gly Pro Thr Arg Lys His Tyr Gln Pro Tyr Ala Pro Pro Arg Asp Phe
            340                 345                 350

Ala Ala Tyr Arg Ser Arg Val Lys Phe Ser Arg Ser Ala Asp Ala Pro
        355                 360                 365

Ala Tyr Gln Gln Gly Gln Asn Gln Leu Tyr Asn Glu Leu Asn Leu Gly
    370                 375                 380

Arg Arg Glu Glu Tyr Asp Val Leu Asp Lys Arg Arg Gly Arg Asp Pro
385                 390                 395                 400

Glu Met Gly Gly Lys Pro Gln Arg Arg Lys Asn Pro Gln Glu Gly Leu
                405                 410                 415

Tyr Asn Glu Leu Gln Lys Asp Lys Met Ala Glu Ala Tyr Ser Glu Ile
            420                 425                 430

Gly Met Lys Gly Glu Arg Arg Arg Gly Lys Gly His Asp Gly Leu Tyr
        435                 440                 445

Gln Gly Leu Ser Thr Ala Thr Lys Asp Thr Tyr Asp Ala Leu His Met
    450                 455                 460

Gln Ala Leu Pro Pro Arg
465                 470

<210> 38
<211> 512
<212> PRT
<213> Artificial Sequence

<220>

<223>  Synthesized sequence

<400>  38

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Gly Gly Asn
            20                  25                  30

Ala Met Ser Trp Val Arg Gln Ala Pro Gly Lys Gly Leu Glu Trp Val
        35                  40                  45

Ser Ala Ile Ser Gly Asn Gly Gly Ser Thr Phe Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Val Arg Pro Phe Trp Gly Thr Phe Asp Tyr Trp Gly Gln Gly
            100                 105                 110

Thr Leu Val Thr Val Ser Ser Gly Gly Gly Gly Ser Gly Gly Gly Gly
        115                 120                 125

Ser Gly Gly Gly Gly Ser Glu Ile Val Leu Thr Gln Ser Pro Gly Thr
    130                 135                 140

Leu Ser Leu Ser Pro Gly Glu Arg Ala Thr Leu Ser Cys Arg Ala Ser
145                 150                 155                 160

Gln Ser Val Ser Ser Ser Tyr Leu Ala Trp Tyr Gln Gln Lys Pro Gly
                165                 170                 175

Gln Ala Pro Arg Leu Leu Ile Tyr Gly Ala Ser Ser Arg Ala Thr Gly
            180                 185                 190

Ile Pro Asp Arg Phe Ser Gly Ser Gly Ser Gly Thr Asp Phe Thr Leu
        195                 200                 205

Thr Ile Ser Arg Leu Glu Pro Glu Asp Phe Ala Val Tyr Tyr Cys Gln
    210                 215                 220

Gln Tyr Phe Asn Pro Pro Glu Tyr Thr Phe Gly Gln Gly Thr Lys Val
225                 230                 235                 240

Glu Ile Lys Arg Thr Thr Thr Pro Ala Pro Arg Pro Pro Thr Pro Ala
                245                 250                 255

Pro Thr Ile Ala Ser Gln Pro Leu Ser Leu Arg Pro Glu Ala Cys Arg
            260                 265                 270

Pro Ala Ala Gly Gly Ala Val His Thr Arg Gly Leu Asp Phe Ala Cys
        275                 280                 285

Asp Phe Trp Val Leu Val Val Val Gly Gly Val Leu Ala Cys Tyr Ser
    290                 295                 300

Leu Leu Val Thr Val Ala Phe Ile Ile Phe Trp Val Arg Ser Lys Arg
305                 310                 315                 320

Ser Arg Leu Leu His Ser Asp Tyr Met Asn Met Thr Pro Arg Arg Pro
                325                 330                 335

Gly Pro Thr Arg Lys His Tyr Gln Pro Tyr Ala Pro Pro Arg Asp Phe
            340                 345                 350

Ala Ala Tyr Arg Ser Lys Arg Gly Arg Lys Lys Leu Leu Tyr Ile Phe
        355                 360                 365

Lys Gln Pro Phe Met Arg Pro Val Gln Thr Thr Gln Glu Glu Asp Gly
    370                 375                 380

Cys Ser Cys Arg Phe Pro Glu Glu Glu Glu Gly Gly Cys Glu Leu Arg
385                 390                 395                 400

Val Lys Phe Ser Arg Ser Ala Asp Ala Pro Ala Tyr Gln Gln Gly Gln
                405                 410                 415

Asn Gln Leu Tyr Asn Glu Leu Asn Leu Gly Arg Arg Glu Glu Tyr Asp
            420                 425                 430

Val Leu Asp Lys Arg Arg Gly Arg Asp Pro Glu Met Gly Gly Lys Pro
        435                 440                 445

Gln Arg Arg Lys Asn Pro Gln Glu Gly Leu Tyr Asn Glu Leu Gln Lys
    450                 455                 460

Asp Lys Met Ala Glu Ala Tyr Ser Glu Ile Gly Met Lys Gly Glu Arg
465                 470                 475                 480

Arg Arg Gly Lys Gly His Asp Gly Leu Tyr Gln Gly Leu Ser Thr Ala
                485                 490                 495

Thr Lys Asp Thr Tyr Asp Ala Leu His Met Gln Ala Leu Pro Pro Arg
                500                 505                 510

<210>  39
<211>  467
<212>  PRT
<213>  Artificial Sequence

<220>
<223>  Synthesized sequence

<400>  39

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Arg Ser Tyr
            20                  25                  30

Ala Met Ser Trp Val Arg Gln Ala Pro Gly Lys Gly Leu Glu Trp Val
        35                  40                  45

Ser Ala Ile Ser Gly Gly Gly Gly Asn Thr Phe Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Val Arg Pro Phe Trp Gly Thr Phe Asp Tyr Trp Gly Gln Gly
            100                 105                 110

Thr Leu Val Thr Val Ser Ser Gly Gly Gly Gly Ser Gly Gly Gly Gly
        115                 120                 125

Ser Gly Gly Gly Gly Ser Glu Ile Val Leu Thr Gln Ser Pro Gly Thr
    130                 135                 140

Leu Ser Leu Ser Pro Gly Glu Arg Ala Thr Leu Ser Cys Arg Ala Ser
145                 150                 155                 160

Gln Ser Val Ser Ser Ser Tyr Leu Ala Trp Tyr Gln Gln Lys Pro Gly
                165                 170                 175

Gln Ala Pro Arg Leu Leu Ile Tyr Gly Ala Ser Ser Arg Ala Thr Gly
            180                 185                 190

Ile Pro Asp Arg Phe Ser Gly Ser Gly Ser Gly Thr Asp Phe Thr Leu
        195                 200                 205

Thr Ile Ser Arg Leu Glu Pro Glu Asp Phe Ala Val Tyr Tyr Cys Gln
    210                 215                 220

Gln Tyr Phe Asn Pro Pro Glu Tyr Thr Phe Gly Gln Gly Thr Lys Val
225                 230                 235                 240

Glu Ile Lys Arg Thr Thr Thr Pro Ala Pro Arg Pro Pro Thr Pro Ala
                245                 250                 255

Pro Thr Ile Ala Ser Gln Pro Leu Ser Leu Arg Pro Glu Ala Cys Arg
            260                 265                 270

Pro Ala Ala Gly Gly Ala Val His Thr Arg Gly Leu Asp Phe Ala Cys
        275                 280                 285

Asp Ile Tyr Ile Trp Ala Pro Leu Ala Gly Thr Cys Gly Val Leu Leu
    290                 295                 300

Leu Ser Leu Val Ile Thr Leu Tyr Cys Lys Arg Gly Arg Lys Lys Leu
305                 310                 315                 320

Leu Tyr Ile Phe Lys Gln Pro Phe Met Arg Pro Val Gln Thr Thr Gln
                325                 330                 335

Glu Glu Asp Gly Cys Ser Cys Arg Phe Pro Glu Glu Glu Glu Gly Gly
            340                 345                 350

Cys Glu Leu Arg Val Lys Phe Ser Arg Ser Ala Asp Ala Pro Ala Tyr
        355                 360                 365

Lys Gln Gly Gln Asn Gln Leu Tyr Asn Glu Leu Asn Leu Gly Arg Arg
    370                 375                 380

Glu Glu Tyr Asp Val Leu Asp Lys Arg Arg Gly Arg Asp Pro Glu Met
385                 390                 395                 400

Gly Gly Lys Pro Arg Arg Lys Asn Pro Gln Glu Gly Leu Tyr Asn Glu
                405                 410                 415

Leu Gln Lys Asp Lys Met Ala Glu Ala Tyr Ser Glu Ile Gly Met Lys
            420                 425                 430

Gly Glu Arg Arg Arg Gly Lys Gly His Asp Gly Leu Tyr Gln Gly Leu
        435                 440                 445

Ser Thr Ala Thr Lys Asp Thr Tyr Asp Ala Leu His Met Gln Ala Leu
    450                 455                 460

Pro Pro Arg
465

<210>  40
<211>  470
<212>  PRT
<213>  Artificial Sequence

<220>
<223>  Synthesized sequence

<400>  40

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Arg Ser Tyr
            20                  25                  30

Ala Met Ser Trp Val Arg Gln Ala Pro Gly Lys Gly Leu Glu Trp Val
        35                  40                  45

Ser Ala Ile Ser Gly Gly Gly Gly Asn Thr Phe Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Val Arg Pro Phe Trp Gly Thr Phe Asp Tyr Trp Gly Gln Gly
            100                 105                 110

Thr Leu Val Thr Val Ser Ser Gly Gly Gly Gly Ser Gly Gly Gly Gly
        115                 120                 125

Ser Gly Gly Gly Gly Ser Glu Ile Val Leu Thr Gln Ser Pro Gly Thr
    130                 135                 140

Leu Ser Leu Ser Pro Gly Glu Arg Ala Thr Leu Ser Cys Arg Ala Ser
145                 150                 155                 160

Gln Ser Val Ser Ser Ser Tyr Leu Ala Trp Tyr Gln Gln Lys Pro Gly
                165                 170                 175

Gln Ala Pro Arg Leu Leu Ile Tyr Gly Ala Ser Ser Arg Ala Thr Gly
        180                 185                 190

Ile Pro Asp Arg Phe Ser Gly Ser Gly Ser Gly Thr Asp Phe Thr Leu
    195                 200                 205

Thr Ile Ser Arg Leu Glu Pro Glu Asp Phe Ala Val Tyr Tyr Cys Gln
    210                 215                 220

Gln Tyr Phe Asn Pro Pro Glu Tyr Thr Phe Gly Gln Gly Thr Lys Val
225                 230                 235                 240

Glu Ile Lys Arg Thr Thr Thr Pro Ala Pro Arg Pro Pro Thr Pro Ala
                245                 250                 255

Pro Thr Ile Ala Ser Gln Pro Leu Ser Leu Arg Pro Glu Ala Cys Arg
            260                 265                 270

Pro Ala Ala Gly Gly Ala Val His Thr Arg Gly Leu Asp Phe Ala Cys
        275                 280                 285

Asp Phe Trp Val Leu Val Val Val Gly Gly Val Leu Ala Cys Tyr Ser
    290                 295                 300

Leu Leu Val Thr Val Ala Phe Ile Ile Phe Trp Val Arg Ser Lys Arg
305                 310                 315                 320

Ser Arg Leu Leu His Ser Asp Tyr Met Asn Met Thr Pro Arg Arg Pro
                325                 330                 335

Gly Pro Thr Arg Lys His Tyr Gln Pro Tyr Ala Pro Pro Arg Asp Phe
            340                 345                 350

Ala Ala Tyr Arg Ser Arg Val Lys Phe Ser Arg Ser Ala Asp Ala Pro
        355                 360                 365

Ala Tyr Gln Gln Gly Gln Asn Gln Leu Tyr Asn Glu Leu Asn Leu Gly
    370                 375                 380

Arg Arg Glu Glu Tyr Asp Val Leu Asp Lys Arg Arg Gly Arg Asp Pro
385                 390                 395                 400

Glu Met Gly Gly Lys Pro Gln Arg Arg Lys Asn Pro Gln Glu Gly Leu
                405                 410                 415

Tyr Asn Glu Leu Gln Lys Asp Lys Met Ala Glu Ala Tyr Ser Glu Ile
            420                 425                 430

Gly Met Lys Gly Glu Arg Arg Arg Gly Lys Gly His Asp Gly Leu Tyr
        435                 440                 445

Gln Gly Leu Ser Thr Ala Thr Lys Asp Thr Tyr Asp Ala Leu His Met
    450                 455                 460

Gln Ala Leu Pro Pro Arg
465                 470

<210>  41
<211>  512
<212>  PRT
<213>  Artificial Sequence

<220>
<223>  Synthesized sequence

<400>  41

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Arg Ser Tyr
            20                  25                  30

Ala Met Ser Trp Val Arg Gln Ala Pro Gly Lys Gly Leu Glu Trp Val
        35                  40                  45

Ser Ala Ile Ser Gly Gly Gly Gly Asn Thr Phe Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Val Arg Pro Phe Trp Gly Thr Phe Asp Tyr Trp Gly Gln Gly
            100                 105                 110

Thr Leu Val Thr Val Ser Ser Gly Gly Gly Gly Ser Gly Gly Gly Gly
        115                 120                 125

Ser Gly Gly Gly Gly Ser Glu Ile Val Leu Thr Gln Ser Pro Gly Thr
    130                 135                 140

Leu Ser Leu Ser Pro Gly Glu Arg Ala Thr Leu Ser Cys Arg Ala Ser
145                 150                 155                 160

Gln Ser Val Ser Ser Ser Tyr Leu Ala Trp Tyr Gln Gln Lys Pro Gly
                165                 170                 175

Gln Ala Pro Arg Leu Leu Ile Tyr Gly Ala Ser Ser Arg Ala Thr Gly
            180                 185                 190

Ile Pro Asp Arg Phe Ser Gly Ser Gly Ser Gly Thr Asp Phe Thr Leu
        195                 200                 205

Thr Ile Ser Arg Leu Glu Pro Glu Asp Phe Ala Val Tyr Tyr Cys Gln
    210                 215                 220

Gln Tyr Phe Asn Pro Pro Glu Tyr Thr Phe Gly Gln Gly Thr Lys Val
225                 230                 235                 240

Glu Ile Lys Arg Thr Thr Thr Pro Ala Pro Arg Pro Pro Thr Pro Ala
                245                 250                 255

Pro Thr Ile Ala Ser Gln Pro Leu Ser Leu Arg Pro Glu Ala Cys Arg
            260                 265                 270

Pro Ala Ala Gly Gly Ala Val His Thr Arg Gly Leu Asp Phe Ala Cys
        275                 280                 285

Asp Phe Trp Val Leu Val Val Val Gly Gly Val Leu Ala Cys Tyr Ser
    290                 295                 300

Leu Leu Val Thr Val Ala Phe Ile Ile Phe Trp Val Arg Ser Lys Arg
305                 310                 315                 320

Ser Arg Leu Leu His Ser Asp Tyr Met Asn Met Thr Pro Arg Arg Pro
                325                 330                 335

Gly Pro Thr Arg Lys His Tyr Gln Pro Tyr Ala Pro Pro Arg Asp Phe
            340                 345                 350

Ala Ala Tyr Arg Ser Lys Arg Gly Arg Lys Lys Leu Leu Tyr Ile Phe
        355                 360                 365

Lys Gln Pro Phe Met Arg Pro Val Gln Thr Thr Gln Glu Glu Asp Gly
    370                 375                 380

Cys Ser Cys Arg Phe Pro Glu Glu Glu Glu Gly Gly Cys Glu Leu Arg
385                 390                 395                 400

Val Lys Phe Ser Arg Ser Ala Asp Ala Pro Ala Tyr Gln Gln Gly Gln
                405                 410                 415

Asn Gln Leu Tyr Asn Glu Leu Asn Leu Gly Arg Arg Glu Glu Tyr Asp
            420                 425                 430

Val Leu Asp Lys Arg Arg Gly Arg Asp Pro Glu Met Gly Gly Lys Pro
        435                 440                 445

Gln Arg Arg Lys Asn Pro Gln Glu Gly Leu Tyr Asn Glu Leu Gln Lys
    450                 455                 460

Asp Lys Met Ala Glu Ala Tyr Ser Glu Ile Gly Met Lys Gly Glu Arg
465                 470                 475                 480

Arg Arg Gly Lys Gly His Asp Gly Leu Tyr Gln Gly Leu Ser Thr Ala
                485                 490                 495

Thr Lys Asp Thr Tyr Asp Ala Leu His Met Gln Ala Leu Pro Pro Arg
            500                 505                 510

<210>  42
<211>  23
<212>  DNA
<213>  Artificial Sequence

<220>
<223>  Synthesized sequence

<400>  42
ctgagaccca ctccagcccc ttc                                              23

<210>  43
<211>  19
<212>  DNA
<213>  Artificial Sequence

<220>
<223>  Synthesized sequence

<400>  43
gcggtaatgc catgtcctg                                                   19

<210>  44
<211>  21
<212>  DNA
<213>  Artificial Sequence

<220>
<223>  Synthesized sequence

<400>  44
gcagatacag cgtgttcttg g                                                21